# EUROPEAN PATENT SPECIFICATION

(11) **EP 4 115 951 B1**
(45) Date of publication and mention of the grant of the patent: **09.12.2026**
(21) Application number: 22172338.0
(22) Date of filing: 04.10.2017
(51) Int. Cl.: A61P 35/00, C12N 9/90, C07K 14/725, C07K 14/705, C07K 14/715, C07K 16/28

(54) **CO-STIMULATORY DOMAINS FOR USE IN GENETICALLY-MODIFIED CELLS**
CO-STIMULIERENDE DOMÄNEN ZUR VERWENDUNG BEI GENETISCH VERÄNDERTEN ZELLEN
DOMAINES CO-STIMULATEURS DESTINÉS À ÊTRE UTILISÉS DANS DES CELLULES GÉNÉTIQUEMENT MODIFIÉES

(30) Priority: 04.10.2016 US 201662403880 P; 04.05.2017 US 201762501475 P; 08.09.2017 US 201762556199 P
(43) Date of publication of application: 11.01.2023
(62) Document number(s) of the earlier application(s) in accordance with Art. 76 EPC: 20176441.2 / 3 757 120; 17800638.3 / 3 523 326
(73) Proprietor: Precision Biosciences, Inc., Durham, NC 27701 (US)
(72) Inventor: JANTZ, Derek, Durham, 27701 (US); MARTIN, Aaron, Carrboro, 27510 (US); MACLEOD, Daniel, T., La Jolla, 92037 (US)
(74) Representative: Grund, Martin

(56) References cited:
- WO-A1-2011/041093
- US-A1- 2016 051 651
- US-A1- 2016 158 359
- DATABASE UniProt [online] EBI; 18 February 2014 (2014-02-18), UNKNOWN: "PREDICTED: tumor necrosis factor receptor superfamily member 9 [Elephantulus edwardii]", XP002808091, Database accession no. UPI0003F05851

## Description

### FIELD OF THE INVENTION

The present disclosure relates to the field of molecular biology and recombinant nucleic acid technology. In particular, the present disclosure relates to novel co-stimulatory domains engineered to stimulate T cell proliferation and avert T cell exhaustion. The present disclosure further relates to genetically-modified cells comprising the novel co-stimulatory domains and such cells for use in treatment of disease, such as cancer.

### BACKGROUND OF THE INVENTION

T cell adoptive immunotherapy is a promising approach for cancer treatment. This strategy utilizes isolated human T cells that have been genetically-modified to enhance their specificity for a specific tumor associated antigen. Genetic modification may involve the expression of a chimeric antigen receptor (CAR) or an exogenous T cell receptor to graft antigen specificity onto the T cell. In contrast to exogenous T cell receptors, CARs derive their specificity from the variable domains of a monoclonal antibody. Thus, T cells expressing CARs induce tumor immunoreactivity in a major histocompatibility complex (MHC) non-restricted manner. To date, T cell adoptive immunotherapy has been utilized as a clinical therapy for a number of cancers, including B cell malignancies (*e.g*., acute lymphoblastic leukemia (ALL), B cell non-Hodgkin lymphoma (NHL), and chronic lymphocytic leukemia), multiple myeloma, neuroblastoma, glioblastoma, advanced gliomas, ovarian cancer, mesothelioma, melanoma, and pancreatic cancer.

T cell activation is initiated by the endogenous T cell receptor, which also provides antigen specificity to the cell. Signaling by the T cell is amplified by cell-surface co-stimulatory receptors, such as CD28. In the absence of co-stimulation, stimulation via the T cell receptor can be insufficient to drive T cell proliferation, resulting in cell anergy. Co-stimulation also serves a role in averting T cell exhaustion and some forms of activation-induced cell death. Early chimeric T cell-activating receptors were generated by fusing the ζ-chain of CD3 to the extracellular domain of T cell co-receptors, including CD4, CD8, and CD25.

So called "first generation" chimeric antigen receptors were then generated by fusing the CD3 ζ-chain to a single-chain variable fragment (scFv), which afforded antigen specificity and antigen-induced T cell activation. Although first generation CARs were able to mediate cytotoxicity, they were unable to direct antigen-induced expansion of modified primary T cells. Indeed, studies in early transgenic mouse models revealed that T cells expressing first generation CARs only produced a moderate effect on tumor progression *in vivo* due to anergy and the production of low amounts of interferon gamma (IFN-γ). "Second generation" chimeric antigen receptors were generated by further fusing a single co-stimulatory domain in cis with the cytoplasmic CD3 ζ-chain. Studies demonstrated that the addition of the co-stimulatory domain allowed for expansion of primary CAR-T cells following repeated antigen exposure, as well as increased secretion of cytokines such as IFN-γ. Indeed, early clinical trials utilizing second-generation CAR-T cells displayed significantly enhanced persistence and expansion in patients with B-cell lymphoma, CLL, and B-cell ALL. A number of co-stimulatory domains have been introduced into CARs, such as CD28, 4-1BB, or OX-40 elements, which have been utilized in CAR-T cells administered to patients with B cell malignancies. So called "third generation" chimeric antigen receptors introduced two co-stimulatory domains in tandem with the cytoplasmic CD3 ζ-chain to allow for further enhancement of cell expansion and/or cytokine secretion following repeated antigen exposure.

In addition to the use of co-stimulatory domains within CAR constructs, groups have also incorporated co-stimulatory domains into various "safety switches" that can be separate from the CAR. One such safety switch comprises MyD88 and CD40 signaling domains, fused to a binding domain which dimerizes when bound by a small molecule such as rimiducid. This safety switch is used in tandem with a CAR construct that only comprises a cytoplasmic CD3 ζ-chain to promote cell activation. By administering the small molecule, the safety switch dimerizes and allows for MyD88/CD40 co-stimulatory signaling to promote CAR-T cell expansion and cytokine secretion following antigen recognition by the separate CAR construct.

A number of co-stimulatory domains have been previously disclosed in both patents and in the literature. For example, U.S. Patent No. 8,399,645 claims a polynucleotide encoding chimeric antigen receptor comprising both the 4-1BB signaling domain and a CD3 ζ signaling domain. WO 2011/041093 A1, US 2016/051651 A1, and US 2016/158359 A1 also disclose chimeric antigen receptors comprising different co-stimulatory domains. However, none have disclosed the domains of the present disclosure or any domains having even 80% sequence identity to the domains of the present disclosure.

### SUMMARY OF THE INVENTION

The invention is defined by the appended claims.

The present disclosure provides novel co-stimulatory domains that are useful in providing genetically-modified cells to promote cell proliferation and/or promote cytokine secretion. The present disclosure advances the art by providing new co-stimulatory domains which promote different degrees of cell proliferation and/or cytokine secretion following antigen-induced cell activation. For example, the co-stimulatory domains disclosed herein provide superior activity when compared to those domains, such as 4-1BB and CD28. Also disclosed herein are genetically-modified cells that comprise a chimeric antigen receptor (CAR) that incorporates one or more co-stimulatory domains disclosed herein. In other examples, genetically-modified cells disclosed herein comprise an inducible regulatory construct that incorporates one or more co-stimulatory domains disclosed herein. Also provided herein are nucleic acid molecules, recombinant DNA constructs (*e.g*., plasmids), and viral vectors comprising a nucleic acid sequence encoding the co-stimulatory domains, and compositions comprising the novel co-stimulatory domains for use in methods of administering the same to subjects in order to reduce the symptoms, progression, or occurrence of disease. In some embodiments, genetically-modified cells comprising the novel co-stimulatory domains disclosed herein are formulated as pharmaceutical compositions for use, for example, as immunotherapy in the treatment of cancer.

Thus, in one aspect, the present disclosure provides a nucleic acid molecule comprising a nucleotide sequence encoding a co-stimulatory domain having at least 90% sequence identity to the amino acid sequence of SEQ ID NO: 5. In the various aspects of the present disclosure, the co-stimulatory domain disclosed herein has at least 95%, at least 96%, at least 97%, at least 98%, or at least 99% sequence identity to the amino acid sequence of SEQ ID NO: 5.

In some embodiments, the amino acid sequence encoding a co-stimulatory domain comprises at least one TRAF-binding motif. In certain embodiments, the TRAF-binding motif is selected from the group consisting of SEQ ID NOs: 9-11. In specific embodiments, the amino acid sequence encoding a co-stimulatory domain comprises two TRAF-binding motifs separated by a spacer sequence. In some such embodiments, the spacer sequence is selected from the group consisting of SEQ ID NOs: 12-15. In certain embodiments, the amino acid sequence encoding a co-stimulatory domain comprises two TRAF-binding motifs selected from the group consisting of SEQ ID NOs: 9-11 separated by a spacer sequence, wherein the spacer sequence is selected from the group consisting of SEQ ID NOs: 12-15.

In particular embodiments, the amino acid sequence encoding a co-stimulatory domain comprises the TRAF binding motifs of SEQ ID NOs: 9 and 11, separated by a spacer sequence, such as the spacer sequence set forth in SEQ ID NO: 12. In some embodiments, the amino acid sequence encoding the co-stimulatory domain comprises the TRAF binding motifs of SEQ ID NOs: 10 and 11, separated by a spacer sequence, such as the spacer sequence set forth in any of SEQ ID NOs: 13-15.

In specific embodiments, the nucleic acid molecule comprises a nucleotide sequence encoding a co-stimulatory domain comprising the amino acid sequence set forth in SEQ ID NO: 5.

In some embodiments, the nucleic acid molecule comprises the nucleotide sequence set forth in SEQ ID NO: 1, or a variant or fragment thereof having at least 80% identity to the nucleotide sequence of SEQ ID NO: 1, wherein the nucleotide sequence encodes a co-stimulatory domain. In some embodiments, the nucleic acid molecule comprises a nucleotide sequence comprising a variant or fragment of SEQ ID NO: 1 having at least 80%, at least 85%, at least 90%, at least 95%, at least 96%, at least 97%, at least 98%, or at least 99% sequence identity to the nucleic acid sequence set forth in SEQ ID NO: 1, wherein the nucleotide sequence encodes an active co-stimulatory domain.

In some embodiments, the nucleic acid molecule comprises a nucleotide sequence encoding a chimeric antigen receptor (CAR), wherein the CAR comprises at least one co-stimulatory domain described herein having at least 90% sequence identity to the amino acid sequence of SEQ ID NO: 5. In some such embodiments, the nucleic acid molecule comprises a nucleotide sequence encoding a CAR comprising at least one co-stimulatory domain comprising an amino acid sequence set forth in SEQ ID NO: 5.

In other such embodiments, the CAR comprises an extracellular antigen-binding domain. In particular embodiments, the extracellular antigen-binding domain is a single-chain variable fragment (scFv). In various embodiments, the antigen-binding domain has specificity for a cancer or tumor antigen. In specific embodiments, the encoded CAR comprises a CD19-specific antigen-binding domain.

In further such embodiments, the encoded CAR comprises at least two co-stimulatory domains. In such embodiments, the at least two co-stimulatory domains are co-stimulatory domains described herein or, alternatively, are at least one co-stimulatory domain described herein and at least one additional co-stimulatory domain known in the art (*e.g.,* 4-1BB, CD28, OX40, ICOS). In some embodiments, the encoded CAR further comprises at least one intracellular signaling domain. In particular embodiments, the at least one intracellular signaling domain is a CD3 ζ domain.

In other embodiments, the nucleic acid molecule comprises a nucleotide sequence encoding an inducible regulatory construct that comprises at least one co-stimulatory domain having at least 90% sequence identity to the amino acid sequence of SEQ ID NO: 5. In some embodiments, the nucleic acid molecule comprises a nucleotide sequence encoding an inducible regulatory construct comprising at least one co-stimulatory domain comprising the amino acid sequence of SEQ ID NO: 5. In particular embodiments, the encoded inducible regulatory construct further comprises a binding domain which allows two inducible regulatory constructs to dimerize, wherein dimerization initiates a co-stimulatory signal to a cell. In some embodiments, the binding domain binds a small molecule (*e.g*., rimiducid), an antibody, or other molecule which allows for dimerization. In particular embodiments wherein the binding domain binds a small molecule, the binding domain comprises an analogue of FKBP12 (*e.g*., comprising an F36V substitution) and the small molecule is rimiducid (*i.e.,* AP1903).

In particular embodiments, the nucleic acid molecule is an mRNA, a recombinant DNA construct (*e.g*., plasmid), or included within a viral genome of a viral vector.

In another aspect, the present disclosure provides a recombinant DNA construct, wherein the recombinant DNA construct comprises the nucleic acid molecule described herein which comprises a nucleotide sequence encoding at least one co-stimulatory domain having at least 90% sequence identity to the amino acid sequence of SEQ ID NO: 5. In specific embodiments, the recombinant DNA construct comprises a nucleotide sequence encoding a co-stimulatory domain comprising the amino acid sequence set forth in SEQ ID NO: 5. In some embodiments, the recombinant DNA construct comprises a nucleotide sequence encoding a CAR described herein, wherein the CAR comprises at least one co-stimulatory domain having at least 90% sequence identity to the amino acid sequence of SEQ ID NO: 5. In other embodiments, the recombinant DNA construct comprises a nucleotide sequence encoding an inducible regulatory construct described herein which comprises at least one co-stimulatory domain having at least 90% sequence identity to the amino acid sequence of SEQ ID NO: 5.

In some such embodiments, the recombinant DNA construct encodes a viral vector which comprises a nucleotide sequence encoding at least one co-stimulatory domain having at least 90% sequence identity to the amino acid sequence of SEQ ID NO: 5. In some embodiments, the viral vector is a retroviral vector, a lentiviral vector, an adenoviral vector, or an adeno-associated virus (AAV) vector. In a specific embodiment, the viral vector is a recombinant AAV vector.

In some aspects, the present disclosure provides a viral vector comprising the nucleic acid molecule described herein which comprises a nucleotide sequence encoding at least one co-stimulatory domain having at least 90% sequence identity to the amino acid sequence of SEQ ID NO: 5. In specific embodiments, the viral vector comprises a nucleotide sequence encoding a co-stimulatory domain comprising the amino acid sequence of SEQ ID NO: 5. In some embodiments, the viral vector comprises a nucleotide sequence encoding a CAR described herein, wherein the CAR comprises at least one co-stimulatory domain having at least 90% sequence identity to the amino acid sequence of SEQ ID NO: 5. In other embodiments, the viral vector comprises a nucleotide sequence encoding an inducible regulatory construct described herein which comprises at least one co-stimulatory domain having at least 90% sequence identity to the amino acid sequence of SEQ ID NO: 5.

In particular embodiments, the viral vector is a retroviral vector, a lentiviral vector, an adenoviral vector, or an adeno-associated virus (AAV) vector. In a specific embodiment, the viral vector is a recombinant AAV vector.

In another aspect, the present disclosure provides a genetically-modified T cell or natural killer (NK) cell, wherein the genetically-modified cell comprises the nucleic acid molecule described herein which comprises a nucleotide sequence encoding at least one co-stimulatory domain having at least 90% sequence identity to the amino acid sequence of SEQ ID NO: 5. In some embodiments, the genetically-modified T cell or NK cell comprises an expression cassette comprising a nucleotide sequence encoding a CAR described herein, wherein the CAR comprises at least one co-stimulatory domain having at least 90% sequence identity to the amino acid sequence of SEQ ID NO: 5. In particular embodiments, the genetically-modified T cell or NK cell comprises an expression cassette comprising a nucleotide sequence encoding an inducible regulatory construct described herein, wherein the inducible regulatory construct comprises at least one co-stimulatory domain having at least 90% sequence identity to the amino acid sequence of SEQ ID NO: 5. In some such embodiments, the nucleic acid molecule described herein, or the expression cassette described herein, is present within the genome of the genetically-modified T cell or NK cell or, alternatively, is not integrated into the genome of the cell. In some such embodiments, the nucleic acid molecule described herein, or the expression cassette described herein, is present in the genetically-modified T cell or NK cell in a recombinant DNA construct, in an mRNA, or in a viral genome, which is not integrated into the genome of the cell.

In further embodiments, the genetically-modified T cell or NK cell comprises: (i) a CAR expression cassette comprising a nucleotide sequence encoding a CAR that does not comprise a co-stimulatory domain described herein, and (ii) a regulatory expression cassette encoding an inducible regulatory construct described herein which comprises at least one co-stimulatory domain having at least 90% sequence identity to the amino acid sequence of SEQ ID NO: 5. Thus, in a particular embodiment, the genetically-modified T cell or NK cell comprises: (i) a CAR that does not incorporate a co-stimulatory domain described herein, and (ii) an inducible regulatory domain which comprises at least one co-stimulatory domain described herein. In some embodiments, the genetically-modified T cell or NK cell comprises an expression cassette comprising: (i) a nucleotide sequence encoding a CAR that does not comprise a co-stimulatory domain described herein, and (ii) a nucleotide sequence encoding an inducible regulatory domain which comprises at least one co-stimulatory domain described herein. In each embodiment, the CAR expression cassette and/or the regulatory expression cassette are within the genome of the genetically-modified T cell or NK cell or, alternatively, are not integrated into the genome of the cell. In some such embodiments, the CAR expression cassette and/or the regulatory expression cassette is present in the genetically-modified T cell or NK cell in a recombinant DNA construct, in an mRNA, or in a viral genome, which are not integrated into the genome of the cell.

In some embodiments, the genetically-modified cell is a primary human T cell or primary human NK cell. In further embodiments, the genetically-modified cell is a human CAR-T cell or human CAR-NK cell.

In particular embodiments, the genetically-modified T cell or NK cell described herein exhibits increased proliferation and/or cytokine secretion compared to a control cell that does not comprise a co-stimulatory domain described herein. In some embodiments, the increased proliferation and/or cytokine secretion is exhibited *in vitro* and/or *in vivo.* In particular embodiments, increased cytokine secretion comprises an increase in IFN-y, IL-2, TNF-alpha, or other cytokines associated with cell activation and/or proliferation compared to a control cell that does not comprise a co-stimulatory domain described herein.

In another aspect, the present disclosure provides an *in vitro* method for producing a genetically-modified T cell or NK cell comprising at least one co-stimulatory domain described herein, the method comprising introducing into a T cell or NK cell at least one nucleic acid molecule described herein encoding at least one co-stimulatory domain having at least 90% sequence identity to the amino acid sequence of SEQ ID NO: 5.

In some embodiments of the method, the introduced nucleic acid molecule encodes a CAR described herein comprising at least one co-stimulatory domain having at least 90% sequence identity to the amino acid sequence of SEQ ID NO: 5.
In some embodiments of the method, the introduced nucleic acid molecule encodes an inducible regulatory construct described herein comprising at least one co-stimulatory domain having at least 90% sequence identity to the amino acid sequence of SEQ ID NO: 5.

In some embodiments, the method further comprises introducing into the cell: (i) a second nucleic acid molecule encoding an engineered nuclease, wherein the engineered nuclease is expressed in the cell, or (ii) an engineered nuclease protein; wherein the engineered nuclease recognizes and cleaves a recognition sequence to produce a cleavage site in the genome of the cell, and wherein the nucleic acid encoding the at least one co-stimulatory domain or active variant or fragment thereof is inserted into the genome at the cleavage site. In some embodiments, the engineered nuclease is an engineered meganuclease, a recombinant zinc-finger nuclease (ZFN), a recombinant transcription activator-like effector nuclease (TALEN), a CRISPR/Cas nuclease, or a megaTAL nuclease. In a particular embodiment of the method, the engineered nuclease is an engineered meganuclease. In a specific embodiment, the engineered meganuclease is a single-chain meganuclease.

In one such embodiment of the method, the nucleic acid molecule encoding at least one co-stimulatory domain having at least 90% sequence identity to the amino acid sequence of SEQ ID NO: 5 introduced into the cell further comprises sequences homologous to sequences flanking the nuclease cleavage site, such that the nucleic acid molecule is inserted into the genome at the cleavage site by homologous recombination. In another such embodiment of the method, the nucleic acid molecule lacks substantial homology to the nuclease cleavage site such that the nucleic acid molecule is inserted into the genome by non-homologous end joining.

In some embodiments of the method, the cell is a primary human T cell or a primary human NK cell. In certain embodiments, the genetically-modified cell produced by the method is a human CAR-T cell or human CAR-NK cell.

In specific embodiments of the method, the nucleic acid molecule encoding at least one co-stimulatory domain having at least 90% sequence identity to the amino acid sequence of SEQ ID NO: 5 is introduced into the cell using an mRNA described herein, a recombinant DNA construct described herein, or a viral vector described herein.

In some embodiments, introduction of the at least one nucleic acid molecule described herein encoding at least one co-stimulatory domain having at least 90% sequence identity to the amino acid sequence of SEQ ID NO: 5 increases the activation, proliferation, and/or cytokine secretion of the genetically-modified cell. In specific embodiments, increased cytokine secretion comprises an increased secretion of IFN-y, IL-2, TNF-alpha, or any other cytokine associated with cell activation and/or proliferation when compared to a control cell that does not comprise a co-stimulatory domain described herein.

In another aspect, the present disclosure provides a pharmaceutical composition comprising a pharmaceutically-acceptable carrier and genetically-modified T cell or NK cells described herein which comprise at least one co-stimulatory domain having at least 90% sequence identity to the amino acid sequence of SEQ ID NO: 5. In particular embodiments, the genetically-modified T cells or NK cells comprise a CAR described herein which comprises at least one co-stimulatory domain having at least 90% sequence identity to the amino acid sequence of SEQ ID NO: 5. In other embodiments, the genetically-modified T cells or NK cells comprise an inducible regulatory construct described herein which comprises at least one co-stimulatory domain having at least 90% sequence identity to the amino acid sequence of SEQ ID NO: 5.

In specific embodiments, the genetically-modified cells are CAR-T cells. In some such embodiments, the CAR-T cells comprise a CAR which includes a co-stimulatory domain described herein having at least 90% sequence identity to the amino acid sequence of SEQ ID NO: 5. In other such embodiments, the CAR-T cells comprise a CAR which does not include a co-stimulatory domain or active variant or fragment thereof described herein, and further comprise an inducible regulatory construct comprising at least one co-stimulatory domain described herein having at least 90% sequence identity to the amino acid sequence of SEQ ID NO: 5. In various embodiments, the CAR-T cells have specificity for a cancer or tumor-specific antigen, and the pharmaceutical composition is useful in methods of cancer immunotherapy.

In another aspect, the present disclosure provides genetically-modified T cells or NK cells for use in a method of immunotherapy for treating cancer in a subject, wherein the genetically-modified T cells or NK cells are those described herein which comprise at least one co-stimulatory domain having at least 90% sequence identity to the amino acid sequence of SEQ ID NO: 5. In some embodiments of the method, the genetically-modified T cells or NK cells comprise a CAR described herein comprising at least one co-stimulatory domain having at least 90% sequence identity to the amino acid sequence of SEQ ID NO: 5. In other embodiments of the method, the genetically-modified T cells or NK cells comprise an inducible regulatory construct described herein comprising at least one co-stimulatory domain having at least 90% sequence identity to the amino acid sequence of SEQ ID NO: 5.

In some embodiments, the genetically-modified cells are CAR-T cells. In such embodiments, the CAR-T cells have specificity for a cancer or tumor-specific antigen and are useful in methods of cancer immunotherapy. In some embodiments, the cancer is a B cell malignancy (*e.g*., acute lymphoblastic leukemia (ALL), B cell non-Hodgkin lymphoma (NHL), or chronic lymphocytic leukemia), multiple myeloma, neuroblastoma, glioblastoma, advanced gliomas, ovarian cancer, mesothelioma, melanoma, or pancreatic cancer. In particular embodiments, the cancer is a B cell lymphoma.

In some embodiments, the cancer is selected from the group consisting of a cancer of carcinoma, lymphoma, sarcoma, blastomas, and leukemia.

In some embodiments, the cancer is selected from the group consisting of a cancer of B-cell origin, breast cancer, gastric cancer, neuroblastoma, osteosarcoma, lung cancer, melanoma, prostate cancer, colon cancer, renal cell carcinoma, ovarian cancer, rhabdomyo sarcoma, leukemia, and Hodgkin's lymphoma. In some embodiments, the cancer of B-cell origin is selected from the group consisting of B-lineage acute lymphoblastic leukemia, B-cell chronic lymphocytic leukemia, and B-cell non-Hodgkin's lymphoma.

The foregoing and other aspects and embodiments of the present invention can be more fully understood by reference to the following detailed description and claims. Certain features of the invention, which are, for clarity, described in the context of separate embodiments, may also be provided in combination in a single embodiment. All combinations of the embodiments are specifically embraced by the present invention and are disclosed herein just as if each and every combination was individually and explicitly disclosed. Conversely, various features of the invention, which are, for brevity, described in the context of a single embodiment, may also be provided separately or in any suitable sub-combination. All subcombinations of features listed in the embodiments are also specifically embraced by the present invention and are disclosed herein just as if each and every such sub-combination was individually and explicitly disclosed herein. Embodiments of each aspect of the present invention disclosed herein apply to each other aspect of the invention *mutatis mutandis.*

### BRIEF DESCRIPTION OF THE FIGURES

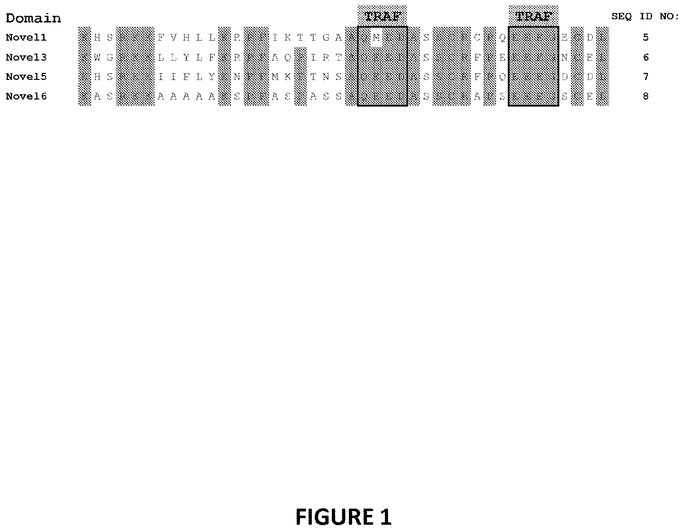

Figure 1 shows the alignment of novel co-stimulatory domains Novel1 (SEQ ID NO: 5), Novel3 (SEQ ID NO: 6), Novel5 (SEQ ID NO: 7), and Novel6 (SEQ ID NO: 8). Individual TRAF-binding motifs are identified, and spacer regions can be found between the TRAF-binding motifs within each listed co-stimulatory domain.

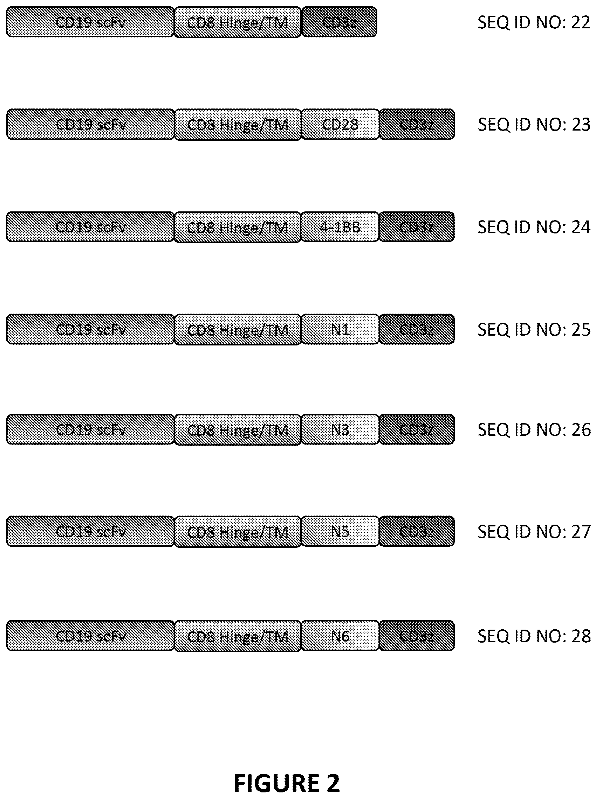

Figure 2 shows CAR constructs comprising an anti-CD19 scFv, a CD8 hinge and transmembrane domain, a co-stimulatory domain, and a CD3 ζ intracellular signaling domain. The co-stimulatory domains shown include CD28, 4-1BB, Novel1 (N1), Novel3 (N3), Novel5 (N5), and Novel6 (N6). Also shown is a CAR construct lacking a co-stimulatory domain.

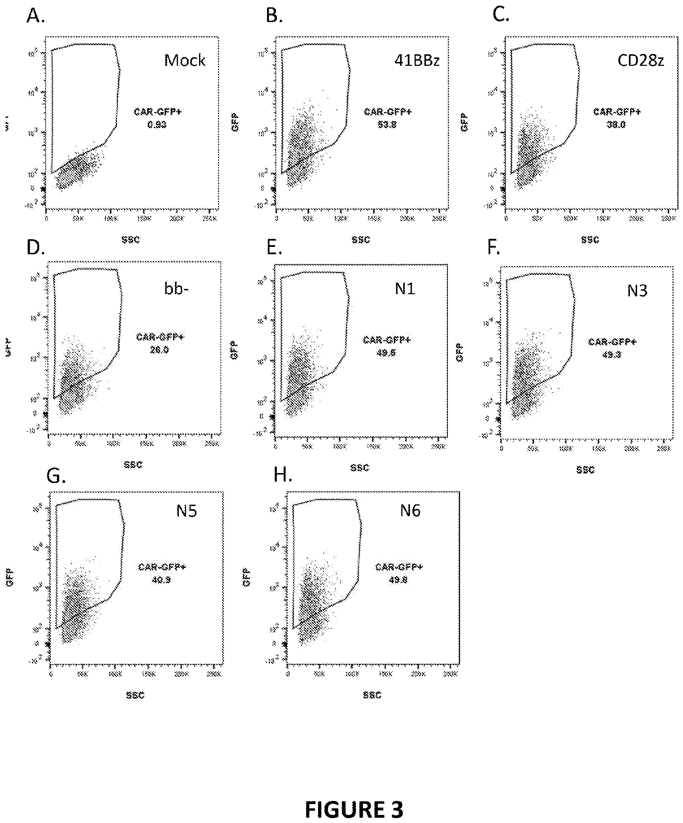

Figures 3A-3H report the results of a GFP analysis that demonstrates CAR expression following lentivirus transduction of donor human T cells. Results are reported for each of the novel co-stimulatory domains Novel1 (N1), Novel3 (N3), Novel5 (N5), and Novel6 (N6). Figure 3A) Mock-transduced. Figure 3B) Transduced with CAR comprising 4-1BB co-stimulatory domain. Figure 3C) Transduced with CAR comprising CD28 co-stimulatory domain. Figure 3D) Transduced with CAR comprising no co-stimulatory domain (BB-). Figure 3E) Transduced with CAR comprising Novel1 (N1) co-stimulatory domain. Figure 3F) Transduced with CAR comprising Novel3 (N3) co-stimulatory domain. Figure 3G) Transduced with CAR comprising Novel5 (N5) co-stimulatory domain. Figure 3H) Transduced with CAR comprising Novel6 (N6) co-stimulatory domain.

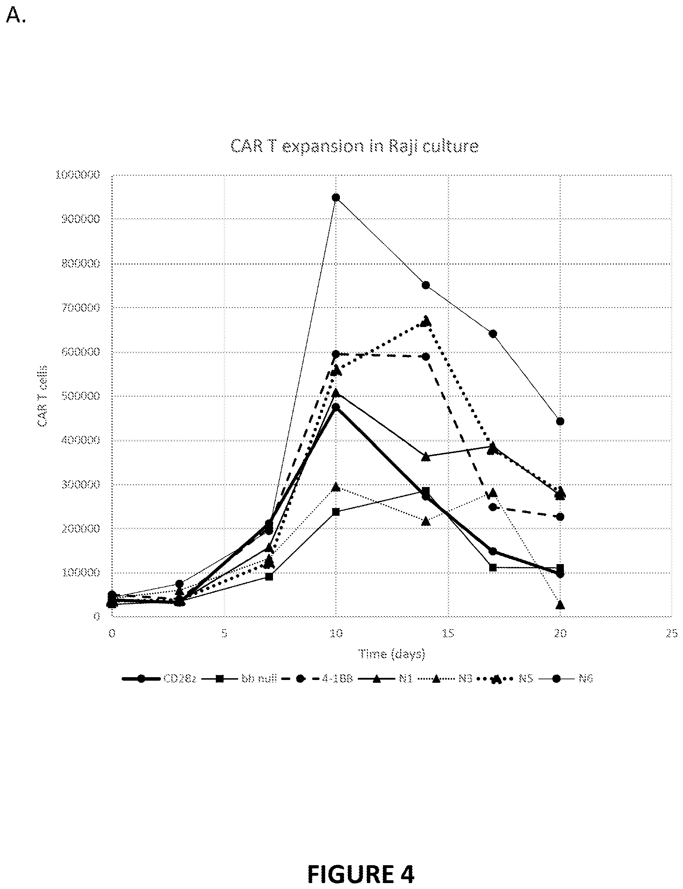

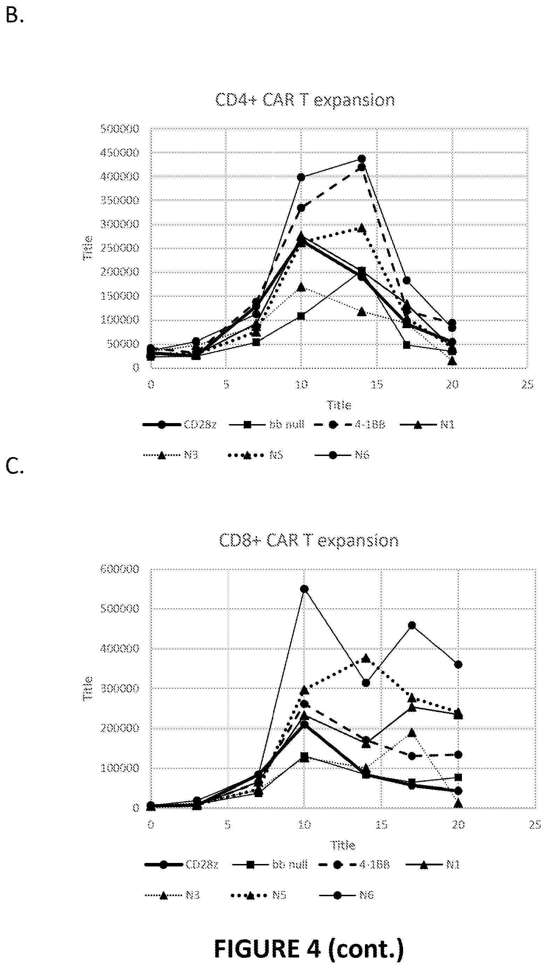

Figure 4A, 4B, and 4C show CAR-T cell expansion numbers measured over time following repeated antigen-induced activation in Raji culture. Results are reported for each of the novel co-stimulatory domains Novel1 (N1), Novel3 (N3), Novel5 (N5), and Novel6 (N6). Figure 4A shows results obtained for mixed population of CD4⁺ and CD8⁺ CAR-T cells. Figure 4B shows results obtained for CD4⁺ CAR-T cell population. Figure 4C shows results obtained for CD8⁺ CAR-T cell population.

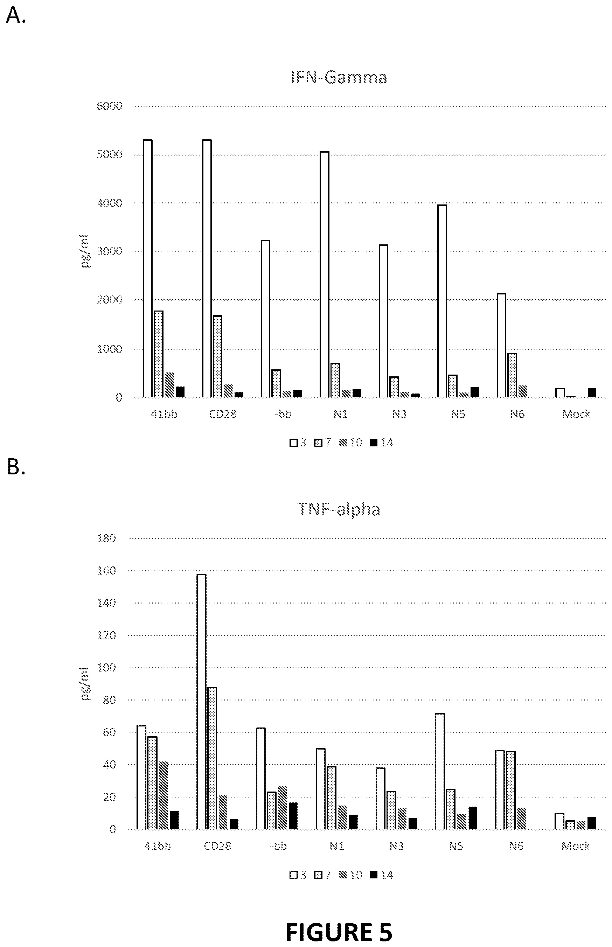

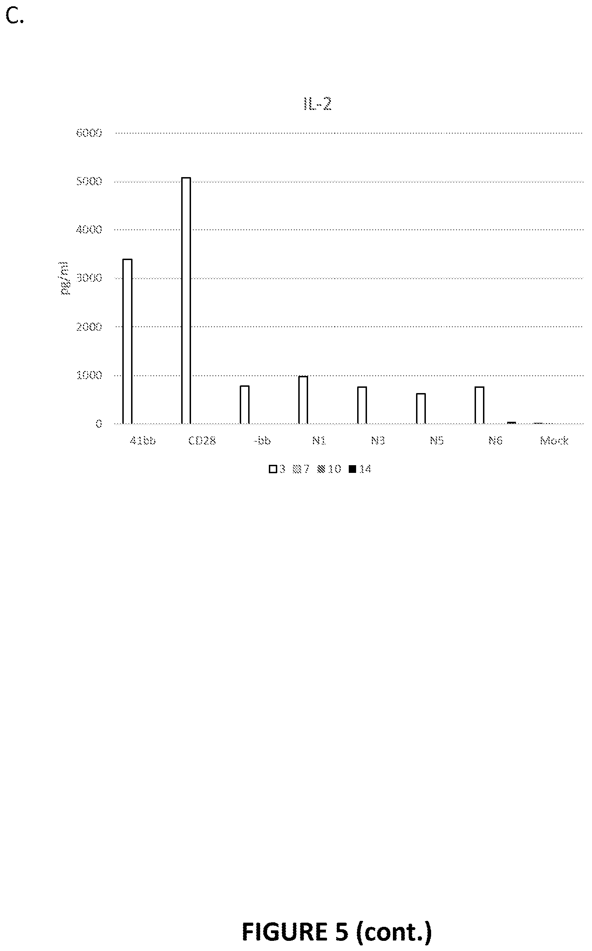

Figure 5A, 5B and 5C show cytokine secretion in each transduced CAR-T cell population at 3, 7, 10, and 14 days post-transduction. Figure 5A shows Interferon-gamma (IFN-γ) secretion. Figure 5B shows TNF-alpha (TNF-α) secretion. Figure 5C shows IL-2 secretion.

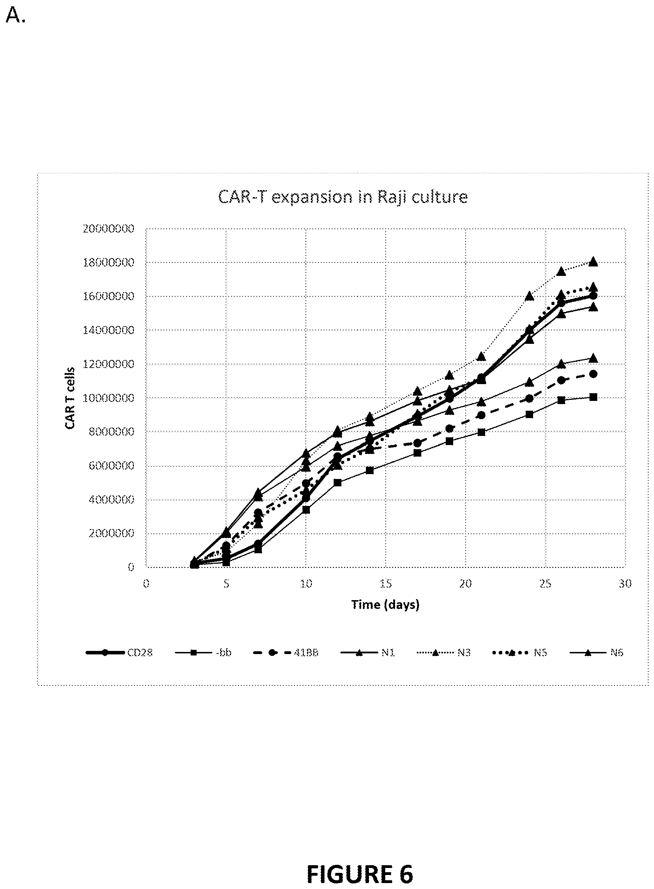

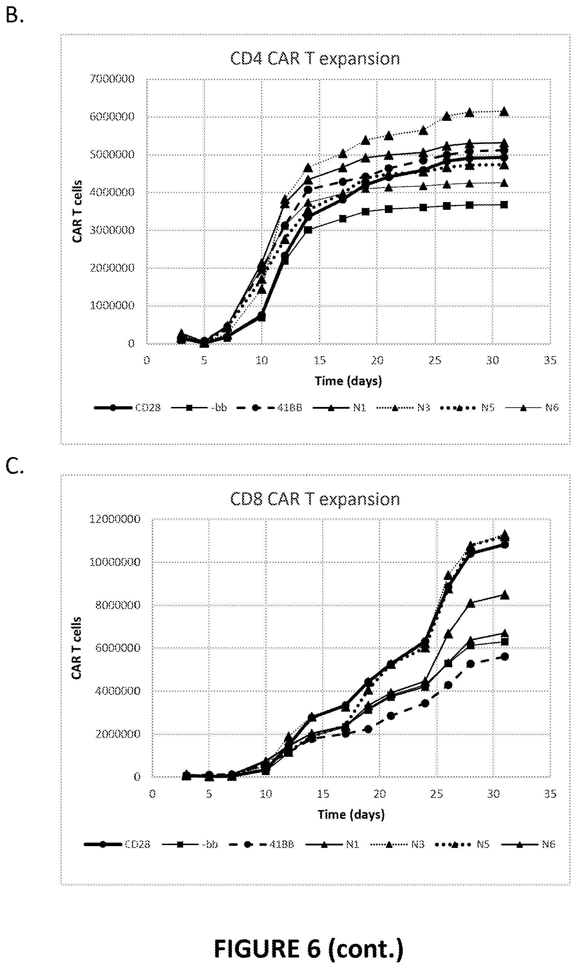

Figure 6A, 6B, and 6C show CAR-T cell expansion numbers measured over time following repeated antigen-induced activation in Raji culture using more frequent antigen encounter and a higher target: effector ratio. Results are reported for each of the novel co-stimulatory domains Novel1 (N1), Novel3 (N3), Novel5 (N5), and Novel6 (N6). Figure 5A shows results obtained for mixed population of CD4⁺ and CD8⁺ CAR-T cells. Figure 5B shows results obtained for CD4⁺ CAR-T cell population. Figure 5C shows results obtained for CD8⁺ CAR-T cell population.

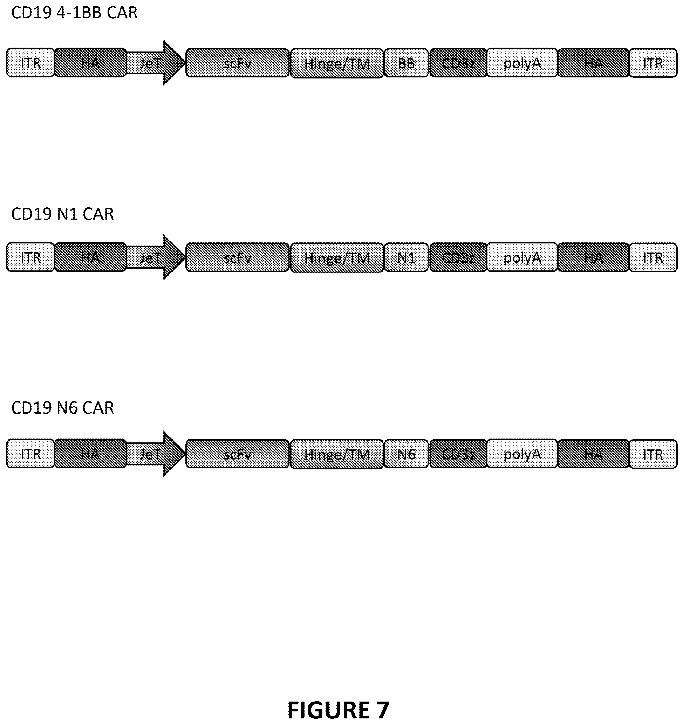

Figure 7 shows donor template constructs comprising, from 5' to 3', a 5' inverted terminal repeat (ITR), a 5' homology arm, a promoter, coding sequences for an anti-CD19 scFv, a CD8 hinge and transmembrane domain, a co-stimulatory domain, and a CD3 ζ intracellular signaling domain, an SV40 polyA signal, a 3' homology arm, and a 3' ITR. The co-stimulatory domains shown include 4-1BB, Novel1 (N1), and Novel6 (N6).

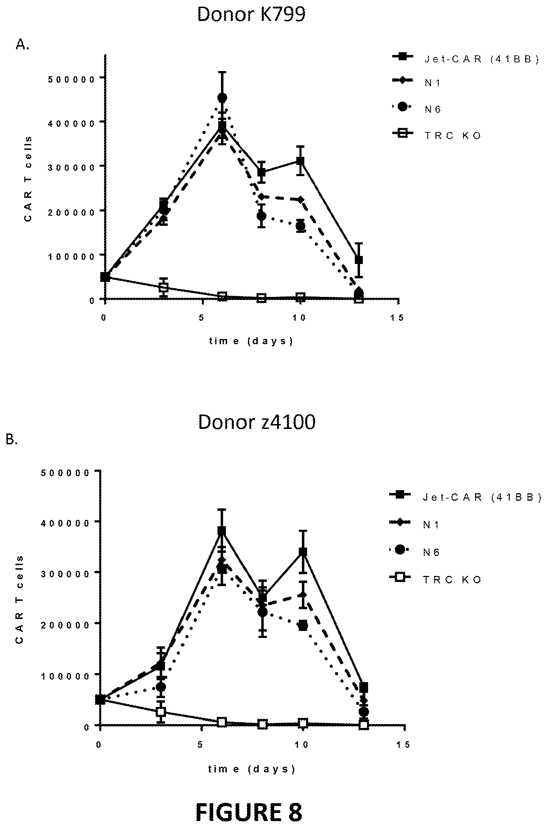

Figure 8A and 8B show CAR-T proliferation over time in response to antigen using CAR-T cells prepared from two different donors. Proliferation was measured for CAR-T cells comprising the 4-1BB, N1, or N6 co-stimulatory domains. Novel costimulatory signaling domains were found to support levels of proliferation that are equal to or better than those levels supported by 4-1BB signaling. Figure 8A shows results using CAR-T cells prepared from Donor K799. Figure 8B shows results using CAR-T cells prepared from donor z4100.

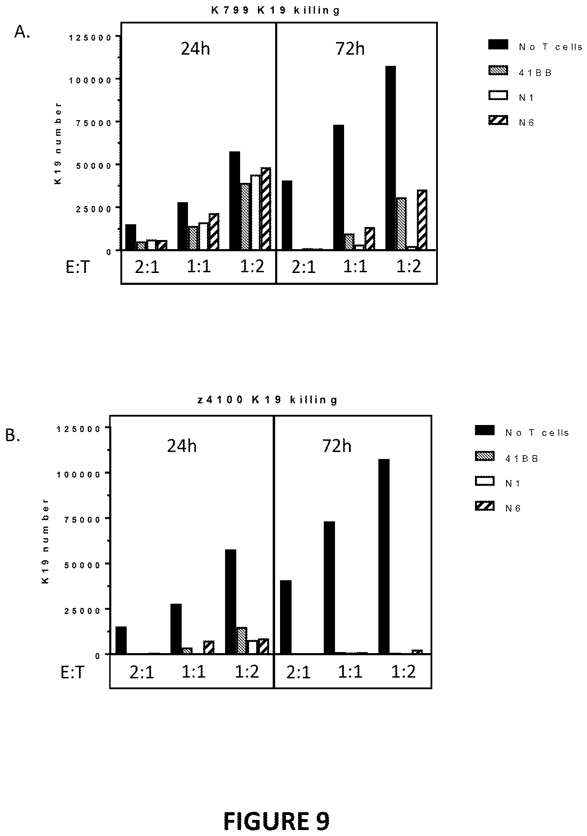

Figure 9A and 9B show killing of K19 cells at various effector:target (E:T) ratios at 24hr and 72hr of coculture with CAR-T cells comprising the 4-1BB, N1, or N6 co-stimulatory domains. Figure 9A shows CAR-T cells prepared from donor K799. Figure 9B shows CAR-T cells prepared from donor z4100.

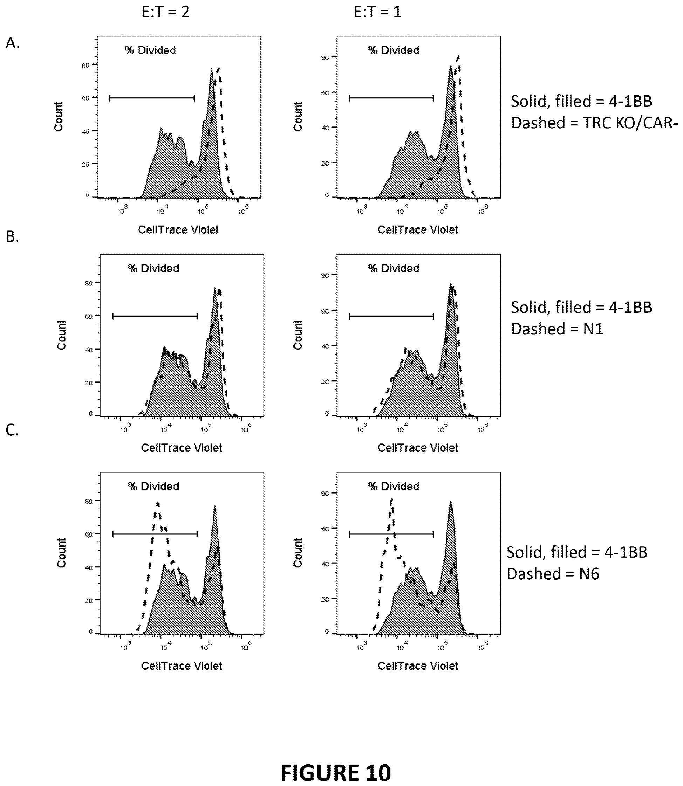

Figure 10A, 10B, and 10C show histograms of the results of the cell proliferation assay used to determine relative proliferation of CAR-T cells comprising the 4-1BB, N1, or N6 co-stimulatory domains in response to CD19+ target cells. Figure 10A shows proliferation of CAR-4-1BB CAR-T cells compared to the negative control TRC KO T cells at two different E:T ratios. Figure 10B shows proliferation of CAR-4-1BB T cells compared to CAR-N1 T cells. Figure 10C shows proliferation of CAR-4-1BB T cells compared to CAR-N6 T cells.

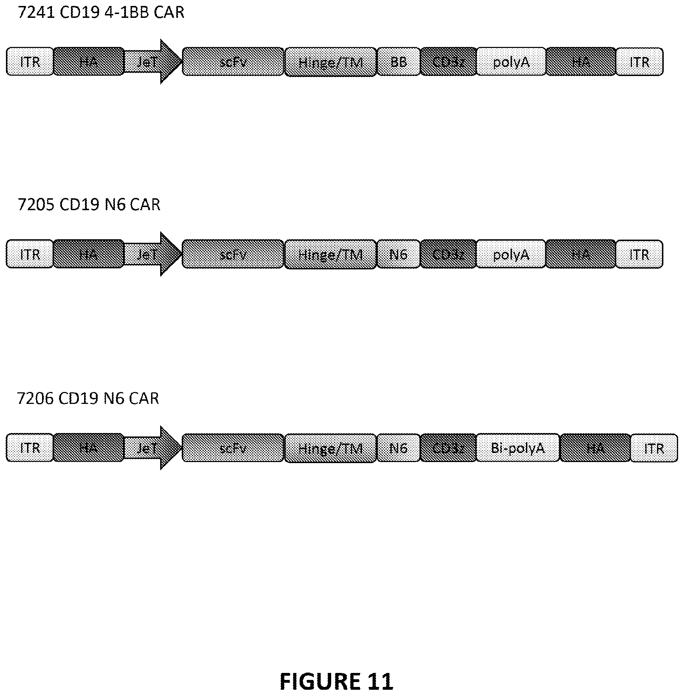

Figure 11 shows the 7241, 7205, and 7206 donor template constructs comprising, from 5' to 3', a 5' inverted terminal repeat (ITR), a 5' homology arm, a promoter, coding sequences for an anti-CD19 scFv, a CD8 hinge and transmembrane domain, a co-stimulatory domain, and a CD3 ζ intracellular signaling domain, an SV40 polyA signal or SV40 bi-polyA signal, a 3' homology arm, and a 3' ITR. The co-stimulatory domains shown include 4-1BB (construct 7241) and Novel 6 (N6; constructs 7205 and 7206).

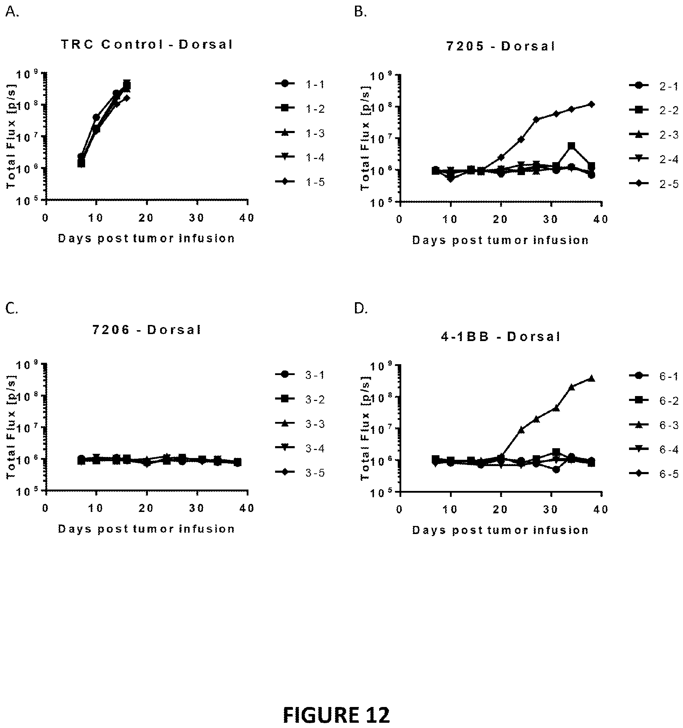

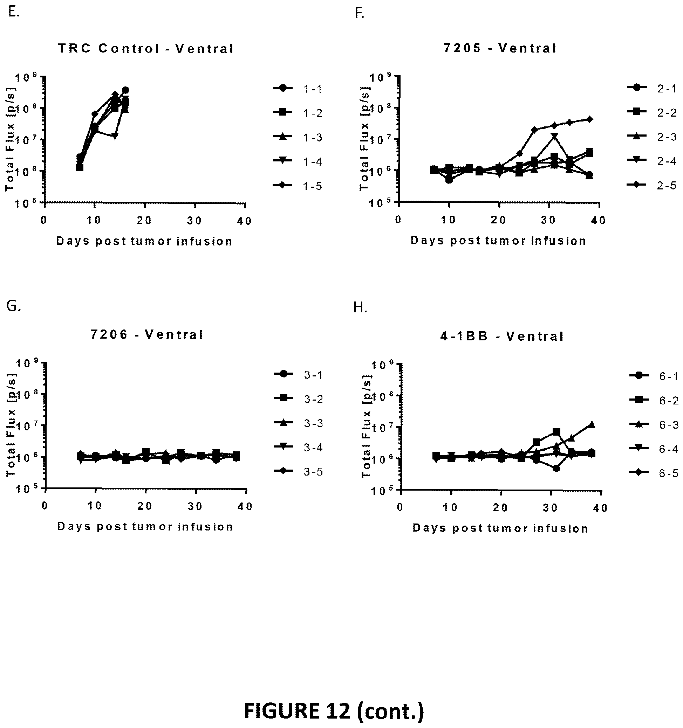

Figures 12A-12G show values of dorsal and ventral total flux observed *in vivo* in mice following engraftment and growth of Raji-ffluc cells and subsequent treatment with TCR KO cells, or CAR T cells bearing the 7205, 7206, or 4-1BB CAR constructs. Figure 12A shows dorsal flux following treatment with TCR KO cells. Figure 12B shows dorsal flux following treatment with 7205 CAR T cells. Figure 12C shows dorsal flux following treatment with 7206 CAR T cells. Figure 12D shows dorsal flux following treatment with 4-1BB CAR T cells. Figure 12E shows ventral flux following treatment with TCR KO cells. Figure 12F shows ventral flux following treatment with 7205 CAR T cells. Figure 12G shows ventral flux following treatment with 7206 CAR T cells. Figure 12H shows ventral flux following treatment with 4-1BB CAR T cells.

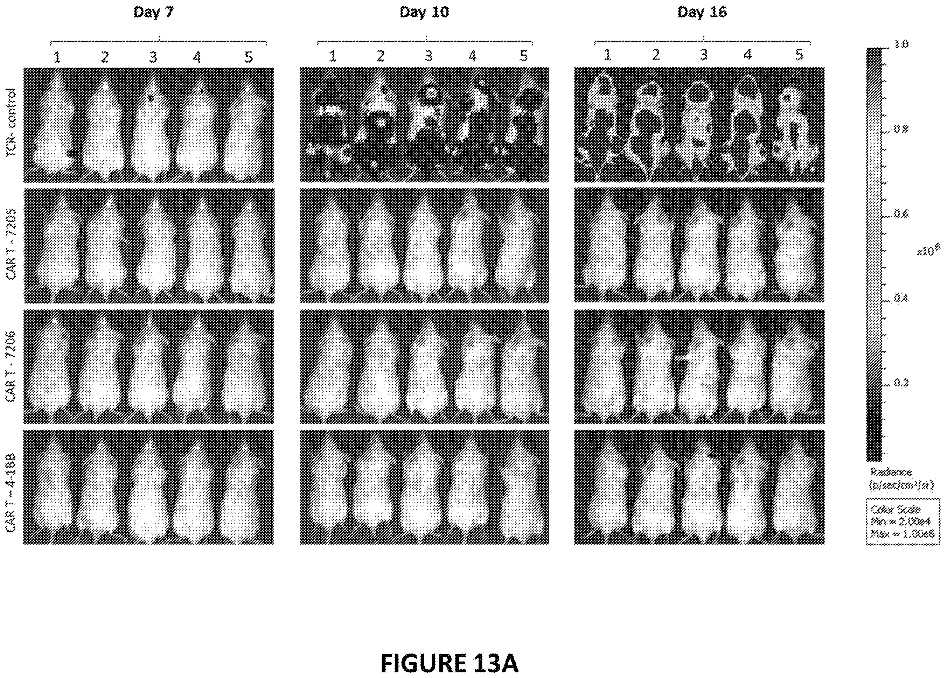

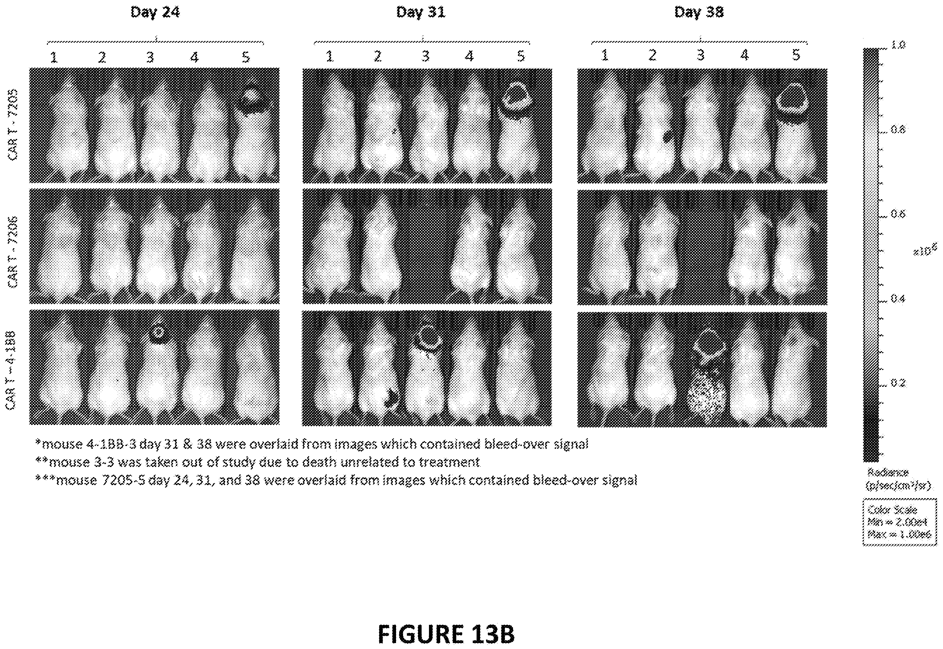

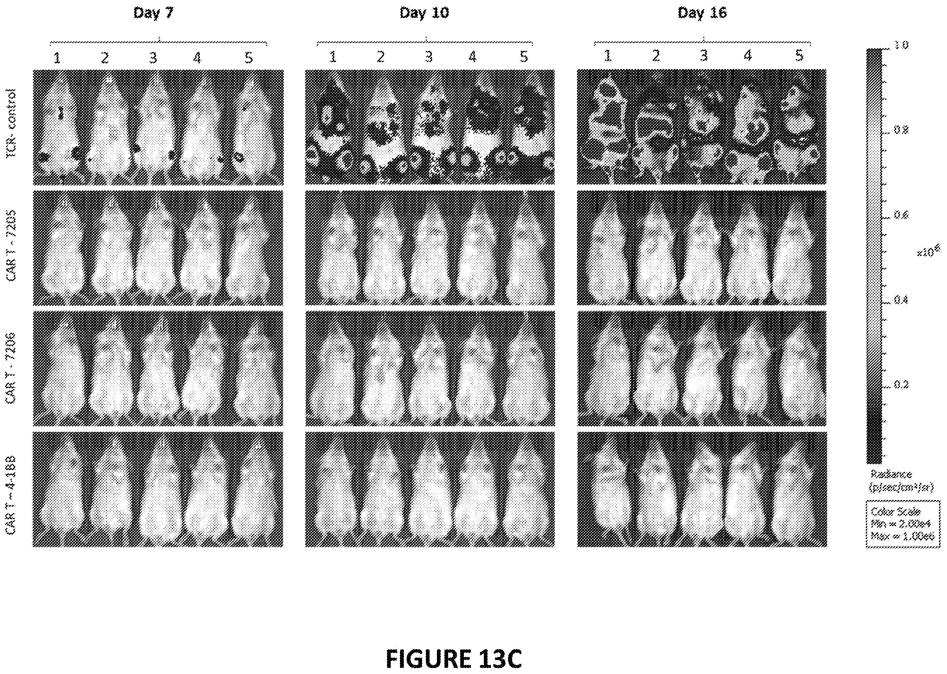

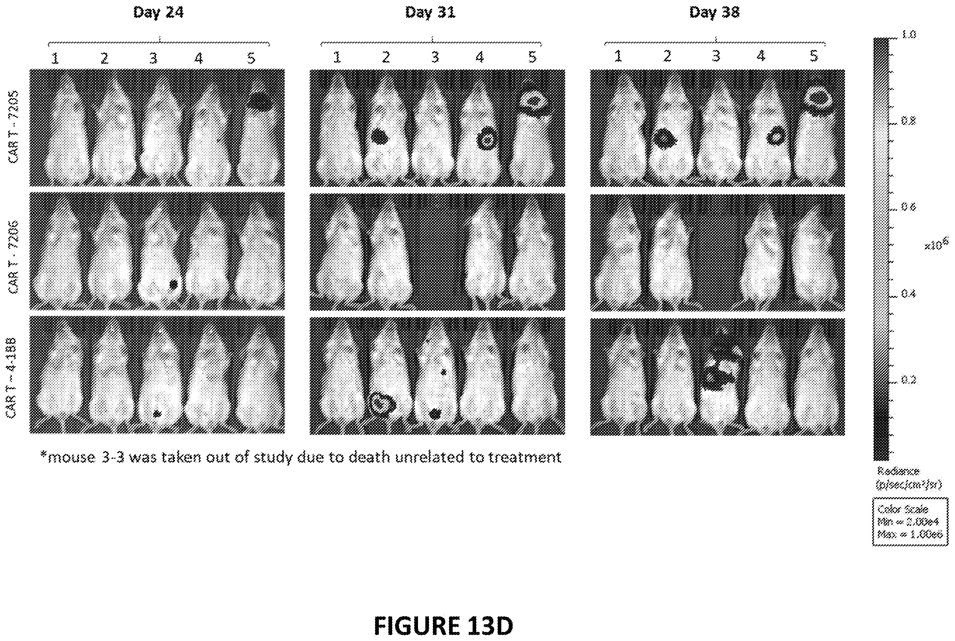

Figures 13A-13D show imaging of dorsal and ventral total flux observed *in vivo* in mice following engraftment and growth of Raji-ffluc cells and subsequent treatment with TCR KO cells, or CAR T cells bearing the 7205, 7206, or 4-1BB CAR constructs. Figure 13A shows imaging of dorsal flux on days 7, 10, and 16 in groups treated with TCR KO cells, 7205 CAR T cells, 7206 CAR T cells, and 4-1BB CAR T cells. Figure 13B shows imaging of dorsal flux on days 24, 31, and 38 in groups treated with 7205 CAR T cells, 7206 CAR T cells, and 4-1BB CAR T cells. Figure 13C shows imaging of ventral flux on days 7, 10, and 16 in groups treated with TCR KO cells, 7205 CAR T cells, 7206 CAR T cells, and 4-1BB CAR T cells. Figure 13D shows imaging of ventral flux on days 24, 31, and 38 in groups treated with 7205 CAR T cells, 7206 CAR T cells, and 4-1BB CAR T cells.

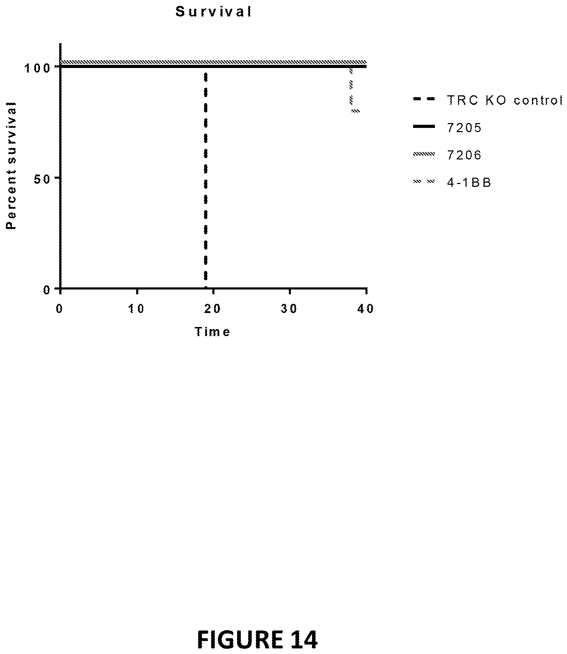

Figure 14 shows a survival curve of mice following engraftment and growth of Raji-ffluc cells and subsequent treatment with TCR KO cells, or CAR T cells bearing the 7205, 7206, or 4-1BB CAR constructs.

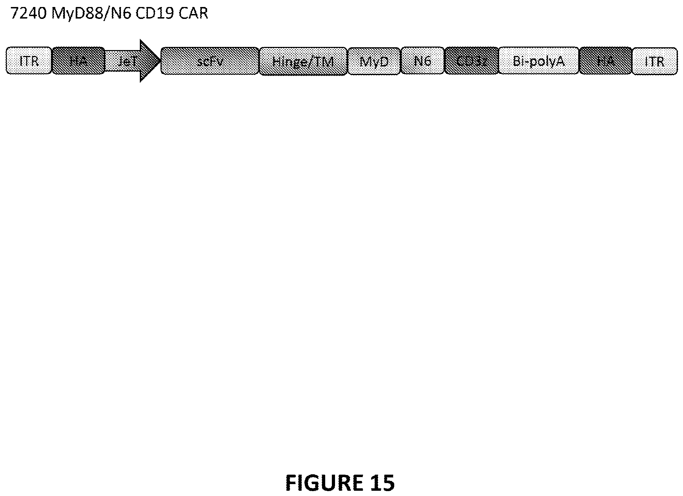

Figure 15 shows the 7240 donor template construct comprising, from 5' to 3', a 5' inverted terminal repeat (ITR), a 5' homology arm, a promoter, coding sequences for an anti-CD19 scFv, a CD8 hinge and transmembrane domain, a MyD88 co-stimulatory domain, a Novel6 (N6) co-stimulatory domain, and a CD3 ζ intracellular signaling domain, an SV40 bi-polyA signal, a 3' homology arm, and a 3' ITR.

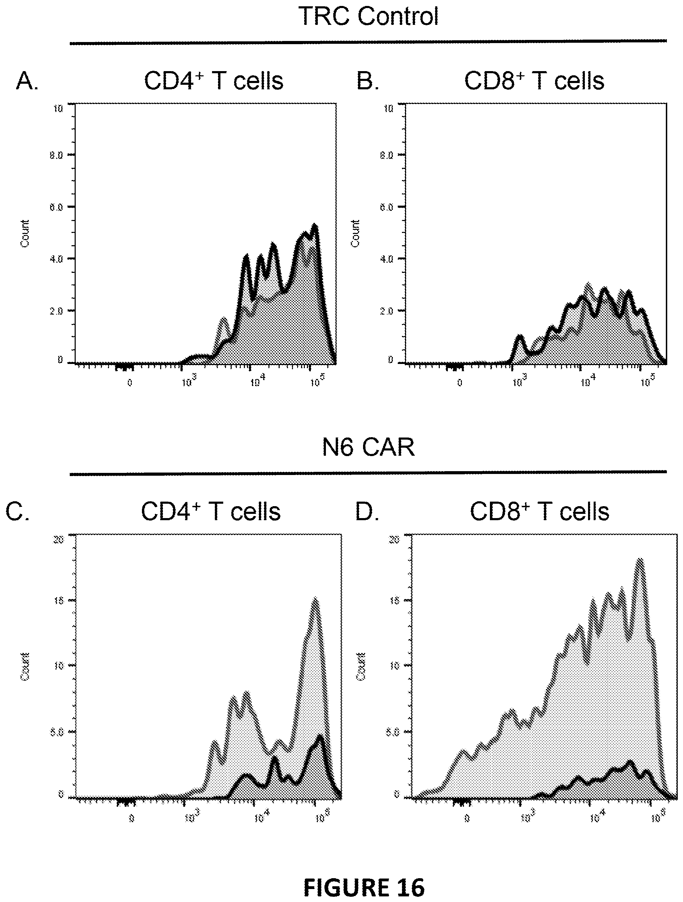

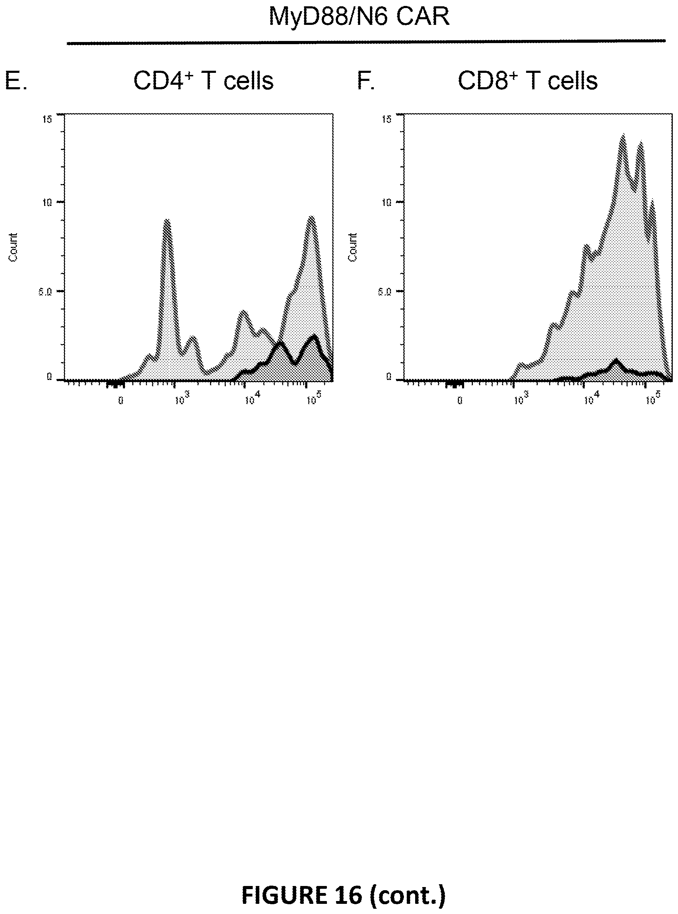

Figures 16A-16F show the results of a Cell Trace Violet proliferation assay using human T cells transfected to express an anti-CD19 CAR comprising the Novel6 (N6) co-stimulatory domain, or a CAR comprising both the MyD88 and Novel6 (N6) co-stimulatory domains. Transfected cells were labeled with Cell Trace Violet and co-cultured with CD19-negative K562 cells or engineered CD19-positive K562 cells (K19 cells). Proliferation was assessed by flow cytometry for CD4+ and CD8+ subsets of T cells. Figure 16A shows CD4+ cells transfected with the TRC 1-2x.87EE meganuclease only. Figure 16B shows CD8+ cells transfected with the TRC 1-2x.87EE meganuclease only. Figure 16C shows CD4+ cells transfected with the TRC 1-2x.87EE meganuclease and an N6 CAR donor template. Figure 16D shows CD8+ cells transfected with the TRC 1-2x.87EE meganuclease and an N6 CAR donor template. Figure 16E shows CD4+ cells transfected with the TRC 1-2x.87EE meganuclease and a MyD88/N6 CAR donor template. Figure 16F shows CD8+ cells transfected with the TRC 1-2x.87EE meganuclease and a MyD88/N6 CAR donor template.

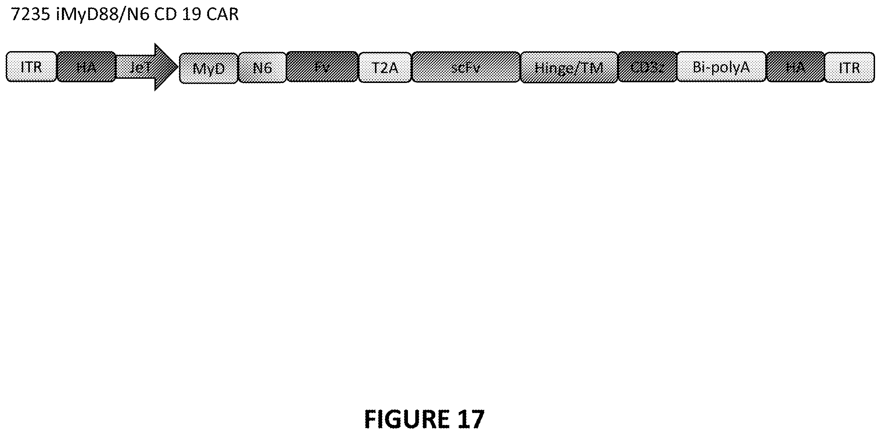

Figure 17 shows the 7235 donor template construct comprising, from 5' to 3', a 5' inverted terminal repeat (ITR), a 5' homology arm, a promoter, coding sequences for a MyD88 co-stimulatory domain, a Novel6 (N6) co-stimulatory domain, and tandem ligand-binding FKBP12v36 domains (Fv), a T2A element, coding sequences for an anti-CD19 scFv, a CD8 hinge and transmembrane domain, and a CD3 ζ intracellular signaling domain, an SV40 bi-polyA signal, a 3' homology arm, and a 3' ITR.

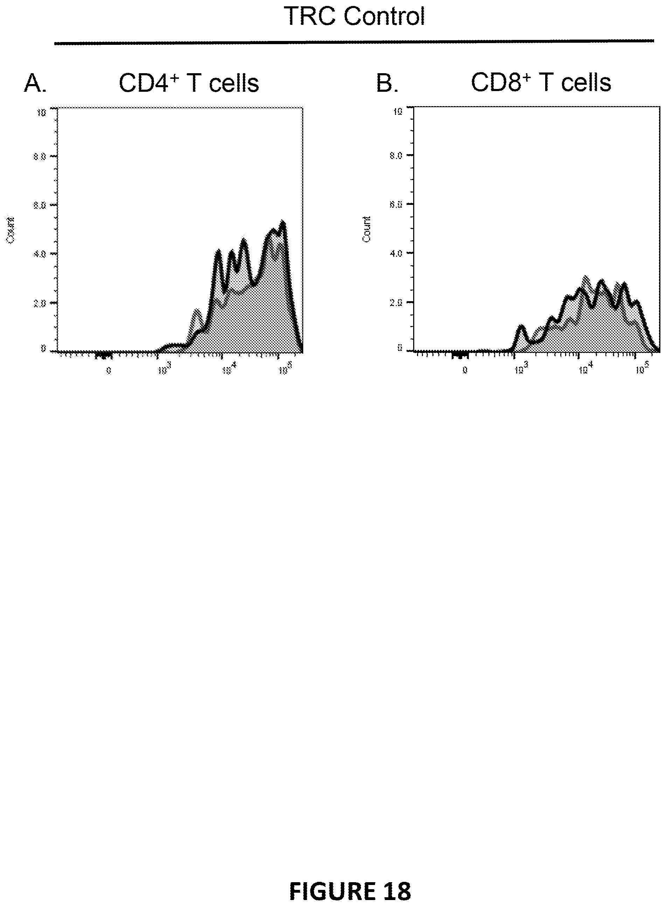

Figures 18A and 18B show the results of a Cell Trace Violet proliferation assay using human T cells transfected to express the TRC 1-2x.87EE meganuclease only without a donor template. Transfected cells were labeled with Cell Trace Violet and co-cultured with CD19-negative K562 cells or engineered CD19-positive K562 cells (K19 cells). Proliferation was assessed by flow cytometry for CD4+ and CD8+ subsets of T cells. Figure 18A shows the CD4+ subset of cells. Figure 18B shows the CD8+ subset of cells.

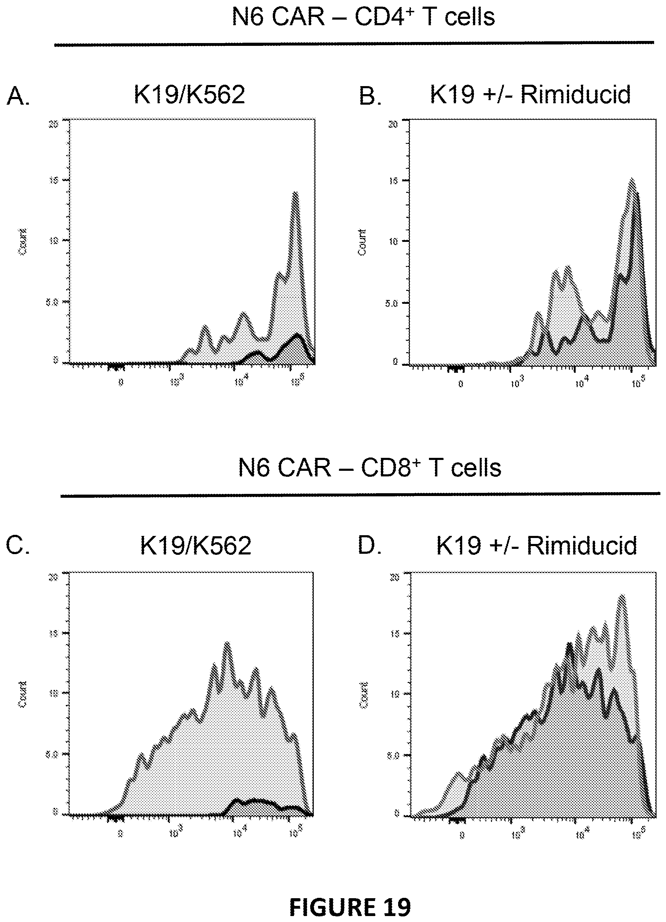

Figures 19A-9D show the show the results of a Cell Trace Violet proliferation assay using human T cells transfected to express an anti-CD19 CAR comprising a Novel6 (N6) domain. Transfected cells were labeled with Cell Trace Violet and co-cultured with CD19-negative K562 cells or engineered CD19-positive K562 cells (K19 cells). Proliferation was assessed by flow cytometry for CD4+ and CD8+ subsets of T cells. Further, cells were assessed in K19 co-culture in the presence or absence of rimiducid. Figure 19A shows the CD4+ subset of cells cultured on K19 or K562 cells. Figure 19B shows the CD4+ subset of cells cultured on K19 cells in the presence or absence of rimiducid. Figure 19C shows the CD8+ subset of cells cultured on K19 or K562 cells. Figure 19D shows the CD8+ subset of cells cultured on K19 cells in the presence or absence of rimiducid.

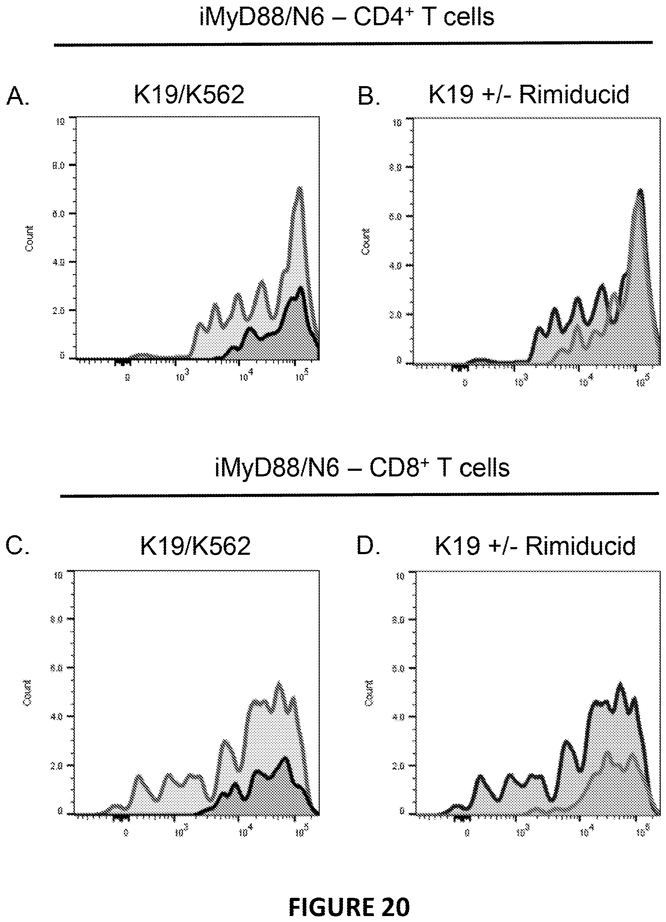

Figures 20A-20D show the show the results of a Cell Trace Violet proliferation assay using human T cells transfected to express an anti-CD19 CAR lacking a co-stimulatory domain in combination with an inducible construct comprising both the MyD88 and the Novel6 (N6) co-stimulatory domains (iMyD88/N6 CAR). Transfected cells were labeled with Cell Trace Violet and co-cultured with CD19-negative K562 cells or engineered CD19-positive K562 cells (K19 cells). Further, cells were assessed in K19 co-culture in the presence or absence of rimiducid. Proliferation was assessed by flow cytometry for CD4+ and CD8+ subsets of T cells. Figure 20A shows the CD4+ subset of cells cultured on K19 or K562 cells. Figure 20B shows the CD4+ subset of cells cultured on K19 cells in the presence or absence of rimiducid. Figure 20C shows the CD8+ subset of cells cultured on K19 or K562 cells. Figure 20D shows the CD8+ subset of cells cultured on K19 cells in the presence or absence of rimiducid.

### BRIEF DESCRIPTION OF THE SEQUENCES

SEQ ID NO:1 sets forth the nucleic acid sequence encoding the Novel1 co-stimulatory domain.
SEQ ID NO:2 sets forth the nucleic acid sequence encoding the Novel3 co-stimulatory domain.
SEQ ID NO:3 sets forth the nucleic acid sequence encoding the Novel5 co-stimulatory domain.
SEQ ID NO:4 sets forth the nucleic acid sequence encoding the Novel6 co-stimulatory domain.
SEQ ID NO:5 sets forth the amino acid sequence of the Novel1 co-stimulatory domain.
SEQ ID NO:6 sets forth the amino acid sequence of the Novel3 co-stimulatory domain.
SEQ ID NO:7 sets forth the amino acid sequence of the Novel5 co-stimulatory domain.
SEQ ID NO:8 sets forth the amino acid sequence of the Novel6 co-stimulatory domain.
SEQ ID NO:9 sets forth the amino acid sequence of a TRAF-binding motif found in a novel co-stimulatory domain.
SEQ ID NO:10 sets forth the amino acid sequence of a TRAF-binding motif found in a novel co-stimulatory domain.
SEQ ID NO:11 sets forth the amino acid sequence of a TRAF-binding motif found in a novel co-stimulatory domain.
SEQ ID NO:12 sets forth the amino acid sequence of a spacer sequence found in a novel co-stimulatory domain.
SEQ ID NO:13 sets forth the amino acid sequence of a spacer sequence found in a novel co-stimulatory domain.
SEQ ID NO:14 sets forth the amino acid sequence of a spacer sequence found in a novel co-stimulatory domain.
SEQ ID NO:15 sets forth the amino acid sequence of a spacer sequence found in a novel co-stimulatory domain.
SEQ ID NO: 16 sets forth the amino acid sequence of a chimeric antigen receptor signal peptide.
SEQ ID NO: 17 sets forth the amino acid sequence of an anti-CD19 chimeric antigen receptor scFv.
SEQ ID NO: 18 sets forth the amino acid sequence of a chimeric antigen receptor CD8 hinge and transmembrane region.
SEQ ID NO: 19 sets forth the amino acid sequence of a CD3-ζ intracellular signaling domain.
SEQ ID NO: 20 sets forth the amino acid sequence of a CD28 co-stimulatory domain.
SEQ ID NO: 21 sets forth the amino acid sequence of a 4-1BB co-stimulatory domain.
SEQ ID NO: 22 sets forth the amino acid sequence of an anti-CD19 chimeric antigen receptor lacking a co-stimulatory domain.
SEQ ID NO: 23 sets forth the amino acid sequence of an anti-CD19 chimeric antigen receptor comprising a CD28 co-stimulatory domain.
SEQ ID NO: 24 sets forth the amino acid sequence of an anti-CD19 chimeric antigen receptor comprising a 4-1BB co-stimulatory domain.
SEQ ID NO: 25 sets forth the amino acid sequence of an anti-CD19 chimeric antigen receptor comprising a Novel1 co-stimulatory domain.
SEQ ID NO: 26 sets forth the amino acid sequence of an anti-CD19 chimeric antigen receptor comprising a Novel3 co-stimulatory domain.
SEQ ID NO: 27 sets forth the amino acid sequence of an anti-CD19 chimeric antigen receptor comprising a Novel5 co-stimulatory domain.
SEQ ID NO: 28 sets forth the amino acid sequence of an anti-CD19 chimeric antigen receptor comprising a Novel6 co-stimulatory domain.
SEQ ID NO: 29 sets forth the nucleic acid sequence of vector encoding an anti-CD19 CAR comprising a 4-1BB co-stimulatory domain.
SEQ ID NO: 30 sets forth the nucleic acid sequence of vector encoding an anti-CD19 CAR comprising a Novel1 co-stimulatory domain.
SEQ ID NO: 31 sets forth the nucleic acid sequence of vector encoding an anti-CD19 CAR comprising a Novel6 co-stimulatory domain.
SEQ ID NO: 32 sets forth the nucleic acid sequence of the JeT promoter.
SEQ ID NO: 33 sets forth the nucleic acid sequence of an SV40 polyA signal sequence.
SEQ ID NO: 34 sets forth the nucleic acid sequence of a first SV40 bi-polyA signal sequence.
SEQ ID NO: 35 sets forth the nucleic acid sequence of a second SV40 bi-polyA signal sequence.
SEQ ID NO: 36 sets forth the nucleic acid sequence of a vector encoding the 7241 anti-CD19 CAR construct comprising a 4-1BB co-stimulatory domain and an SV40 polyA signal.
SEQ ID NO: 37 sets forth the nucleic acid sequence of a vector encoding the 7205 anti-CD19 CAR construct comprising a Novel6 co-stimulatory domain and an SV40 polyA signal.
SEQ ID NO: 38 sets forth the nucleic acid sequence of a vector encoding the 7206 anti-CD19 CAR construct comprising a Novel6 co-stimulatory domain and an SV40 bi-polyA signal.
SEQ ID NO: 39 sets forth the amino acid sequence of a MyD88 co-stimulatory domain.
SEQ ID NO: 40 sets forth the nucleic acid sequence of a vector encoding the 7240 anti-CD19 CAR construct which comprises MyD88 and Novel6 co-stimulatory domains.
SEQ ID NO: 41 sets forth the amino acid sequence of tandem ligand-binding FKBP12v36 domains.
SEQ ID NO: 42 sets forth the nucleic acid sequence of a vector encoding the 7235 anti-CD19 CAR construct which encodes a first generation CAR and an inducible regulatory construct comprising MyD88 and Novel6 co-stimulatory domains and tandem ligand-binding FKBP12v36 domains.

### DETAILED DESCRIPTION OF THE INVENTION

### 1.1 References and Definitions

The patent and scientific literature referred to herein establishes knowledge that is available to those of skill in the art.

The present disclosure can be embodied in different forms and should not be construed as limited to the embodiments set forth herein. Rather, these embodiments are provided so that this disclosure will be thorough and complete, and will fully convey the scope of the present disclosure to those skilled in the art. For example, features illustrated with respect to one embodiment can be incorporated into other embodiments, and features illustrated with respect to a particular embodiment can be deleted from that embodiment. In addition, numerous variations and additions to the embodiments suggested herein will be apparent to those skilled in the art in light of the instant disclosure, which do not depart from the present disclosure.

Unless otherwise defined, all technical and scientific terms used herein have the same meaning as commonly understood by one of ordinary skill in the art to which this disclosure belongs. The terminology used in the description of the present disclosure herein is for the purpose of describing particular embodiments only and is not intended to be limiting of the present disclosure.

As used herein, "a," "an," or "the" can mean one or more than one. For example, "a" cell can mean a single cell or a multiplicity of cells.

As used herein, unless specifically indicated otherwise, the word "or" is used in the inclusive sense of "and/or" and not the exclusive sense of "either/or."

As used herein, a "co-stimulatory domain" refers to a polypeptide domain which transmits an intracellular proliferative and/or cell-survival signal upon activation. Activation of a co-stimulatory domain may occur following homodimerization of two co-stimulatory domain polypeptides. Activation may also occur, for example, following activation of a construct comprising the co-stimulatory domain (*e.g.,* a chimeric antigen receptor or an inducible regulatory construct). Generally, a co-stimulatory domain can be derived from a transmembrane co-stimulatory receptor, particularly from an intracellular portion of a co-stimulatory receptor. Non-limiting examples of co-stimulatory polypeptides include, but are not limited to, those co-stimulatory domains described herein, 4-1BB, CD28, ICOS, OX-40, and CD27.

As used herein, a "chimeric antigen receptor" or "CAR" refers to an engineered receptor that grafts specificity for an antigen or other ligand or molecule onto an immune effector cell (*e.g.,* a T cell or NK cell). A chimeric antigen receptor typically comprises at least an extracellular ligand-binding domain or moiety and an intracellular domain that comprises one or more signaling domains and/or co-stimulatory domains.

In some embodiments, the extracellular ligand-binding domain or moiety is in the form of a single-chain variable fragment (scFv) derived from a monoclonal antibody, which provides specificity for a particular epitope or antigen (*e.g*., an epitope or antigen preferentially present on the surface of a cell, such as a cancer cell or other disease-causing cell or particle). In some embodiments, the scFv is attached via a linker sequence. In some embodiments, the extracellular ligand-binding domain is specific for any antigen or epitope of interest. In some embodiments, the scFv is humanized. In some embodiments, the extracellular domain of a chimeric antigen receptor comprises an autoantigen (see, Payne et al. (2016) Science, Vol. 353 (6295): 179-184), which is recognized by autoantigen-specific B cell receptors on B lymphocytes, thus directing T cells to specifically target and kill autoreactive B lymphocytes in antibody-mediated autoimmune diseases. Such CARs can be referred to as chimeric autoantibody receptors (CAARs), and the incorporation of one or more co-stimulatory domains described herein into such CAARs is encompassed by the present disclosure.

Intracellular signaling domains are cytoplasmic domains which transmit an activation signal to the cell following binding of the extracellular domain. An intracellular signaling domain can be any intracellular signaling domain of interest that is known in the art. Such cytoplasmic signaling domains can include, without limitation, CD3 ζ.

In some embodiments, the intracellular domain also includes one or more intracellular co-stimulatory domains, such as those described herein, which transmit a co-stimulatory signal which promotes cell proliferation, cell survival, and/or cytokine secretion after binding of the extracellular domain. Such intracellular co-stimulatory domains can include, without limitation, any co-stimulatory domain disclosed herein or those domains known in the art, such as, for example, a CD28 domain, a 4-1BB domain, an OX-40 domain, an ICOS domain, or a CD27 domain. In some embodiments, a chimeric antigen receptor further includes additional structural elements, including a transmembrane domain which is attached to the extracellular ligand-binding domain via a hinge or junction sequence.

As used herein, an "inducible regulatory construct" refers to a transmembrane or intracellular construct expressed in a cell that provides an inducible co-stimulatory signal to promote cell proliferation, cell survival, and/or cytokine secretion. Such constructs comprise one or more co-stimulatory domains, such as those described herein and/or others known in the art, which provide a co-stimulatory signal upon activation. In some embodiments, a co-stimulatory signal is induced, for example, by homodimerization of two inducible regulatory construct polypeptides. An inducible regulatory construct generally comprises a binding domain which allows for homodimerization following binding of a small molecule, an antibody, or other molecule that allows for homodimerization of two construct polypeptides.

As used herein, a "co-stimulatory signal" refers to an intracellular signal induced by a co-stimulatory domain that promotes cell proliferation, expansion of a cell population *in vitro* and/or *in vivo,* promotes cell survival, modulates (*e.g.,* upregulates or downregulates) the secretion of cytokines, and/or modulates the production and/or secretion of other immunomodulatory molecules. In some embodiments, a co-stimulatory signal is induced following homodimerization of two co-stimulatory domain polypeptides. In some embodiments, a co-stimulatory signal is induced following activation of a construct comprising the co-stimulatory domain (*e.g.,* a chimeric antigen receptor or an inducible regulatory construct).

As used herein, the term "activation" refers to the state of a cell (*e.g.,* a T cell) that has been sufficiently stimulated to induce detectable effector function. In some embodiments, activation is associated with induced cytokine production and/or induced cell proliferation and expansion.

As used herein, the term "anti-tumor activity" or "anti-tumor effect" refers to a biological effect which can be manifested by a decrease in tumor volume, a decrease in the number of tumor cells, a decrease in the number of metastases, an increase in life expectancy, or amelioration of various physiological symptoms associated with the cancerous condition. An "anti-tumor effect" can also be manifested by the ability of the genetically-modified cells of the present disclosure in prevention of the occurrence of tumor in the first place.

As used herein, with respect to a protein, the term "engineered" or "recombinant" means having an altered amino acid sequence as a result of the application of genetic engineering techniques to nucleic acids which encode the protein, and cells or organisms which express the protein. With respect to a nucleic acid, the term "engineered" or "recombinant" means having an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In accordance with this definition, a protein having an amino acid sequence identical to a naturally-occurring protein, but produced by cloning and expression in a heterologous host, is not considered recombinant.

As used herein, the term "wild-type" refers to the most common naturally-occurring polynucleotide or polypeptide sequence responsible for a given phenotype. Whereas a wild-type allele or polypeptide can confer a normal phenotype in an organism, a mutant or variant allele or polypeptide can, in some instances, confer an altered phenotype.

As used herein with respect to recombinant proteins, the term "modification" means any insertion, deletion or substitution of an amino acid residue in the recombinant sequence relative to a reference sequence (*e.g*., a wild-type or a native sequence).

As used herein, the term "homologous recombination" or "HR" refers to the natural, cellular process in which a double-stranded DNA-break is repaired using a homologous DNA sequence as a donor template (see, *e.g.* Cahill et al. (2006), Front. Biosci. 11:1958-1976). The homologous DNA sequence may be an endogenous chromosomal or episomal sequence or an exogenous nucleic acid that was delivered to the cell.

As used herein, the term "non-homologous end-joining" or "NHEJ" refers to the natural, cellular process in which a double-stranded DNA-break is repaired by the direct joining of two non-homologous DNA segments (see, *e.g.* Cahill et al. (2006), Front. Biosci. 11:1958-1976).

As used herein, the term "reduced" refers to any reduction in the symptoms or severity of a disease or any reduction in the proliferation or number of cancerous cells. In either case, such a reduction may be up to 5%, 10%, 20%, 30%, 40%, 50%, 60%, 70%, 80%, 90%, 95%, or up to 100%. Accordingly, the term "reduced" encompasses both a partial reduction and a complete reduction of a disease state.

As used herein, the term "increased" refers to any increase in the activation, proliferation, or cytokine signaling of a cell genetically-modified to comprise a co-stimulatory domain disclosed herein, or an active fragment or variant thereof. Such an increase may be up to 5%, 10%, 20%, 30%, 40%, 50%, 60%, 70%, 80%, 90%, 95%, or up to 100%, or more. Any method can be used to measure an increase in the activation, proliferation, or cytokine signaling of a cell. For example, increased activation and/or cytokine expression can encompass an increase of expression of any one of IFN-γ, IL-2, TNF-α, or any other cytokine that could be used to determine a change in cell activation and/or proliferation. In some embodiments, an increase in proliferation encompasses an increase in cell number or cell division, and includes an expansion of a cell population.

As used herein with respect to both amino acid sequences and nucleic acid sequences, the terms "percent identity," "sequence identity," "percentage similarity," "sequence similarity," and the like, refer to a measure of the degree of similarity of two sequences based upon an alignment of the sequences which maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov), and are described in, for example, Altschul et al. (1990), J. Mol. Biol. 215:403-410; Gish and States (1993), Nature Genet. 3:266-272; Madden et al. (1996), Meth. Enzymol.266:131-141*;* Altschul et al. (1997), Nucleic Acids Res. 25:33 89-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3.

As used herein with respect to modifications of two proteins or amino acid sequences, the term "corresponding to" is used to indicate that a specified modification in the first protein is a substitution of the same amino acid residue as in the modification in the second protein, and that the amino acid position of the modification in the first proteins corresponds to or aligns with the amino acid position of the modification in the second protein when the two proteins are subjected to standard sequence alignments (e.g., using the BLASTp program). Thus, the modification of residue "X" to amino acid "A" in the first protein will correspond to the modification of residue "Y" to amino acid "A" in the second protein if residues X and Y correspond to each other in a sequence alignment, and despite the fact that X and Y may be different numbers.

The terms "recombinant DNA construct," "recombinant construct," "expression cassette," "expression construct," "chimeric construct," "construct," and "recombinant DNA fragment" are used interchangeably herein and are linear or circular nucleic acid molecules. A recombinant construct comprises an artificial or non-naturally-occurring combination of nucleic acid molecules, including, without limitation, regulatory and coding sequences. Although the recombinant construct as a whole does not occur in nature, portions of the construct may be found in nature. For example, a recombinant DNA construct may comprise regulatory sequences and coding sequences that are derived from different sources, or regulatory sequences and coding sequences derived from the same source and arranged in a manner different than that found in nature. Such a construct may be used by itself or may be used in conjunction with a vector.

As used herein, a "vector" or "recombinant DNA vector" may be a construct that includes a replication system and sequences that are capable of transcription and translation of a polypeptide-encoding sequence in a given host cell. If a vector is used then the choice of vector is dependent upon the method that will be used to transform host cells as is well known to those skilled in the art. Vectors can include, without limitation, plasmid vectors and recombinant lentiviral or recombinant AAV vectors, or any other vector known in that art suitable for delivering a gene encoding a co-stimulatory domain of the present disclosure to a target cell. The skilled artisan is well aware of the genetic elements that must be present on the vector in order to successfully transform, select and propagate host cells comprising any of the isolated nucleotides or nucleic acid sequences of the present disclosure.

As used herein, a "vector" can also refer to a viral vector. Viral vectors can include, without limitation, retroviral vectors, lentiviral vectors, adenoviral vectors, and adeno-associated viral vectors (AAV).

As used herein, the term "operably linked" is intended to mean a functional linkage between two or more elements. For example, an operable linkage between a nucleic acid sequence encoding a nuclease as disclosed herein and a regulatory sequence (*e.g*., a promoter) is a functional link that allows for expression of the nucleic acid sequence encoding the nuclease. Operably linked elements may be contiguous or non-contiguous. When used to refer to the joining of two protein coding regions, by operably linked is intended that the coding regions are in the same reading frame.

As used herein, "transfected" or "transformed" or "transduced" or "nucleofected" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

As used herein, a "human T cell" or "T cell" refers to a T cell isolated from a human donor. Human T cells, and cells derived therefrom, include isolated T cells that have not been passaged in culture, T cells that have been passaged and maintained under cell culture conditions without immortalization, and T cells that have been immortalized and can be maintained under cell culture conditions indefinitely.

As used herein, a "human natural killer cell" or "human NK cell" or "natural killer cell" or "NK cell" refers to a type of cytotoxic lymphocyte critical to the innate immune system. The role NK cells play is analogous to that of cytotoxic T-cells in the vertebrate adaptive immune response. NK cells provide rapid responses to virally infected cells and respond to tumor formation, acting at around 3 days after infection. Human NK cells, and cells derived therefrom, include isolated NK cells that have not been passaged in culture, NK cells that have been passaged and maintained under cell culture conditions without immortalization, and NK cells that have been immortalized and can be maintained under cell culture conditions indefinitely.

As used herein, a "control" or "control cell" refers to a cell that provides a reference point for measuring changes in genotype or phenotype of a genetically-modified cell. A control cell may comprise, for example: (a) a wild-type cell, *i.e.,* of the same genotype as the starting material for the genetic alteration which resulted in the genetically-modified cell; (b) a cell of the same genotype as the genetically-modified cell but which has been transformed with a null construct (*i*.*e*., with a construct which has no known effect on the trait of interest); or, (c) a cell genetically identical to the genetically-modified cell but which is not exposed to conditions, stimuli, or further genetic modifications that would induce expression of altered genotype or phenotype.

The term "effective amount" or "therapeutically effective amount" refers to an amount sufficient to effect beneficial or desirable biological and/or clinical results. The amount will vary depending on the therapeutic (*e.g*., genetically-modified cell, CAR-T cell, CAR-NK cell) formulation or composition, the disease and its severity, and the age, weight, physical condition and responsiveness of the subject to be treated. In specific embodiments, an effective amount of a cell comprising a co-stimulatory domain disclosed herein or pharmaceutical compositions disclosed herein reduces at least one symptom or the progression of a disease.

As used herein, the term "treat" or "treatment" means to reduce the frequency or severity of at least one sign or symptom of a disease or disorder experienced by a subject.

As used herein, the term "cancer" should be understood to encompass any neoplastic disease (whether or not invasive or metastatic) which is characterized by abnormal cell growth. Invasive or metastatic caners have the potential to spread to other parts of the body. Cancers with uncontrolled cell division can cause malignant growth or tumors whereas cancers with slowly dividing cells can cause benign growth or tumors.

As used herein, the term "carcinoma" refers to a malignant growth made up of epithelial cells.

As used herein, the term "leukemia" refers to malignancies of the hematopoietic organs/systems and is generally characterized by an abnormal proliferation and development of leukocytes and their precursors in the blood and bone marrow.

As used herein, the term "sarcoma" refers to a tumor which is made up of a substance like the embryonic connective tissue and is generally composed of closely packed cells embedded in a fibrillary, heterogeneous, or homogeneous substance.

As used herein, the term "melanoma" refers to a tumor arising from the melanocytic system of the skin and other organs.

As used herein, the term "lymphoma" refers to a group of blood cell tumors that develop from lymphocytes.

As used herein, the term "blastoma" refers to a type of cancer that is caused by malignancies in precursor cells or blasts (immature or embryonic tissue).

As used herein, the term "meganuclease" refers to an endonuclease that binds double-stranded DNA at a recognition sequence that is greater than 12 base pairs. In some embodiments, the recognition sequence for a meganuclease of the present disclosure is 22 base pairs. A meganuclease can be an endonuclease that is derived from I-CreI, and can refer to an engineered variant of I-CreI that has been modified relative to natural I-CreI with respect to, for example, DNA-binding specificity, DNA cleavage activity, DNA-binding affinity, or dimerization properties. Methods for producing such modified variants of I-CreI are known in the art (*e.g.* WO 2007/047859). A meganuclease as used herein binds to double-stranded DNA as a heterodimer. A meganuclease may also be a "single-chain meganuclease" in which a pair of DNA-binding domains are joined into a single polypeptide using a peptide linker. The term "homing endonuclease" is synonymous with the term "meganuclease." Meganucleases of the present disclosure are substantially non-toxic when expressed in cells, particularly in human T cells, such that cells can be transfected and maintained at 37°C without observing deleterious effects on cell viability or significant reductions in meganuclease cleavage activity when measured using the methods described herein.

As used herein, the term "single-chain meganuclease" refers to a polypeptide comprising a pair of nuclease subunits joined by a linker. A single-chain meganuclease has the organization: N-terminal subunit - Linker - C-terminal subunit. The two meganuclease subunits will generally be non-identical in amino acid sequence and will recognize non-identical DNA sequences. Thus, single-chain meganucleases typically cleave pseudo-palindromic or non-palindromic recognition sequences. A single-chain meganuclease may be referred to as a "single-chain heterodimer" or "single-chain heterodimeric meganuclease" although it is not, in fact, dimeric. For clarity, unless otherwise specified, the term "meganuclease" can refer to a dimeric or single-chain meganuclease.

As used herein, the term "linker" refers to an exogenous peptide sequence used to join two meganuclease subunits into a single polypeptide. A linker may have a sequence that is found in natural proteins, or may be an artificial sequence that is not found in any natural protein. A linker may be flexible and lacking in secondary structure or may have a propensity to form a specific three-dimensional structure under physiological conditions. A linker can include, without limitation, any of those encompassed by U.S. Patent Nos. 8,445,251 and 9,434,931.

As used herein, the term "zinc finger nuclease" or "ZFN" refers to chimeric proteins comprising a zinc finger DNA-binding domain fused to a nuclease domain from an endonuclease or exonuclease, including but not limited to a restriction endonuclease, homing endonuclease, S1 nuclease, mung bean nuclease, pancreatic DNAse I, micrococcal nuclease, and yeast HO endonuclease. Nuclease domains useful for the design of zinc finger nuclease include those from a Type IIs restriction endonuclease, including but not limited to FokI, FoM, StsI restriction enzyme. Additional Type IIs restriction endonucleases are described in International Publication No. WO 2007/014275. The structure of a zinc finger domain is stabilized through coordination of a zinc ion. DNA binding proteins comprising one or more zinc finger domains bind DNA in a sequence-specific manner. The zinc finger domain can be a native sequence or can be redesigned through rational or experimental means to produce a protein which binds to a pre-determined DNA sequence ~18 basepairs in length. See, for example, U.S. Pat. Nos. 5,789,538, 5,925,523, 6,007,988, 6,013,453, 6,200,759, and International Publication Nos. WO 95/19431, WO 96/06166, WO 98/53057, WO 98/54311, WO 00/27878, WO 01/60970, WO 01/88197, and WO 02/099084. By fusing this engineered protein domain to a nuclease domain, such as FokI nuclease, it is possible to target DNA breaks with genome-level specificity. The selection of target sites, zinc finger proteins and methods for design and construction of zinc finger nucleases are known to those of skill in the art and are described in detail in U.S. Publications Nos. 20030232410, 20050208489, 2005064474, 20050026157, 20060188987 and International Publication No. WO 07/014275.

As used herein, the term "TALEN" refers to an endonuclease comprising a DNA-binding domain comprising a plurality of TAL domain repeats fused to a nuclease domain or an active portion thereof from an endonuclease or exonuclease, including but not limited to a restriction endonuclease, homing endonuclease, S1 nuclease, mung bean nuclease, pancreatic DNAse I, micrococcal nuclease, and yeast HO endonuclease. *See,* for example, Christian et al. (2010) Genetics 186:757-761. Nuclease domains useful for the design of TALENs include those from a Type IIs restriction endonuclease, including but not limited to FokI, FoM, StsI, HhaI, HindIII, Nod, BbvCI, EcoRI, BglI, and AlwI. Additional Type IIs restriction endonucleases are described in International Publication No. WO 2007/014275. In some embodiments, the nuclease domain of the TALEN is a FokI nuclease domain or an active portion thereof. TAL domain repeats can be derived from the TALE (transcription activator-like effector) family of proteins used in the infection process by plant pathogens of the *Xanthomonas* genus. TAL domain repeats are 33-34 amino acid sequences with divergent 12^{th} and 13^{th} amino acids. These two positions, referred to as the repeat variable dipeptide (RVD), are highly variable and show a strong correlation with specific nucleotide recognition. Each base pair in the DNA target sequence is contacted by a single TAL repeat, with the specificity resulting from the RVD. In some embodiments, the TALEN comprises 16-22 TAL domain repeats. DNA cleavage by a TALEN requires two DNA recognition regions flanking a nonspecific central region (*i.e.,* the "spacer"). The term "spacer" in reference to a TALEN refers to the nucleic acid sequence that separates the two nucleic acid sequences recognized and bound by each monomer constituting a TALEN. The TAL domain repeats can be native sequences from a naturally-occurring TALE protein or can be redesigned through rational or experimental means to produce a protein which binds to a pre-determined DNA sequence (see, for example, Boch et al. (2009) Science 326(5959):1509-1512 and Moscou and Bogdanove (2009) Science 326(5959):1501). See also, U.S. Publication No. 20110145940 and International Publication No. WO 2010/079430 for methods for engineering a TALEN to recognize a specific sequence and examples of RVDs and their corresponding target nucleotides. In some embodiments, each nuclease (*e.g*., FokI) monomer can be fused to a TAL effector sequence that recognizes a different DNA sequence, and only when the two recognition sites are in close proximity do the inactive monomers come together to create a functional enzyme.

As used herein, the term "compact TALEN" refers to an endonuclease comprising a DNA-binding domain with one or more TAL domain repeats fused in any orientation to any portion of the I-TevI homing endonuclease or any of the endonucleases listed in Table 2 in U.S. Application No. 20130117869, including but not limited to MmeI, EndA, End1, I-BasI, I-TevII, I-TevIII, I-TwoI, MspI, MvaI, NucA, and NucM. Compact TALENs do not require dimerization for DNA processing activity, alleviating the need for dual target sites with intervening DNA spacers. In some embodiments, the compact TALEN comprises 16-22 TAL domain repeats.

As used herein, the term "CRISPR" refers to a caspase-based endonuclease comprising a caspase, such as Cas9, and a guide RNA that directs DNA cleavage of the caspase by hybridizing to a recognition site in the genomic DNA. The caspase component of a CRISPR is an RNA-guided DNA endonuclease. In certain embodiments, the caspase is a class II Cas enzyme. In some of these embodiments, the caspase is a class II, type II enzyme, such as Cas9. In other embodiments, the caspase is a class II, type V enzyme, such as Cpf1. The guide RNA comprises a direct repeat and a guide sequence (often referred to as a spacer in the context of an endogenous CRISPR system), which is complementary to the target recognition site. In certain embodiments, the CRISPR further comprises a tracrRNA (trans-activating CRISPR RNA) that is complementary (fully or partially) to a direct repeat sequence (sometimes referred to as a tracrmate sequence) present on the guide RNA. In particular embodiments, the caspase can be mutated with respect to a corresponding wild-type enzyme such that the enzyme lacks the ability to cleave one strand of a target polynucleotide, functioning as a nickase, cleaving only a single strand of the target DNA. Non-limiting examples of caspase enzymes that function as a nickase include Cas9 enzymes with a D10A mutation within the RuvC I catalytic domain, or with a H840A, N854A, or N863A mutation.

As used herein, the term "megaTAL" refers to a single-chain nuclease comprising a transcription activator-like effector (TALE) DNA binding domain with an engineered, sequence-specific homing endonuclease.

As used herein, the term "recognition sequence" refers to a DNA sequence that is bound and cleaved by an endonuclease. In the case of a meganuclease, a recognition sequence comprises a pair of inverted, 9 base pair "half sites" which are separated by four basepairs. In the case of a single-chain meganuclease, the N-terminal domain of the protein contacts a first half-site and the C-terminal domain of the protein contacts a second half-site. Cleavage by a meganuclease produces four base pair 3' "overhangs". "Overhangs," or "sticky ends" are short, single-stranded DNA segments that can be produced by endonuclease cleavage of a double-stranded DNA sequence. In the case of meganucleases and single-chain meganucleases derived from I-CreI, the overhang comprises bases 10-13 of the 22 base pair recognition sequence. In the case of a compact TALEN, the recognition sequence can comprises a first CNNNGN sequence that is recognized by the I-TevI domain, followed by a non-specific spacer 4-16 basepairs in length, followed by a second sequence 16-22 bp in length that is recognized by the TAL-effector domain (this sequence typically has a 5' T base). Cleavage by a Compact TALEN produces two base pair 3' overhangs. In the case of a CRISPR, the recognition sequence is the sequence, typically 16-24 basepairs, to which the guide RNA binds to direct Cas9 cleavage. Full complementarity between the guide sequence and the recognition sequence is not necessarily required to effect cleavage. Cleavage by a CRISPR can produce blunt ends (such as by a class II, type II caspase) or overhanging ends (such as by a class II, type V caspase), depending on the caspase. In those embodiments wherein a CpfI caspase is utilized, cleavage by the CRISPR complex comprising the same will result in 5' overhangs and in certain embodiments, 5 nucleotide 5' overhangs. Each caspase enzyme also requires the recognition of a PAM (protospacer adjacent motif) sequence that is near the recognition sequence complementary to the guide RNA. The precise sequence, length requirements for the PAM, and distance from the target sequence differ depending on the caspase enzyme, but PAMs are typically 2-5 base pair sequences adjacent to the target/recognition sequence. PAM sequences for particular caspase enzymes are known in the art (see, for example, U.S. Patent No. 8,697,359 and U.S. Publication No. 20160208243) and PAM sequences for novel or engineered caspase enzymes can be identified using methods known in the art, such as a PAM depletion assay (see, for example, Karvelis et al. (2017) Methods 121-1223-8).

As used herein, the term "target site" or "target sequence" refers to a region of the chromosomal DNA of a cell comprising a recognition sequence for a nuclease.

As used herein, the terms "T cell receptor alpha gene" or "TCR alpha gene" are interchangeable and refer to the locus in a T cell which encodes the T cell receptor alpha subunit. The T cell receptor alpha can refer to NCBI gene ID number 6955, before or after rearrangement. Following rearrangement, the T cell receptor alpha gene comprises an endogenous promoter, rearranged V and J segments, an endogenous splice donor site, an intron, an endogenous splice acceptor site, and the T cell receptor alpha constant region locus, which comprises the subunit coding exons.

As used herein, the term "T cell receptor alpha constant region gene" refers to the coding sequence of the T cell receptor alpha gene. The T cell receptor alpha constant region gene includes the wild-type sequence, and functional variants thereof, identified by NCBI Gen ID NO. 28755.

As used herein, the recitation of a numerical range for a variable is intended to convey that the present disclosure may be practiced with the variable equal to any of the values within that range. Thus, for a variable which is inherently discrete, the variable can be equal to any integer value within the numerical range, including the end-points of the range. Similarly, for a variable which is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

### 2.1 Principle of the Invention

The invention is defined by the appended claims.

The present disclosure is based, in part, on the discovery that engineered co-stimulatory domains can demonstrate equivalent or superior activity compared to conventional co-stimulatory domains. In specific examples, the co-stimulatory domains disclosed herein have equivalent or superior co-stimulatory activity in terms of cell proliferation following antigen-induced activation and/or cytokine secretion. In some embodiments, a nucleic acid molecule is provided that comprises a nucleic acid sequence encoding one of the co-stimulatory domains disclosed herein. In some embodiments, the co-stimulatory domain is expressed in a genetically-modified cell as part of a construct such as, for example, a chimeric antigen receptor or an inducible regulatory construct. Accordingly, cells are provided comprising the novel co-stimulatory domains disclosed herein, as well as methods of making cells comprising the novel co-stimulatory domains. Further disclosed herein are genetically-modified cells comprising a co-stimulatory domain disclosed herein for use in reducing the symptoms or severity of a disease. In some embodiments, administration of genetically-modified cells comprising the co-stimulatory domains disclosed herein reduces the symptoms or severity of diseases, such cancers, autoimmune disorders, and other conditions which can be targeted by genetically-modified cells of the present disclosure. Also disclosed herein are pharmaceutical compositions comprising a genetically-modified cell disclosed herein and a pharmaceutically acceptable carrier for use in methods of immunotherapy for treating cancer in a subject in need thereof.

### 2.2 Nucleic Acid Molecules Encoding Co-stimulatory Domains

Provided herein are nucleic acid molecules encoding novel co-stimulatory domains and variants thereof having co-stimulatory activity (*i.e.,* active variants). The co-stimulatory activity of an individual domain can be determined using any method known in the art that measures the activation, proliferation, and cytokine secretion of cells, such as immune cells. One example of such a method is that disclosed in Linsley et al., Journal of Experimental Medicine 176 (1992), 1595-604. Further examples include those methods described herein for measuring cell proliferation and cytokine secretion.

Accordingly, nucleic acid molecules are provided comprising nucleic acid sequences that encode the co-stimulatory domains set forth in SEQ ID NOs: 5-8 and active variants thereof. Specifically, the nucleic acid sequence set forth in SEQ ID NO: 1 encodes the co-stimulatory domain of SEQ ID NO: 5, referred to herein as the Novel1 domain. The nucleic acid sequence set forth in SEQ ID NO: 2 encodes the co-stimulatory domain of SEQ ID NO: 6, referred to herein as the Novel3 domain (not part of the invention). The nucleic acid sequence set forth in SEQ ID NO: 3 encodes the co-stimulatory domain of SEQ ID NO: 7, referred to herein as the Novel5 domain (not part of the invention). The nucleic acid sequence set forth in SEQ ID NO: 4 encodes the co-stimulatory domain of SEQ ID NO: 8, referred to herein as the Novel6 domain (not part of the invention).

Also provided herein are active variants of the nucleic acid sequences encoding the co-stimulatory domains disclosed herein, wherein the variant nucleic acid sequences encode a domain having co-stimulatory activity. Further provided are variants of the co-stimulatory domains disclosed herein that retain co-stimulatory activity. As used herein, "variants" is intended to mean substantially similar sequences. A "variant" polypeptide is intended to mean a polypeptide derived from the "native" polypeptide by deletion and/or addition of one or more amino acids at one or more internal sites in the native protein and/or substitution of one or more amino acids at one or more sites in the native polypeptide. Likewise, a "variant" polynucleotide is a polynucleotide derived from the "native" polynucleotide by deletion and/or addition of one or more nucleic acids at one or more sites in the native nucleotide sequence. As used herein, a "native" polynucleotide or polypeptide comprises a parental sequence from which variants are derived. The parental nucleic acid sequences of variant polynucleotides encoding co-stimulatory domains may include SEQ ID NO: 1. Likewise, the parental polypeptide sequences of variant polypeptides encoding the novel co-stimulatory domains may include SEQ ID NO: 5.

Variant polypeptides encompassed by the embodiments are biologically active. That is, they continue to possess the desired biological activity of the native protein; *i.e.,* co-stimulatory activity. Such variants may result, for example, from human manipulation. Biologically active variants of a native co-stimulatory domain of the disclosure (*e.g*., SEQ ID NO: 5), or variants of the native nucleic acid sequences (*e.g*., SEQ ID NO: 1) encoding the co-stimulatory domains disclosed herein , will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99%, sequence identity to the amino acid sequence of the native polypeptide or nucleic acid sequence of the native polynucleotide, as determined by sequence alignment programs and parameters described elsewhere herein. A biologically active variant of a co-stimulatory domain of the embodiments may differ from that co-stimulatory domain by as few as about 1-5, as few as about 4, as few as 3, 2, or even 1 amino acid residue.

The polypeptides of the embodiments may be altered in various ways including amino acid substitutions, deletions, truncations, and insertions. Methods for such manipulations are generally known in the art. For example, amino acid sequence variants can be prepared by mutations in the DNA. Methods for mutagenesis and polynucleotide alterations are well known in the art. See, for example, Kunkel (1985) Proc. Natl. Acad. Sci. USA 82:488-492; Kunkel et al. (1987) Methods in Enzymol. 154:367-382; U.S. Pat. No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Conservative substitutions, such as exchanging one amino acid with another having similar properties, may be optimal.

Depending on the context, "fragment" refers to a portion of the amino acid sequence of a polypeptide or protein, or polynucleotide encoding a portion of the amino acid sequence of a polypeptide or protein. Fragments may retain the activity of the original protein and hence, such "active" fragments include, for example, fragments of co-stimulatory domain, such as a fragment of any one of SEQ ID NO: 5-8 that retains co-stimulatory activity. A fragment of a nucleotide sequence encoding a co-stimulatory domain, such as a fragment of any one of SEQ ID NOs: 1-4 may encode a protein fragment that is biologically active. A biologically active nucleotide fragment can be prepared by isolating a portion of a nucleic acid sequence encoding a co-stimulatory domain, expressing the encoded portion of the co-stimulatory domain, and assessing the activity of the encoded portion of co-stimulatory domain. Fragments of co-stimulatory domains include fragments of SEQ ID NOS: 5-8. Fragments of co-stimulatory domains comprise at least about 15, 20, 30, 35, 36, 37, 38, 39, 40, 41, or 42 amino acids.

In specific embodiments, variants or fragments of the co-stimulatory domains disclosed herein comprise at least one TNFR-associated Factor (TRAF) binding motif, referred to herein as TRAF motifs. Examples of TRAF motifs provided herein include, but are not limited to, QMED (SEQ ID NO:9), QEED (SEQ ID NO:10), and EEEG (SEQ ID NO:11). For example, in some embodiments, active variants or fragments of the co-stimulatory domains disclosed herein comprise SEQ ID NO: 9 and 11 or SEQ ID NO: 10 and 11.

In some embodiments, the co-stimulatory domains or variants or fragments thereof comprise two TRAF motifs separated by a spacer region. As used herein, the term "spacer region" refers to the region between two predicted TRAF motifs of a co-stimulatory domain. In specific embodiments, the spacer region comprises one of the following amino acid sequences: ASSCRCPQ (SEQ ID NO: 12), ASSCRFPE (SEQ ID NO: 13), ASSCRFPQ (SEQ ID NO: 14), and ASSCRAPS (SEQ ID NO: 15). In specific active variant co-stimulatory domains, the spacer region of SEQ ID NO: 12 is located between the TRAF binding motifs of SEQ ID NO: 9 and SEQ ID NO: 11. In other active variant co-stimulatory domains, the spacer region of SEQ ID NO: 13 is located between the TRAF binding motifs of SEQ ID NO: 10 and SEQ ID NO: 11. In some active variant co-stimulatory domains, the spacer region of SEQ ID NO: 14 is located between the TRAF binding motifs of SEQ ID NO: 10 and SEQ ID NO: 11. In other active variant co-stimulatory domains, the spacer region of SEQ ID NO: 15 is located between the TRAF binding motifs of SEQ ID NO: 10 and SEQ ID NO: 11. Alternatively, the spacer region could be any sequence that maintains co-stimulatory activity of the variant domain.

In certain embodiments, expression cassettes or expression constructs are provided for the expression of at least one co-stimulatory domain disclosed herein, or active variant thereof, in a cell. In some embodiments, the cassette includes 5' and 3' regulatory sequences operably linked to a nucleic acid molecule provided herein encoding a novel co-stimulatory domain, or active variant thereof. "Operably linked" is intended to mean a functional linkage between two or more elements. For example, an operable linkage between a nucleic acid sequence encoding a co-stimulatory domain as disclosed herein and a regulatory sequence (*e.g*., a promoter) is a functional link that allows for expression of the nucleic acid sequence encoding the co-stimulatory domain. Operably linked elements may be contiguous or non-contiguous. When used to refer to the joining of two protein coding regions, by operably linked is intended that the coding regions are in the same reading frame.

In some embodiments, the cassette further comprises at least one additional gene to be co-transformed into a cell. In further embodiments, the additional gene(s) are provided on multiple expression cassettes. In some embodiments, such an expression cassette is provided with a plurality of restriction sites and/or recombination sites for insertion of a recombinant polynucleotide to be under the transcriptional regulation of the regulatory regions. In some embodiments, the expression cassette additionally contains selectable marker genes.

In some embodiments, the expression cassette includes in the 5'-3' direction of transcription, a transcriptional and translational initiation region (*i.e.,* a promoter), a nucleic acid sequence encoding a co-stimulatory domain, or active variant thereof, as disclosed herein, and a transcriptional and translational termination region (*i.e.,* termination region) functional in genetically-modified cells of the present disclosure. The regulatory regions (*i.e.,* promoters, transcriptional regulatory regions, and translational termination regions) and/or a nucleic acid molecule provided herein may be native/analogous to the host cell or to each other. Alternatively, the regulatory regions and/or a nucleic acid molecule provided herein may be heterologous to the host cell or to each other. As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous nucleic acid molecule is from a species different from the species from which the nucleic acid molecule was derived, or, if from the same/analogous species, one or both are substantially modified from their original form and/or genomic locus, or the promoter is not the native promoter for the operably linked nucleic acid molecule. Alternatively, the regulatory regions and/or a nucleic acid molecule provided herein may be entirely synthetic.

The termination region may be native with the transcriptional initiation region, may be native with the operably linked nucleic acid molecule, may be native with the cell host, or may be derived from another source (*i.e.,* foreign or heterologous) to the promoter, the nucleic acid molecule, the cell host, or any combination thereof. In preparing the expression cassette, the various DNA fragments may be manipulated, so as to provide for the DNA sequences in the proper orientation. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, *e.g*., transitions and transversions, may be involved.

In some embodiments, a number of promoters are used in the expression cassettes provided herein. One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present disclosure should not be limited to the use of constitutive promoters. Inducible promoters are also contemplated as part of the present disclosure. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter. Synthetic promoters are also contemplated as part of the present disclosure, for example, the JeT promoter (see, WO/2002/012514).

In some embodiments, the promoters are selected based on the desired outcome. It is recognized that different applications can be enhanced by the use of different promoters in the expression cassettes to modulate the timing, location and/or level of expression of the polynucleotides disclosed herein. Such expression constructs may also contain, if desired, a promoter regulatory region (*e.g.,* one conferring inducible, constitutive, environmentally- or developmentally-regulated, or cell- or tissue-specific/selective expression), a transcription initiation start site, a ribosome binding site, an RNA processing signal, a transcription termination site, and/or a polyadenylation signal.

In order to assess the expression of a co-stimulatory domain or CAR polypeptide comprising a co-stimulatory domain, the expression cassette can also contain either a selectable marker gene or a reporter gene, or both, to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes and fluorescent marker genes.

In specific embodiments, expression cassettes comprising nucleic acid molecules encoding CARs including at least one co-stimulatory domain, or an active variant thereof, disclosed herein are provided. As used herein a "CAR expression cassette" refers to an expression cassette comprising at least one nucleic acid molecule encoding a CAR. In some embodiments, a CAR expression cassette encodes a CAR without a co-stimulatory domain. A CAR expression cassette can also encode a CAR comprising a co-stimulatory domain as disclosed herein, or can encode a CAR comprising a co-stimulatory domain that is not disclosed herein. For example, in some embodiments an expression cassette comprises one or more sequences encoding an extracellular ligand-binding domain and an intracellular stimulatory domain comprising a co-stimulatory domain, or an active variant thereof, as disclosed herein. In specific embodiments, the extracellular ligand-binding domain is specific for an antigen of a cancer cell, such as, for example, an antigen specific for B cell lymphoma.

In a specific embodiment, the expression cassette encodes a CAR comprising an anti-CD19 scFv, the Novel1 co-stimulatory domain (SEQ ID NO: 5), or an active variant thereof, and a CD3ζ signaling domain. It is contemplated that these expression cassettes can be engineered to have specificity for any suitable disease-specific antigen or molecule.

In other specific embodiments, expression cassettes comprising nucleic acid molecules encoding an inducible regulatory construct including at least one co-stimulatory domain disclosed herein, or an active variant thereof, are provided. As used herein, a "regulatory expression cassette" refers to an expression cassette comprising a nucleic acid molecule encoding an inducible regulatory construct. An expression cassette can be both a CAR expression cassette and a regulatory expression cassette. In some embodiments a single expression cassette can comprise a nucleotide sequence encoding a CAR that does not comprise a co-stimulatory domain of any one of SEQ ID NOs: 5-8, or active fragments or variants thereof, and a nucleotide sequence encoding an inducible regulatory construct as described herein.

For example, in some embodiments, expression cassettes comprise sequences encoding a binding domain and at least one co-stimulatory domain, or an active variant thereof, as disclosed herein, wherein a small molecule, antibody, or other molecule binds to the binding domain to induce dimerization of two inducible regulatory constructs. In some embodiments, such dimerization initiates the co-stimulatory signal to the cell to promote proliferation, survival, and/or cytokine secretion. In some embodiments, wherein the binding domain can bind a small molecule, the binding domain comprises an analogue of FKBP12 (*e.g*., comprising an F36V substitution) and the small molecule is rimiducid (*i.e.,* AP1903). Any binding domains known in the art to be useful in such inducible regulatory constructs, such as CAR-T cell safety switches and the like, are contemplated in the present disclosure.

In a specific embodiment, the expression cassette encodes an inducible regulatory construct comprising a binding domain and the Novel1 co-stimulatory domain (SEQ ID NO: 5), or an active variant thereof.

Also provided herein are vectors comprising the nucleic acid molecules encoding the novel co-stimulatory domains of the present disclosure. In some embodiments, vectors comprise a nucleic acid molecule encoding the novel co-stimulatory domains or an expression cassette as disclosed herein. In some embodiments, nucleic acids encoding the co-stimulatory domains disclosed herein are cloned into a number of types of vectors. For example, in some embodiments the nucleic acid is cloned into a vector including, but not limited to a plasmid, a phagemid, a phage derivative, an animal virus, and a cosmid. Vectors of particular interest include expression vectors, replication vectors, probe generation vectors, and sequencing vectors.

In specific embodiments, nucleic acid molecules encoding a co-stimulating domain are provided on viral vectors, such as retroviral vectors, lentiviral vectors, adenoviral vectors, and adeno-associated viral (AAV) vectors. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (*e.g*., WO 01/96584; WO 01/29058; and U.S. Pat. No. 6,326,193).

### 2.3 Chimeric Antigen Receptors (CARs) and Inducible Regulatory Constructs

Provided herein are genetically-modified cells expressing a cell surface chimeric antigen receptor (CAR). Generally, a CAR of the present disclosure will comprise at least an extracellular domain and an intracellular domain. In some embodiments, the extracellular domain comprises a target-specific binding element otherwise referred to as a ligand-binding domain or moiety. In some embodiments, the intracellular domain, or cytoplasmic domain, comprises at least one co-stimulatory domain, or active variant thereof, as disclosed herein, and one or more signaling domains such as, for example, CD3 ζ. For example, in some embodiments, the CARs disclosed herein comprise an intracellular domain comprising at least one co-stimulatory domain, such as that provided in SEQ ID NO: 5, or an active variant thereof. In specific embodiments, the CARs disclosed herein comprise at least two co-stimulatory domains, wherein at least one of the co-stimulatory domains is set forth in SEQ ID NO: 5, or an active fragment or variant disclosed herein.

In some embodiments, a CAR of the present disclosure comprises an extracellular, target-specific binding element otherwise referred to as a ligand-binding domain or moiety. The choice of ligand-binding domain depends upon the type and number of ligands that define the surface of a target cell. For example, the ligand-binding domain may be chosen to recognize a ligand that acts as a cell surface marker on target cells associated with a particular disease state. Thus, examples of cell surface markers that may act as ligands for the ligand-binding domain in the CAR of the present disclosure can include those associated with viral, bacterial and parasitic infections, autoimmune disease, and cancer cells. In some embodiments, the CAR of the present disclosure is engineered to target a tumor antigen of interest by way of engineering a desired ligand-binding moiety that specifically binds to an antigen on a tumor cell. In the context of the present disclosure, "tumor antigen" refers to antigens that are common to specific hyperproliferative disorders such as cancer.

In some embodiments, the extracellular ligand-binding domain of the CAR is specific for any tumor antigen or epitope of interest. As non-limiting examples, in some embodiments the antigen of the target is a tumor-associated surface antigen, such as ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, ductal-epithelial mucine, gp36, TAG-72, glycosphingolipids, glioma-associated antigen, B-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostate specific antigen (PSA), PAP, NY-ESO-1, LAGA-la, p53, prostein, PSMA, surviving and telomerase, prostate-carcinoma tumor antigen-1 (PCTA-1), MAGE, ELF2M, neutrophil elastase, ephrin B2, CD22, insulin growth factor (IGFl)-l, IGF-II, IGFI receptor, mesothelin, a major histocompatibility complex (MHC) molecule presenting a tumor-specific peptide epitope, 5T4, ROR1, Nkp30, NKG2D, tumor stromal antigens, the extra domain A (EDA) and extra domain B (EDB) of fibronectin and the Al domain of tenascin-C (TnC Al) and fibroblast associated protein (fap); a lineage-specific or tissue specific antigen such as CD3, CD4, CD8, CD24, CD25, CD33, CD34, CD133, CD138, CTLA-4, B7- 1 (CD80), B7-2 (CD86), endoglin, a major histocompatibility complex (MHC) molecule, BCMA (CD269, TNFRSF 17), CS1, or a virus-specific surface antigen such as an HIV-specific antigen (such as HIV gpl20); an EBV-specific antigen, a CMV-specific antigen, a HPV-specific antigen such as the E6 or E7 oncoproteins, a Lasse Virus-specific antigen, an Influenza Virus-specific antigen, as well as any derivate or variant of these surface markers. In a particular embodiment of the present disclosure, the ligand-binding domain is specific for CD19.

In some embodiments, the extracellular domain of a chimeric antigen receptor further comprises an autoantigen (see, Payne et al. (2016) Science, Vol. 353 (6295): 179-184), which can be recognized by autoantigen-specific B cell receptors on B lymphocytes, thus directing T cells to specifically target and kill autoreactive B lymphocytes in antibody-mediated autoimmune diseases. Such CARs can be referred to as chimeric autoantibody receptors (CAARs), and the incorporation of one or more co-stimulatory domains described herein into such CAARs is encompassed by the present disclosure.

In some embodiments, a CAR disclosed herein further comprises a transmembrane domain which links the extracellular ligand-binding domain or autoantigen with the intracellular signaling and co-stimulatory domains. In some embodiments, the transmembrane domain is a CD8α transmembrane polypeptide.

The intracellular signaling domain of a CAR of the present disclosure is responsible for activation of at least one of the normal effector functions of the cell in which the CAR has been placed and/or activation of proliferative and cell survival pathways. The term "effector function" refers to a specialized function of a cell. Effector function of a T cell, for example, may be cytolytic activity or helper activity including the secretion of cytokines. An intracellular signaling domain, such as CD3 ζ, can provide an activation signal to the cell in response to binding of the extracellular domain. As discussed, the activation signal can induce an effector function of the cell such as, for example, cytolytic activity or cytokine secretion.

In some embodiments, the intracellular domain includes one or more intracellular co-stimulatory domains, such as those described herein, which transmit a co-stimulatory signal which promotes cell proliferation, cell survival, and/or cytokine secretion after binding of the extracellular domain. In some embodiments, such intracellular co-stimulatory domains include, without limitation, any co-stimulatory domain disclosed herein or those domains known in the art, such as, for example, a CD28 domain, a 4-1BB domain, an OX-40 domain, an ICOS domain, or a CD27 domain.

Also provided herein are genetically-modified cells expressing an inducible regulatory construct. In some embodiments, an inducible regulatory construct is a transmembrane or intracellular construct that is expressed in a cell which provides an inducible co-stimulatory signal to promote cell proliferation, cell survival, and/or cytokine secretion. In some embodiments, inducible regulatory constructs comprise one or more co-stimulatory domains, such as those described herein and/or others known in the art, which provide a co-stimulatory signal upon activation. Generally, a co-stimulatory signal can be induced, for example, by homodimerization of two inducible regulatory construct polypeptides. An inducible regulatory construct typically comprises a binding domain which allows for homodimerization following binding of a small molecule, an antibody, or other molecule that allows for homodimerization of two construct polypeptides. Dimerization can initiate the co-stimulatory signal to the cell to promote proliferation, survival, and/or cytokine secretion. In some embodiments, wherein the binding domain binds a small molecule, the binding domain comprises an analogue of FKBP12 (*e.g*., comprising an F36V substitution) and the small molecule is rimiducid (*i.e.,* AP1903). Any binding domains known in the art to be useful in such inducible regulatory constructs, such as CAR-T cell safety switches and the like, are contemplated in the present disclosure.

In particular embodiments, the intracellular signaling domain of a CAR of the present disclosure comprises a signaling domain derived from CD3 ζ and at least one novel co-stimulatory domain of SEQ ID NO: 5, or an active variant thereof.

In other particular embodiments, the inducible regulatory constructs disclosed herein comprise a binding domain, which allows for dimerization of two constructs, and at least one novel co-stimulatory domain of SEQ ID NO: 5, or an active variant thereof.

### 2.4 Methods for Producing Recombinant Viral Vectors

In some embodiments, the present disclosure provides recombinant AAV vectors for use in the methods of the present disclosure. Recombinant AAV vectors are typically produced in mammalian cell lines such as HEK-293. Because the viral *cap* and *rep* genes are removed from the vector to prevent its self-replication to make room for the therapeutic gene(s) to be delivered (*e.g.* the endonuclease gene), it is necessary to provide these *in trans* in the packaging cell line. In addition, it is necessary to provide the "helper" (*e.g*. adenoviral) components necessary to support replication (Cots D, Bosch A, Chillon M (2013) Curr. Gene Ther. 13(5): 370-81). Frequently, recombinant AAV vectors are produced using a triple-transfection in which a cell line is transfected with a first plasmid encoding the "helper" components, a second plasmid comprising the *cap* and *rep* genes, and a third plasmid comprising the viral ITRs containing the intervening DNA sequence to be packaged into the virus. Viral particles comprising a genome (ITRs and intervening gene(s) of interest) encased in a capsid are then isolated from cells by freeze-thaw cycles, sonication, detergent, or other means known in the art. Particles are then purified using cesium-chloride density gradient centrifugation or affinity chromatography and subsequently delivered to the gene(s) of interest to cells, tissues, or an organism such as a human patient. Accordingly, methods are provided herein for producing recombinant AAV vectors comprising at least one nucleic acid sequence encoding a co-stimulatory domain described herein, such as SEQ ID NO: 5, or active variants thereof. Likewise, methods are provided herein for producing recombinant AAV vectors encoding CARs or inducible regulatory constructs which include at least one co-stimulatory domain described herein, such as SEQ ID NO: 5, or active variants thereof.

In some embodiments, genetic transfer is accomplished via lentiviral vectors. Lentiviruses, in contrast to other retroviruses, in some contexts may be used for transducing certain non-dividing cells. Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini *et al*., (1996 and 1998); Zufferey *et al.,* (1997); Dull *et al.,* 1998, U.S. Pat. Nos. 6,013,516; and 5,994,136). In some embodiments, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In specific embodiments, lentiviral vectors are prepared using a plasmid encoding the gag, pol, tat, and rev genes cloned from human immunodeficiency virus (HIV) and a second plasmid encoding the envelope protein from vesicular stomatitis virus (VSV-G) used to pseudotype viral particles. A transfer vector, such as the pCDH-EF1-MCS vector, can be used with a suitable promoter such as the JeT promoter or the EF1 promoter. CAR-signaling domains, such as the co-stimulatory domains disclosed herein, and active variants thereof, can then be inserted downstream of the promoter, followed by an IRES and GFP. All three plasmids can then be transfected into lentivirus cells, such as the Lenti-X-293T cells, and lentivirus can then be harvested, concentrated and screened after a suitable incubation time. Accordingly, methods are provided herein for producing recombinant lentiviral vectors comprising at least one nucleic acid sequence a co-stimulatory domain described herein, such as SEQ ID NO: 5, or active variants thereof. Likewise, methods are provided herein for producing recombinant lentiviral vectors encoding a CAR or an inducible regulatory construct which includes at least one co-stimulatory domain described herein, such as SEQ ID NO: 5, or active variants thereof.

### 2.5 Genetically-Modified Cells and Populations Thereof Comprising Novel Co-Stimulatory Domains

Provided herein are T cells and NK cells genetically-modified to contain at least one novel co-stimulatory domain of SEQ ID NO: 5, or an active variant thereof, as disclosed herein. In specific embodiments, the genetically-modified T cell or NK cell comprises a nucleic acid molecule encoding a CAR or an inducible regulatory construct incorporating at least one novel co-stimulatory domain of SEQ ID NO: 5, or an active variant thereof. In different variations of the present disclosure, a nucleic acid molecule or expression cassette which encodes a novel co-stimulatory domain described herein is present within the genome of the genetically-modified cell or, alternatively, is not integrated into the genome of the cell. In some embodiments where the nucleic acid molecule or expression cassette is not integrated into the genome, the nucleic acid molecule or expression cassette is present in the genetically-modified cell in a recombinant DNA construct, in an mRNA, in a viral genome, or other nucleic acid which is not integrated into the genome of the cell. In particular embodiments, the genetically-modified cell can comprise a nucleic acid molecule encoding a co-stimulatory domain described herein and further comprise at least one expression cassette disclosed herein comprising a nucleotide sequence encoding a CAR that does not comprise a co-stimulatory domain disclosed herein and/or a nucleotide sequence encoding an inducible regulatory construct.

In some genetically-modified cells embodied herein, the nucleic acid molecule encoding a CAR or an inducible regulatory construct incorporating at least one novel co-stimulatory domain described herein, is positioned with the endogenous T cell receptor alpha gene of the cell. In some of these embodiments, the nucleic acid molecule is positioned within the endogenous T cell receptor alpha constant region gene, such as within exon 1 of the T cell receptor alpha constant region gene.

In specific embodiments, the cells comprising the novel co-stimulatory domains, or active variants thereof, are human T cells or NK cells. In some embodiments, the cells are primary T cells or primary NK cells.

T cells and NK cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present disclosure, any number of T cell and NK cell lines available in the art may be used. In some embodiments of the present disclosure, T cells and NK cells are obtained from a unit of blood collected from a subject using any number of techniques known to the skilled artisan. In one embodiment, cells from the circulating blood of an individual are obtained by apheresis.

Genetically-modified cells comprising the novel co-stimulatory domains disclosed herein, or active variants thereof, can exhibit increased proliferation when compared to appropriate control cells without the novel co-stimulatory domain, or active variant thereof. In some embodiments, cells comprising the novel co-stimulatory domains disclosed herein, or active variants thereof, further exhibit increased activation and proliferation *in vitro* or *in vivo* following stimulation with an appropriate antigen. For example, cells, such as CAR-T cells and CAR-NK cells, can exhibit increased activation, proliferation, and/or increased cytokine secretion compared to a control cell without the novel co-stimulatory domains disclosed herein, or active variants thereof. Increased cytokine secretion can include the increased secretion of IFN-γ, IL-2, TNF-α, among others. Methods for measuring cell activation and cytokine production are well known in the art, and some suitable methods are provided in the examples herein.

The present disclosure further provides a population of genetically-modified cells comprising a plurality of a genetically-modified cells described herein, which comprise in their genome a nucleic acid molecule encoding a CAR or an inducible regulatory construct incorporating at least one novel co-stimulatory domain of SEQ ID NO: 5, or an active variant thereof. Thus, in various embodiments of the invention, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population are a genetically-modified cell comprising a novel co-stimulatory domain disclosed herein. In certain embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express a CAR comprising a novel co-stimulatory domain described herein. In other embodiments, a population of genetically-modified cells is provided wherein at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or up to 100%, of cells in the population express an inducible regulatory construct comprising a novel co-stimulatory domain disclosed herein and a CAR which does not comprise a co-stimulatory domain described herein.

### 2.6 Methods for Producing Genetically-Modified Cells

The present disclosure provides *in vitro* methods for producing genetically-modified T cells or NK cells comprising the novel co-stimulatory domains disclosed herein, or active variants thereof. In specific embodiments, methods are provided for modifying the cell to comprise a nucleic acid sequence molecule encoding a CAR incorporating at least one novel co-stimulatory domain of SEQ ID NO: 5, or an active variant thereof. In other embodiments, methods are provided for modifying the cell to comprise a nucleic acid molecule encoding an inducible regulatory construct incorporating at least one novel co-stimulatory domain of SEQ ID NOs: 5, or an active variant thereof. In different aspects of the present disclosure, a nucleic acid molecule or expression cassette encoding a novel co-stimulatory domain disclosed herein, or an active variant thereof, is integrated into the genome of the cell or, alternatively, is not integrated into the genome of the cell.

In some embodiments, DNA or RNA encoding the novel co-stimulatory domains (or CARs or inducible regulatory constructs) disclosed herein is introduced into a cell using any technology known in the art. In specific embodiments, vectors or expression cassettes comprising the nucleic acids encoding the novel co-stimulatory domains (or CARs or inducible regulatory constructs) disclosed herein is introduced into a cell using a viral vector. Such vectors are known in the art and include lentiviral vectors, adenoviral vectors, and adeno-associated virus (AAV) vectors (reviewed in Vannucci, et al. (2013 New Microbiol. 36:1-22). Recombinant AAV vectors useful in the present disclosure can have any serotype that allows for transduction of the virus into the cell and insertion of the nuclease gene into the cell and, in particular embodiments, into the cell genome. In particular embodiments, recombinant AAV vectors have a serotype of AAV2 or AAV6. Recombinant AAV vectors can also be self-complementary such that they do not require second-strand DNA synthesis in the host cell (McCarty, et al. (2001) Gene Ther. 8:1248-54).

In some embodiments, nucleic acid molecules or expression cassettes disclosed herein are delivered into a cell in the form of DNA (*e.g*., circular or linearized plasmid DNA or PCR products) or RNA. In some embodiments wherein the engineered nuclease genes are delivered in DNA form (*e.g.* plasmid) and/or via a viral vector (*e.g.* AAV or lentiviral vector), they are operably linked to a promoter or found on an expression cassette disclosed herein. In some embodiments, the promoter is a viral promoter such as endogenous promoters from the viral vector (*e.g.* the LTR of a lentiviral vector) or the well-known cytomegalovirus- or SV40 virus-early promoters. In other embodiments, the promoter is a synthetic promoter, such as the JeT promoter. In certain embodiments, genes encoding the novel co-stimulatory domains or CARs disclosed herein are operably linked to a promoter that drives gene expression preferentially in the target cell (*e.g.,* a human T cell).

In some embodiments, nucleic acid molecules or expression cassettes encoding co-stimulatory domains (or CARs or inducible regulatory constructs) disclosed herein, are coupled covalently or non-covalently to a nanoparticle or encapsulated within such a nanoparticle using methods known in the art (Sharma, et al. (2014) Biomed Res Int. 2014). A nanoparticle is a nanoscale delivery system whose length scale is <1 µm, preferably <100 nm. Such nanoparticles may be designed using a core composed of metal, lipid, polymer, or biological macromolecule, and multiple copies of the nucleic acid molecules or expression cassettes can be attached to or encapsulated with the nanoparticle core. This increases the copy number of the DNA that is delivered to each cell and, so, increases the intracellular expression of each engineered nuclease to maximize the likelihood that the co-stimulatory domains (or CARs or inducible regulatory constructs) will be expressed. The surface of such nanoparticles may be further modified with polymers or lipids (*e.g*., chitosan, cationic polymers, or cationic lipids) to form a core-shell nanoparticle whose surface confers additional functionalities to enhance cellular delivery and uptake of the payload (Jian et al. (2012) Biomaterials. 33(30): 7621-30). Nanoparticles may additionally be advantageously coupled to targeting molecules to direct the nanoparticle to the appropriate cell type and/or increase the likelihood of cellular uptake. Examples of such targeting molecules include antibodies specific for cell-surface receptors and the natural ligands (or portions of the natural ligands) for cell surface receptors.

In some embodiments, nucleic acid molecules or expression cassettes encoding co-stimulatory domains (or CARs or inducible regulatory constructs) disclosed herein, are encapsulated within liposomes or complexed using cationic lipids (see, *e.g*., LIPOFECTAMINE, Life Technologies Corp., Carlsbad, CA; Zuris et al. (2015) Nat Biotechnol. 33: 73-80; Mishra et al. (2011) J Drug Deliv. 2011:863734). The liposome and lipoplex formulations can protect the payload from degradation, and facilitate cellular uptake and delivery efficiency through fusion with and/or disruption of the cellular membranes of the cells.

In some embodiments, nucleic acid molecules or expression cassettes encoding co-stimulatory domains (or CARs or inducible regulatory constructs) disclosed herein, are encapsulated within polymeric scaffolds (*e.g*., PLGA) or complexed using cationic polymers (*e.g.,* PEI, PLL) (Tamboli et al. (2011) Ther Deliv. 2(4): 523-536). In some embodiments, nucleic acid molecules or expression cassettes encoding co-stimulatory domains (or CARs or inducible regulatory constructs) disclosed herein, are combined with amphiphilic molecules that self-assemble into micelles (Tong et al. (2007) J Gene Med. 9(11): 956-66). Polymeric micelles may include a micellar shell formed with a hydrophilic polymer (*e.g*., polyethyleneglycol) that can prevent aggregation, mask charge interactions, and reduce nonspecific interactions outside of the cell.

In some embodiments, nucleic acid molecules or expression cassettes encoding co-stimulatory domains (or CARs or inducible regulatory constructs) disclosed herein, are formulated as emulsions for delivery to the cell. The term "emulsion" refers to, without limitation, any oil-in-water, water-in-oil, water-in-oil-in-water, or oil-in-water-in-oil dispersions or droplets, including lipid structures that can form as a result of hydrophobic forces that drive apolar residues (*e.g*., long hydrocarbon chains) away from water and polar head groups toward water, when a water immiscible phase is mixed with an aqueous phase. These other lipid structures include, but are not limited to, unilamellar, paucilamellar, and multilamellar lipid vesicles, micelles, and lamellar phases. Emulsions are composed of an aqueous phase and a lipophilic phase (typically containing an oil and an organic solvent). Emulsions also frequently contain one or more surfactants. Nanoemulsion formulations are well known, *e.g*., as described in US Patent Application Nos. 2002/0045667 and 2004/0043041, and US Pat. Nos. 6,015,832, 6,506,803, 6,635,676, and 6,559,189.

In some embodiments, nucleic acid molecules or expression cassettes encoding co-stimulatory domains (or CARs or inducible regulatory constructs) disclosed herein, are covalently attached to, or non-covalently associated with, multifunctional polymer conjugates, DNA dendrimers, and polymeric dendrimers (Mastorakos et al. (2015) Nanoscale. 7(9): 3845-56; Cheng et al. (2008) J Pharm Sci. 97(1): 123-43). The dendrimer generation can control the payload capacity and size, and can provide a high payload capacity. Moreover, display of multiple surface groups can be leveraged to improve stability and reduce nonspecific interactions.

Methods of introducing and expressing genes into a cell are known in the art. In the context of an expression vector, the vector can be readily introduced into a host cell, *e.g.,* mammalian, bacterial, yeast, or insect cell by any method in the art. For example, the expression vector can be transferred into a host cell by physical, chemical, or biological means. Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection. Biological methods for introducing a polynucleotide of interest into a host cell include the use of DNA and RNA vectors. Viral vectors, and especially retroviral vectors, have become the most widely used method for inserting genes into mammalian, *e.g*., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362. Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and *in vivo* is a liposome (*e.g.,* an artificial membrane vesicle).

In some embodiments, the invention further provides for the introduction of the nucleic acid molecules or expression cassettes disclosed herein into the T cell receptor alpha gene. In certain embodiments, the nucleic molecule or expression cassettes are introduced into a recognition sequence present in the T cell receptor alpha constant region gene, which comprises the coding sequences for the T cell receptor alpha subunit. As such, introduction of the nucleic acid molecules or expression cassettes disrupts expression of the endogenous T cell receptor alpha subunit, and consequently disrupts expression of the endogenous T cell receptor. In particular embodiments, such recognition sequences can be present within exon 1 of the T cell receptor alpha constant region gene.

In particular embodiments, introducing a nucleic acid molecule encoding a co-stimulatory domain disclosed herein into a cell, such as a T cell or NK cell, can increase activation, proliferation, and/or cytokine secretion of the cell when compared to a control cell without the co-stimulatory domain disclosed herein. In some embodiments, activation, proliferation, and/or cytokine secretion of a cell can be increased *in vitro* or *in vivo* by introducing a nucleic acid molecule encoding a co-stimulatory domain disclosed herein.

In some embodiments, introduction of at least one novel co-stimulatory domain, or an active fragment or variant thereof, into a cell, such as a T cell or NK cell, prolong the period of cell proliferation and/or expansion of the cell population, and/or delay cell exhaustion, when compared to control cells without the novel co-stimulatory domain disclosed herein. Methods of measuring cell expansion and exhaustion (such as T cell or NK cell expansion and exhaustion) are known in the art and disclosed elsewhere herein.

### 2.7 Pharmaceutical Compositions

In some embodiments, the present disclosure provides a pharmaceutical composition comprising a genetically-modified T cell or NK cell, or a population of genetically-modified T cells or NK cells, of the present disclosure and a pharmaceutically-acceptable carrier. Such pharmaceutical compositions can be prepared in accordance with known techniques. See, *e.g.,* Remington, The Science And Practice of Pharmacy (21st ed. 2005). In the manufacture of a pharmaceutical formulation according to the present disclosure, cells are typically admixed with a pharmaceutically acceptable carrier and the resulting composition is administered to a subject. The carrier must, of course, be acceptable in the sense of being compatible with any other ingredients in the formulation and must not be deleterious to the subject. In some embodiments, pharmaceutical compositions of the present disclosure further comprises one or more additional agents useful in the treatment of a disease in the subject. In additional embodiments, where the genetically-modified cell is a genetically-modified human T cell or NK cell (or a cell derived therefrom), pharmaceutical compositions of the present disclosure further include biological molecules, such as cytokines (*e.g.,* IL-2, IL-7, IL-15, and/or IL-21), which promote *in vivo* cell proliferation and engraftment. Pharmaceutical compositions comprising genetically-modified cells of the present disclosure can be administered in the same composition as an additional agent or biological molecule or, alternatively, can be co-administered in separate compositions.

The pharmaceutical compositions and medicaments of the present disclosure are useful as immunotherapy in the treatment of cancer. Non-limiting examples of cancer which may be treated with the pharmaceutical compositions and medicaments of the present disclosure are carcinomas, lymphomas, sarcomas, melanomas, blastomas, leukemias, and germ cell tumors, including but not limited to cancers of B-cell origin, neuroblastoma, osteosarcoma, prostate cancer, renal cell carcinoma, rhabdomyosarcoma, liver cancer, gastric cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, multiple myeloma, Hodgkin lymphoma, non-Hodgkin's lymphomas, acute myeloid lymphoma, chronic myelogenous leukemia, chronic lymphoid leukemia, immunoblastic large cell lymphoma, acute lymphoblastic leukemia, mycosis fungoides, anaplastic large cell lymphoma, and T-cell lymphoma, and any combinations of said cancers. In certain embodiments, cancers of B-cell origin include, without limitation, B-lineage acute lymphoblastic leukemia, B-cell chronic lymphocytic leukemia, B-cell lymphoma, diffuse large B cell lymphoma, pre-B ALL (pediatric indication), mantle cell lymphoma, follicular lymphoma, marginal zone lymphoma, Burkitt's lymphoma, and B-cell non-Hodgkin's lymphoma.

### 2.8 Methods of Administering Genetically-Modified Cells (not part of the invention)

Another aspect disclosed herein is the administration of the genetically-modified cells of the present disclosure to a subject in need thereof. The pharmaceutical compositions described herein may be administered to a subject in need thereof. For example, an effective amount of a population of cells comprising a novel co-stimulatory domain described herein, or an active variant thereof, can be administered to a subject having a disease. The disease can be cancer, such as a cancer of B-cell origin. Thus, the present disclosure also provides a method for providing a T cell-mediated immune response to a target cell population or tissue in a mammal, comprising the step of administering to the mammal a CAR-T cell, wherein the CAR comprises an extracellular ligand-binding domain that specifically interacts with a predetermined target, such as a tumor antigen, and an intracellular domain that comprises at least one signaling domain, such as CD3ζ, and at least one novel co-stimulatory signaling domain described herein, or active variant thereof. The CAR may not comprise a novel co-stimulatory domain described herein, but the cell may further comprise an inducible regulatory construct which comprises at least one novel co-stimulatory domain described herein, wherein dimerization of the inducible regulatory construct initiates a co-stimulatory signal to the cell. The method may further comprise the administration of a small molecule, antibody, or other molecule which induces dimerization of the inducible regulatory construct in order to induce a proliferative and/or survival signal in the CAR-T cell in order to promote cell proliferation and expansion of the CAR-T cell population *in vivo.* The administered CAR-T cells are able to reduce the proliferation, reduce the number, or kill target cells in the recipient. Unlike antibody therapies, genetically-modified cells of the present disclosure are able to replicate and expand *in vivo,* resulting in long-term persistence that can lead to sustained control of a disease.

Examples of possible routes of administration include parenteral, (*e.g.*, intravenous (IV), intramuscular (IM), intradermal, subcutaneous (SC), or infusion) administration. Moreover, the administration may be by continuous infusion or by single or multiple boluses. One or both of the agents may be infused over a period of less than about 12 hours, 6 hours, 4 hours, 3 hours, 2 hours, or 1 hour. The infusion may occur slowly at first and then is increased over time.

A genetically-modified cell of the present disclosure may target a tumor antigen for the purposes of treating cancer. Such cancers can include, without limitation, carcinomas, adenocarcinomas, lymphomas, sarcomas, melanomas, blastomas, leukemias, and germ cell tumors, including but not limited to cancers of B-cell origin, neuroblastoma, osteosarcoma, prostate cancer, renal cell carcinoma, rhabdomyosarcoma, liver cancer, gastric cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, multiple myeloma, Hodgkin lymphoma, non-Hodgkin's lymphomas, acute myeloid lymphoma, chronic myelogenous leukemia, chronic lymphoid leukemia, immunoblastic large cell lymphoma, acute lymphoblastic leukemia, mycosis fungoides, anaplastic large cell lymphoma, and T-cell lymphoma, and any combinations of said cancers. Cancers of B-cell origin include, without limitation, B-lineage acute lymphoblastic leukemia, B-cell chronic lymphocytic leukemia, B-cell lymphoma, diffuse large B cell lymphoma, pre-B ALL (pediatric indication), mantle cell lymphoma, follicular lymphoma, marginal zone lymphoma, Burkitt's lymphoma, and B-cell non-Hodgkin's lymphoma.

Where cancer is treated with the presently disclosed genetically-modified cells, the subject administered the genetically-modified cells is further administered an additional therapeutic, such as radiation, surgery, or a chemotherapeutic agent.

When an "effective amount" or "therapeutic amount" is indicated, the precise amount of the compositions of the present disclosure to be administered can be determined by a physician with consideration of individual differences in age, weight, tumor size (if present), extent of infection or metastasis, and condition of the patient (subject). A pharmaceutical composition comprising the genetically-modified cells described herein may be administered at a dosage of 10⁴ to 10⁹ cells/kg body weight, including all integer values within those ranges. The dosage may be 10⁵ to 10⁶ cells/kg body weight, including all integer values within those ranges. Cell compositions may be administered multiple times at these dosages. The cells can be administered by using infusion techniques that are commonly known in immunotherapy (see, *e.g.,* Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

Administration of genetically-modified cells of the present disclosure may reduce at least one symptom of a target disease or condition. For example, administration of genetically-modified cells of the present disclosure can reduce at least one symptom of a cancer, such as cancers of B-cell origin. Symptoms of cancers, such as cancers of B-cell origin, are well known in the art and can be determined by known techniques.

### EXPERIMENTAL

This disclosure is further illustrated by the following examples, which should not be construed as limiting. Those skilled in the art will recognize, or be able to ascertain, using no more than routine experimentation, numerous equivalents to the specific substances and procedures described herein. Examples not falling within the scope of the claims are for illustrative purposes only.

### EXAMPLE 1

### Production of lentiviral vectors for expression of CARs with novel co-stimulatory domains

The purpose of this study was to evaluate and characterize novel co-stimulatory domains that were developed to promote CAR-T cell expansion and cytokine secretion following antigen stimulation.

As shown in Figure 1, four novel co-stimulatory domains were engineered which comprise two TRAF-binding motifs. These domains are referred to as Novel1 (N1; SEQ ID NO: 5), Novel3 (N3; SEQ ID NO: 6), Novel5 (N5; SEQ ID NO: 7), and Novel6 (N6; SEQ ID NO: 8). In order to evaluate each novel co-stimulatory domain, lentiviral vectors were used to prepare anti-CD19 CAR-T cells. Each CAR comprised, from 5' to 3', a signal peptide (SEQ ID NO: 16), an anti-CD19 scFv (SEQ ID NO: 17) having the heavy chain and light chain variable regions of the FMC63 antibody linked by a (G₄S)₃ polypeptide linker, a CD8 hinge region and transmembrane domain (SEQ ID NO: 18), and an intracellular region comprising two intracellular signaling domains. An SV40 polyadenylation (polyA) sequence (SEQ ID NO: 22) was positioned 3' downstream of the CAR sequence. Some lentiviral vectors encoded an anti-CD19 CAR whose intracellular region comprised: (i) a novel co-stimulatory domain, and (ii) a CD3-ζ signaling domain (SEQ ID NO: 19). A negative control vector was prepared that encoded a CAR lacking a co-stimulatory domain (Null), and additional vectors were prepared that encoded CARs having CD28 (SEQ ID NO: 20) or 4-1BB (SEQ ID NO: 21) co-stimulatory domains and a CD3-ζ signaling domain. The lentiviral vectors prepared for this study are summarized in Table 1. Each CAR is illustrated in Figure 2, and their respective sequences are set forth in SEQ ID NOs: 22-28.

**Table 1.**

| Lentiviral Vector | Co-Stimulatory Domain | Activation Domain | CAR SEQ ID NO: |
|---|---|---|---|
| 1 | - | CD3ζ | 22 |
| 2 | CD28ζ | CD3ζ | 23 |
| 3 | 4-1BB | CD3ζ | 24 |
| 4 | Novel1 | CD3ζ | 25 |
| 5 | Novel3 | CD3ζ | 26 |
| 6 | Novel5 | CD3ζ | 27 |
| 7 | Novel6 | CD3ζ | 28 |

Lentiviral vectors were prepared with a 2^{nd} generation approach, using a plasmid encoding gag, pol, tat, and rev cloned from human immunodeficiency virus (HIV). A second plasmid, encoding the envelope protein from vesicular stomatitis virus (VSV-G) was used to pseudotype viral particles. The transfer vector pCDH-EF1-MCS (purchased from System Biosciences) was modified to contain the JeT promoter (SEQ ID NO: 32) rather than the EF1 promoter, and CAR-signaling variants were cloned downstream of the promoter, followed by and IRES and GFP. All three plasmids were transfected into Lenti-X-293T cells (purchased from ClonTech/Takara), and lentivirus was harvested from supernatants 3d later. Viral particles were concentrated using Lenti-X concentrator (ClonTech/Takara) and quantified, using the Lenti-X qRT-PCR Titration kit (ClonTech/Takeda) to determine the number of viral genomes/ml, as well as titration on 293T cells (ATCC) to determine transducible units/ml.

### EXAMPLE 2

### Expression of chimeric antigen receptors comprising novel co-stimulatory domains in human T cells and characterization in antigen-induced stress test

### 1. Preparation of CAR-T cells and antigen-induced stress test

The purpose of this study was to evaluate the novel co-stimulatory domains in an antigen-induced stress. Briefly, lentiviral vectors were prepared as described in Example 1. To prepare donor human T cells for lentiviral transduction, T cells were stimulated in ImmunoCult anti-CD2/CD3/CD28 multimers (StemCell Technologies) and 20ng/ml of IL-2 for 4 days. Cells were then collected and deposited into separate wells for transduction with individual lentiviral vectors. 5 Transducible Units per T cell were added to cultures. Transduction was performed in X-VIVO 15 medium (Lonza) supplemented with only IL-2 (20ng/ml) and 8µg/ml of polybrene (Sigma). Co-incubation of vector and T cells was carried out overnight prior to medium replacement (X-Vivo 15 + 20ng/ml IL-2 + 5% normal human serum).

Beginning 4 days following lentiviral transduction, CAR expression was confirmed by GFP analysis (Figure 3). A sample of each lentivirus-transduced T cell culture was obtained and GFP signal was measured on a Becton-Dickinson LSR:Fortessa flow cytometer. The GFP⁺ T cell population in each culture is identified by the region entitled CAR-GFP+ in Figure 3, and the frequency of GFP+ events is listed on each dot plot.

Subsequently, 5x10⁴ CAR-T cells were cultivated with an equivalent number of Raji cells. At the times indicated in Figure 4 (d3, 6, 10, 14, 17, and 20), cell number and viability were measured by automated cell counting and trypan blue exclusion. CAR-T cells were identified as CD4⁺ or CD8⁺ using antibodies against human CD4 and CD8, as well as GFP signal using flow cytometry. CAR-T numbers were calculated and 1x10⁵ CAR-T cells were re-cultured with 5x10⁴ additional Raji cells (2:1 effector:target ratio). CD4⁺, CD8⁺, and overall number of CAR-T cells were tracked and plotted over time. At each time point, 50µl of culture supernatants were collected and stored at -20°C for a three-plex cytokine secretion assay. Cytokine levels in supernatants were measured using Ultrasensitive human IL-2, TNFα, and IFNγ magnetic bead kits (Life Technologies) in accord with the manufacturer's recommendations. Data were acquired using a Luminex MagPix instrument.

In a second study, CAR-T cells containing our novel co-stimulatory domains were constructed as described above and 1x10⁵ CAR-T cells were cultivated with 1x10⁵ Raji tumor cells. At the times indicated on the X axes of Figure 6, cell number and viability were measured by automated cell counting and trypan blue exclusion. CAR-T cells were identified using antibodies against human CD4 and CD8 as well as GFP signal using flow cytometry. CAR-T numbers were calculated and 1x10⁵ CAR-T cells were re-cultured with 1x10⁵ additional Raji cells. Note that in Figure 6, the target effector ratios were adjusted to 1:1 and Raji cells were added to the culture more frequently, at d3, 5, 7, 10, 12, 14, 17, 19, 21, 24, 26, 28, and 31. CD4⁺, CD8⁺, and overall number of CAR-T cells were tracked and plotted over time.

4 days after transduction, but prior to co-culture with Raji cells, T cell cultures were assessed for CAR-GFP expression by flow cytometry as previously described. T cells were transduced at an MOI of 5 transducible units per cell and approximately similar efficiencies were observed for all lentiviral samples.

### 2. Results of experiment #1

CAR-T cell numbers were measured over time and plotted in Figure 4. Differences in CAR-T numbers were evident from d10 onward. CAR-T cells with 41BBz signaling domains exhibited more sustained proliferation than those with CD28z domains. The BB null control exhibited the lowest levels of CAR-T expansion in this experiment. Novel domains N5 and N6 exhibited high levels of sustained proliferation. No preference for CD4 (Figure 4B) or CD8 (Figure 4C) T cell expansion was observed, as each subset proliferated at similar rates. In order of descending performance in this proliferation assay, the results are as follows: N6>N5>41BBz>N1>>BBnull=CD28z>N3

Secretion of IFNγ, TNFα, and IL-2 were measured by Luminex multiplex assay and appear in Figure 5A, 5B, and 5C, respectively. In general, secretion levels of all cytokines decreased over time. Ranking of the domains in terms of IFNγ secretion at d3 is as follows: 41BBz=CD28z>N1>N5>BBnull=N3>N6. Levels of IFNγ decreased by 50% or greater in all experimental groups at the d7 time point and continued to decrease for the remainder of the experiment. Ranking the domains in terms of TNFα secretion produces the following list: CD28z>N5=41BBz.BBnull>N1=N6>N3. Between d3 and 7 of culture, the level of TNFα production decreases by approximately 50% in CD28z, BBnull, N3, and N5 cultures while 41BBz, N1, and N6 cultures maintain TNFα production levels through d7. Production of IL-2, on the other hand, was low in all novel domain co-cultures, when compared to 41BBz and CD28z. IL-2 measurements may be confounded by high rates of IL-2 consumption by rapidly proliferating T cells present in some cultures (See Figure 4).

### 3. Results of experiment #2

In the second experiment (Figure 6), using more frequent antigen encounter and a higher target effector ratio, the results were as follows, in order of descending performance: N3>N5>CD28z>N1>N6>41BBz>BB null. In this experiment, CAR-T cells expanded continuously throughout the culture period. CD4 CAR-T cells ceased expanding after approximately 12 days in Raji co-culture (Figure 6B), while the CD8 CAR-T cells continued expanding (Figure 6C).

### 4. Conclusions

Several novel signaling domains perform as well or better than 41BBz and/or CD28z under relatively lower (Figure 4) or higher (Figure 6) antigen burden. CARs carrying the N5 signaling domain outperformed 41BBz and CD28z under both conditions. N5 appears to perform the same as 41BBz in the CD4 compartment in both experiments while N5 outperforms 41BBz in the CD8 compartment in both experiments.

### EXAMPLE 3

### Stress test using AAV for targeted insertion of CAR with 41BB, N1, or N6 co-stimulatory domains

### 1. Preparation of CAR-T cells and antigen-induced stress test

In order to evaluate novel intracellular signaling domains, CAR-T cells were produced and their responses to antigen encounter were measured. To produce CAR-T cells, T cells were isolated from an apheresis sample collected from healthy human donors using Stem Cell Technologies CD3 positive selection kit. Two different donors were used in this assay, designated K799 and z4100. T cells were activated and expanded for 3 days using Immunocult anti-CD2/3/28 (StemCell Tech) prior to nucleofection (Lonza 4D nucleofector) with TRC1-2x87EE. Immediately after nucleofection, cells were transduced with AAV6 vectors encoding anti-CD19 CARs featuring different intracellular signaling domains. CAR variants included in this experiment comprised 4-1BB, N1, or N6 co-stimulatory domains. In all vectors, CAR expression was driven by the JeT promoter. Each CAR donor template was flanked by 5' and 3' homology arms, which have homology to the regions upstream and downstream of the TRC 1-2x.87EE recognition sequence. CAR donor templates were further flanked by 5' and 3' inverted terminal repeats. The donor templates for each CAR are illustrated in Figure 7, and the sequences of the vectors used to generate AAVs encoding the CD19-4-1BB CAR the CD19-N1 CAR, and the CD19-N6 CAR, are provided in SEQ ID NOs: 29-31, respectively.

The multiplicity of infection was 50,000. 5 days post-nucleofection/transduction, non-edited CD3+ cells were removed by magnetic depletion (StemCell Tech CD3 positive selection kit). Cells were then assessed for purity of the CD3- fraction, and for CAR expression by flow cytometry, using anti-CD3-BrillianViolet-711 (Biolegend), and CD19-Fc-biotin (Acro) followed by streptavidin-PE (BioLegend). The following day, co-cultures containing T cells and K562 engineered to express CD19 ("K19" cells) were assembled. CD19-Fc+ frequencies determined in the above flow cytometry assay were used to calculate the input number of CAR-T cells and establish an effector:target (E:T) ratio of 2:1. At days 3, 6, 8, and 10 of coculture, samples were acquired for flow cytometric evaluation of tumor cell and CAR-T cell numbers in the co-culture. The numbers of CAR-T cells at each time are plotted in Figure 8 as a demonstration of CAR-T expansion following antigen encounter. Using the calculated number of CAR-T cells as well as the remaining tumor cells detected at each time point, the necessary number of fresh K19 cells were added back to the co-culture in order to re-establish the E:T of 2:1. In addition, a parallel coculture plate was set up with variable E:T ratios (2:1, 1:1, and 1:2). Samples of these co-cultures were taken at 24h and 72h and the number of CD19+ cells was determined by flow cytometry. The results appear in Figure 9. The number of CD19+ cells surviving co-culture with CAR-T cells serves as an indicator of target cell killing.

### 2. Results of antigen-induced stress test

Input T cell populations were normalized to the frequency of CAR+ cells and equivalent numbers of CAR-T cells were challenged with K19 targets at an E:T of 2. Proliferation of CAR-T cells was assessed for T cells produced from donor K799 (Figure 8A) and donor z4100 (Figure 8B). Cells containing TRAC edits, but no CAR insertion (TRC KO) cells did not proliferate in response to antigen encounter. In contrast, CAR-T cells produced using the 4-1BB signaling domain proliferated robustly for the first week of coculture before contracting by d12. CAR-T cells produced with N1 or N6 variants exhibited proliferation rates that were not found to be substantially different from the rate supported by 4-1BB. Killing of CD19+ target cells was also assessed at various E:T ratios at 24 and 72h of the coculture, at which times culture samples were analyzed for the number of remaining CD19+ cells and the results plotted against a control well that contained the same number of K19 cells, but no CAR-T cells. K19 numbers lower than the No T cell control are interpreted as cell killing. CAR-T cells produced using material from donor K799 displayed little cytolytic potential at the 24h time point, demonstrating noticeable killing only at the least stringent E:T ratio of 2:1 (Figure 9, Panel A). By 72h, however, extensive killing was observed at all E:T ratios. N1 and N6 were comparable or superior to 4-1BB. N1 appeared to be superior in cytolytic activity compared to 4-1BB. Extensive killing was observed at both 24h and 72h time points in the cocultures containing CAR-T cells produced from donor z4100 (Figure 9B). As above, N1 and N6 were comparable or superior in terms of cytolytic activity when compared to 4-1BB. In general, more extensive killing was observed from CAR-T cells produced from donor z4100. It is important to note that the CD4:CD8 ratio in donor K799 is nearly 3:1, while the ratio in z4100 is 1:1. A sample containing a fixed number of total CAR-T cells (as is the case in these experiments) will therefore differ in their respective numbers of cytotoxic CD8+ T cells with z4100 containing nearly twice as many CD8+ T cells, providing an explanation for the enhanced killing activity observed in cells derived from this donor.

### 3. Conclusions

Novel costimulatory signaling domains were found to support levels of proliferation and target cell killing that are equal to or better than those levels supported by 4-1BB signaling. Importantly, we demonstrate this feature of N1 and N6 here in CAR-T cells produced with our targeted insertion strategy in addition to other data in which CAR was delivered by randomly-inserting lentiviral vectors. This method reduces the likelihood that differences in CAR-T responses to antigen can be ascribed to differences in integrated copy number between different CAR-T preparations. Importantly, both random and targeted insertion strategies indicated that N6, especially, is a viable alternative to the costimulatory support provided by native 4-1BB.

### EXAMPLE 4

### Proliferation assay in CAR-T cells having 41BB, N1, or N6 as the co-stimulatory domain.

### 1. Preparation of CAR-T cells with co-stimulatory domains and proliferation assay

CAR elements featuring novel costimulatory domains were cloned out of lentiviral transfer vectors and ligated into the pDI vector. Expression of the CAR element is controlled by the JeT promoter and the element is flanked by TRAC homology arms to enable targeted gene insertion. This donor template is flanked by inverted terminal repeat sequences to enable packaging into AAV6 particles. These plasmids were first linearized by restriction endonuclease digestion and ethanol precipitation. Primed T cells were then nucleofected with TRC1-2x87EE, a linearized CAR plasmid, and STING siRNA to reduce toxicity mediated by intracellular nucleic acid sensors. Nucleic acid delivery was carried out using a Lonza 4D nucleofector. Edited T cells were grown in XVIVO-15 medium (Lonza) supplemented with 5% pooled human serum and 30ng/ml IL-2 (Gibco). Cultures were carried out for 7 days prior to magnetic depletion of non-edited CD3+ cells using a human CD3 positive selection kit (StemCell Technologies). Cells were rested overnight in 2ng/ml of IL-2 prior to labeling with 2µM CellTrace Violet (Life Technologies) in accordance with the manufacturers' recommendations. CellTrace Violet (CTV) is a substrate for intracellular esterases and functions much like carboxyfluoroscein-succinimidyl ester (CFSE). CTV diffuses across membranes into the cytoplasm, where it is cleaved by esterase enzymes, which are abundant in the cytoplasm of live cells. The cleavage product does not diffuse across cell membranes, reacts very strongly with free amino groups found in cytoplasmic proteins, and is fluorescent. When labeled cells divide, the fluorescent cytoplasmic proteins are evenly divided between the daughter cells, resulting in two cells that are each half as bright as the parental generation. By comparing CTV fluorescence to temporal (such as a day 0 control) or biological controls (e.g. non-stimulated cells), rates of proliferation of various cell populations can be measured using flow cytometry by comparing frequencies of CTV dim events. CTV-labeled CD3- fractions of T cells expressing either no CAR, CAR-4-1BB, CAR-N1, or CAR-N6 were then challenged with antigen-bearing tumor cells. For this assay, K562 cells stably expressing CD19 were used at two different effector-to-target (E:T) ratios, 2:1 and 1:1. Importantly, the CAR+ frequency was determined for each co-culture using biotinylated CD19-Fc and streptavidin-PE. Input numbers of T cells were normalized based on their CAR+ frequencies. Cocultures of T cells and CD19+ K562s were carried out for 5 days prior to flow cytometric analysis. CD4-PE and CD8-APC antibodies (BioLegend) were used to positively identify T cells. Data were acquired and analyzed using FlowJo software (TreeStar) and proliferation assessed by dye dilution.

### 2. Results of proliferation assay

The results from the proliferation assay appear in Figure 10. The overlaid histograms in Figure 10A depict the proliferation of CAR-4-1BB CAR-T cells and a lack of proliferation in negative control TRC KO T cells at two different E:T ratios. Slightly more than half of the CAR-4-1BB T cells proliferated in response to CD19+ target cells while 13% divided in the control samples. The overlaid histograms in Figure 10B show the proliferation rates of CAR-4-1BB cells versus CAR-N1 cells. Approximately equal frequencies of divided cells appear in each culture. The overlaid histograms in Figure 10C show proliferation rates of CAR-4-1BB T cells in comparison to CAR-N6 T cells. N6 supports proliferation in 77% of cells while 4-1BB supports proliferation in 56% of cells. A table of the frequencies of divided cells in each culture appears below.

**Table 2. Frequency of dividing CAR-T cells in co-culture with antigen-bearing tumor cells.**

| **Signaling variant** | **% divided** |
|---|---|
| 4-1BB | 56.2 |
| N1 | 55.1 |
| N6 | 77.4 |
| TRC- (CAR-) | 13.3 |

### 3. Conclusions

Lentiviral screens identified N6 as a lead candidate in an effort to identify alternative and/or superior signaling domains to incorporate into a CAR construct. This experiment demonstrated the ability of novel signaling domains to function in a single-copy targeted insertion scenario rather than the random insertion/variable copy number scenario typical of lentiviral delivery. These data support demonstrate that functional signaling variants can be designed and delivered. Importantly, N6 was particularly found to outperform 4-1BB as a costimulatory signal transducer in conjunction with CD3z following encounter with antigen.

### EXAMPLE 5

### Efficacy of CAR T cells bearing 4-1BB and N6 co-stimulatory domains in a murine xenograft model of disseminated B cell lymphoma.

### 1. Preparation of CAR-T cells and injection into tumor-bearing mice

The purpose of this study was to evaluate the efficacy of CAR T cells engineered to express anti-CD19 CAR constructs containing the N6 co-stimulatory domain and compare these cells to CAR T cells with a 4-1BB co-stimulatory domain built into the CAR.

Anti-CD19 CAR sequences featuring 4-1BB or N6 co-stimulatory domains were cloned into the pDI plasmid and used to produce AAV6 viral vectors. Expression of the CAR element was controlled by the JeT promoter and the transgene was flanked by TRAC homology arms to enable targeted gene insertion into the TRAC locus when this donor template was delivered in conjunction with the TRC1-2x.87EE site-specific endonuclease. This CAR transgene donor template was flanked by inverted terminal repeat sequences to enable packaging into AAV6 particles.

For the N6 co-stimulatory domain, two different CAR transgenes were produced and packaged into different AAV vectors for testing. These two N6-containing CAR elements contained different polyadenylation (PolyA) sequences utilized at the 3' end of the CAR transgene to evaluate whether these PolyA sequences impacted the function of the CAR T cells. The polyA sequence utilized in the 7241 (4-1BB) and 7205 (N6) constructs was an SV40 polyA sequence comprising SEQ ID NO: 33. The polyA sequence utilized in the 7206 (N6) construct is an SV40 bi-polyA sequence having a first sequence comprising SEQ ID NO: 34 and a second sequence comprising SEQ ID NO: 35. Table 3 outlines the features of the CAR constructs used in this study, which are illustrated in Figure 11. The sequences of the vectors used to generate AAVs encoding the 7241 (4-1BB) construct, the 7205 (N6) construct, and the 7206 (N6) construct, are provided in SEQ ID NOs: 36-38, respectively.

**Table 3. AAV Vectors**

| Name of AAV Vector | Promoter | scFv | Co-Stimulatory Domain | Activation Domain | PolyA Sequence |
|---|---|---|---|---|---|
| 7205 | JeT | FMC63 | Novel6 (N6) | CD3ζ | SV40 PolyA |
| 7206 | JeT | FMC63 | Novel6 (N6) | CD3ζ | SV40 Bi-PolyA |
| 7241 | JeT | FMC63 | 4-1BB | CD3ζ | SV40 PolyA |

Primed T cells were electroporated with TRC1-2x87EE mRNA. Nucleic acid delivery was carried out using a Lonza 4D Nucleofector. After electroporation, cells were mock transduced, or transduced with AAV6 vectors bearing donor templates with anti-CD19 CAR transgenes including either 4-1BB or N6 co-stimulatory domains.

Edited T cells were grown in XVIVO-15 medium (Lonza) supplemented with 5% pooled human serum and 30ng/ml IL-2 (Gibco). Cells were cultured for 5 days prior to magnetic depletion of non-edited CD3+ cells using a human CD3 positive selection kit (StemCell Technologies). Cells were cultured for an additional 3 days.

NSG mice (n=5 per group) were injected with 2e5 Raji cells expressing firefly luciferase (Raji-ffluc). Three days later, mice were injected with 1e6 control TCR KO cells each, or 1e6 CAR T cells produced using 7205 (N6), 7206 (N6), or 7241 (4-1BB) vectors. On the indicated days, live mice were injected i.p. with Luciferin substrate (150 mg/kg in saline), anesthetized, and Luciferase activity measured after 7 min using IVIS Spectrum (Perkin Elmer, Waltham, MA). Data was analyzed and exported using Living Image software 4.5.2 (Perkin Elmer, Waltham, MA). Luminescence signal intensity in the images is represented by radiance in p/sec/cm2/sr. Total flux was also calculated using Living Image software 4.5.2 (Perkin Elmer, Waltham, MA) using the entire animal as the region of interest. Mice were monitored for symptoms of disease progression and were euthanized when necessary according to pre-defined criteria.

### 2. Results of murine xenograft model

The result of the murine xenograft model of disseminated B cell lymphoma evaluating the efficacy of the CAR constructs described in Table 3 are displayed in Figures 12-14.

Engraftment and growth of Raji-ffluc cells in control mice receiving TCR KO cells was visible by ventral and dorsal imaging of mice at day 7 post-injection of Raji-ffluc, and rapid outgrowth of Raji cells was observed in these mice as indicated by increasing luminescence signal at Day 10 and Day 16 (Figure 12, Figure 13A, and Figure 13C). In contrast, treatment of mice with CAR T cells bearing the 7205 (N6), 7206 (N6), and 7241 (4-1BB) constructs resulted in delayed tumor growth (Figure 12 and Figure 13). Raji outgrowth was detected starting at approximately Day 20 in a subset of mice in the 7205 (N6) and 7241 (4-1BB) CAR T groups, as evidenced by dorsal and ventral imaging of the animals. However, in the 7206 (N6) CAR T treatment group, appreciable tumor growth was not observed during the 40 day study.

As shown in Figure 14, all 5 mice in the TRC KO control group were euthanized at day 19 due to the rapid onset of disease-related symptoms including complete hind-limb paralysis. However, treatment with CAR T cells produced using the 7205 (N6) and 7206 (N6) vectors enhanced survival of the mice, with all mice in these groups remaining alive at day 40 (one mouse in the 7206 group was removed from the study due to death unrelated to tumor growth or CAR T infusion). Mice treated with the 7241 4-1BB-containing CAR construct also extended this survival time of mice in this treatment group, with one mouse requiring euthanization at day 38, and the other four mice remaining alive through study day 40.

### 3. Conclusions

Treatment of mice engrafted with Raji-ffluc CD19+ cells with anti-CD19 CAR T cells expressing second generation CARs bearing the N6 co-stimulatory domain (in both the 7205 and the 7206 configurations) resulted in pro-longed survival of mice and a dramatic reduction in tumor burden compared to mice receiving TCR KO cells. Importantly, through study day 40, the 7205 (N6) construct appeared to perform comparably to the 7241 4-1BB containing-CAR, and the 7206 (N6) construct appeared to outperform both the 7205 (N6) and the 7241 (4-1BB) configurations in terms of durable suppression of Raji cell outgrowth. Overall, these data confirm the *in vitro* findings that the N6 co-stimulatory domain is functional as a co-stimulatory domain and supports the ability of CAR T cells to kill CD19+ targets *in vivo,* in agreement with experiments evaluating the *in vitro* activity of the constructs. Furthermore, constructs bearing the N6 co-stimulatory domain matched or exceeded the activity of a CAR T construct with the 4-1BB co-stimulatory domain.

### EXAMPLE 6

### Characterization of a third-generation CAR comprising multiple co-stimulatory domains

### 1. Production of CAR T cells expressing a third-generation CAR

Further constructs were prepared in order to evaluate the novel co-stimulatory domains encompassed by the invention as part of a third-generation CAR, wherein the intracellular signaling domains include two co-stimulatory domains and a CD3-ζ signaling domain.

In certain examples, a third-generation anti-CD19 CAR was prepared which comprised, from 5' to 3', the signal sequence (SEQ ID NO: 16), FMC63-based CD19-specific scFv (SEQ ID NO: 17), and CD8 hinge and transmembrane domains (SEQ ID NO: 18) described above, followed by a MyD88 co-stimulatory domain (SEQ ID NO: 39; sequence obtained from WO 2016/036746), an N6 co-stimulatory domain (SEQ ID NO: 8), and a CD3-ζ signaling domain (SEQ ID NO: 19). The SV40 bi-polyA signal sequence comprising SEQ ID NO: 34 and SEQ ID NO: 35 was positioned 3' downstream of the CAR sequence. As described in previous examples, the sequence encoding this construct was cloned into the pDI plasmid, and expression of the CAR was controlled by the JeT promoter. Further, the transgene was flanked by TRAC homology arms to enable targeted gene insertion into the TRAC locus when this donor template was delivered in conjunction with the TRC1-2x.87EE site-specific endonuclease. This CAR transgene donor template was further flanked by inverted terminal repeat sequences. A MyD88/N6 CAR donor template is illustrated in Figure 15, and the sequence of a vector comprising the donor template is provided as SEQ ID NO: 40.

In some experiments, the CAR donor template will be delivered as linearized DNA following linearization of the pDI plasmid. In other cases, the donor template will be packaged into AAV6 particles for viral delivery.

### 2. Evaluation of MyD88/N6 CAR T cells for cell killing, proliferation, and cytokine secretion

In some experiments, MyD88/N6 CAR T cells are produced as described above in Example 4 by nucleofection of primed T cells with a linearized CAR template plasmid, the TRC 1-2x.87EE meganuclease, and STING siRNA to reduce toxicity mediated by intracellular nucleic acid sensors. MyD88/N6 CAR T cells are further grown and expanded as described.

MyD88/N6 CAR T cells are characterized for cell killing proficiency, proliferation, and cytokine secretion. To evaluate cell killing proficiency and proliferation, MyD88/N6 CAR T cells are subjected to an antigen-induced stress test as described in Example 3 above, wherein MyD88/N6 CAR T cells are co-cultured with K562 cells engineered to express CD19 ("K19" cells) at various effector:target ratios. Proliferation is also assessed as described in Example 4 by labeling cells with CellTrace Violet and co-culturing with antigen-bearing K19 cells at various effector:target ratios. Cytokine secretion (*e.g.*, human IL-2, TNFα, and IFNγ) is determined as described in Example 2 above following co-culture with K19 cells at various effector:target ratios.

Similar experiments examining cell killing, proliferation, and cytokine secretion are conducted using transduction of recombinant AAV particles for delivery of the CAR donor template to primed T cells, which are further nucleofected with mRNA encoding the TRC 1-2x.87EE meganuclease.

### 3. Proliferation of MyD88/N6 CAR T cells

To compare the function of the N6 and MyD88/N6 co-stimulatory domains in CAR T cells, linearized plasmid DNA expressing the N6 co-stimulatory domain or the novel third-generation CAR containing the MyD88/N6 co-stimulatory domain were nucleofected into human T cells along with the TRC1-2x.87EE site-specific endonuclease and STING siRNA. The MyD88/N6 CAR coding construct is illustrated in Figure 15. The construct encoding the N6 CAR is referred to as 7206 and is provided as SEQ ID NO: 38. The construct encoding the MyD88/N6 CAR is referred to as 7240 and is provided as SEQ ID NO: 40.

After nucleofection, cells were grown in X-Vivo media (Lonza) supplemented with 5% FBS and 30 ng/ml IL-2 (Gibco) for 5 days. On day 5, remaining CD3⁺ T cells were labeled using the human CD3 positive selection kit II (StemCell Technologies) and magnetically removed as per the manufacturer's recommendations. Remaining CD3-depleted fractions were re-suspended in X-Vivo media supplemented with 10 ng/ml IL-15 and 3 ng/ml IL-21 (Gibco) and grown for an additional 2 days. To prepare samples for the assay, 2e⁶ T cells from the N6 and MyD88/N6 conditions, as well as TRC1-2x.87EE only treated control T cells, were labeled *in vitro* with a 2 µM solution of cell trace violet (CTV) solution. Post-incubation, CTV labeling consistency and CAR T cell frequencies were assessed on the Becton-Dickinson LSR:Fortessa flow cytometer after staining with CD19-Biotin Fc (Acro Biosystems) and Streptavidin PE (BD). For the proliferation assay, CAR T cell frequencies were normalized to 1% of the total T cell population added, with 2e⁵ total T cells (2e³ CAR T cells) added to duplicate wells on a 96-well round bottom plate in X-Vivo media without cytokine supplementation. To assess antigen-specific CAR T cell proliferation, 4e³ K19 cells were added to one well with 4e³ K562 cells added to the second in order to calculate non-specific, background proliferation. Cells were mixed and incubated for a total of 6 days.

On day 6 post-co-culture, cells were spun down and washed twice with PBS. To analyze proliferation of individual T cell subsets, samples were stained with CD4 BV711 and CD8 BV785 antibodies (Biolegend), as well as ghost dye BV510 (TONBO biosciences) in order to exclude dead cells. After staining, samples were run and data was collected on the Becton-Dickinson LSR:Fortessa flow cytometer.

### 4. Results of proliferation studies

The results of the proliferation assay comparing the N6 and MyD88/N6 co-stimulatory domains are shown in Figure 16. To gauge background proliferation of CAR negative populations, CTV dilution in T cells derived from the TRC-only nucleofected control sample were compared in wells co-cultured with either K19 or K562 cells. Importantly, both T cell subsets showed similar levels of non-specific proliferation in the presence of K19 (light shading) and K562 cells (dark shading), suggesting any proliferation was CD19-independent (Figure 16A and 16B). By comparison, both CD4⁺ and CD8⁺ T cells nucleofected with linearized plasmid DNA expressing the N6 co-stimulatory domain showed greater dilution of CTV in the presence of K19 cells compared to K562 controls, indicating substantial antigen-specific proliferation (Figure 16C and 16D). Notably, the same assessment done in T cells expressing the MyD88/N6 co-stimulatory domain also showed greater proliferation of CAR T cells in response to K19 cells (Figure 16E and 16F). The overall dilution of CTV was less in MyD88/N6 expressing T cells compared to CAR T cells expressing the N6 co-stimulatory domain alone; however, expression of either co-stimulatory domain resulted in greater dilution of CTV compared to TRC-only control T cells.

### 5. Conclusions

Nucleofection of primed T cells with linearized plasmid DNA expressing either the N6 or the third generation MyD88/N6 co-stimulatory domain resulted in CAR T cells that were able to proliferate, as indicated by dilution of CTV, in an antigen-specific manner. Furthermore, proliferation occurred in both CD4⁺ and CD8⁺ T cells subsets and above background seen in non-CAR expressing control cells. Collectively, this study demonstrated that both N6 and MyD88/N6 can function as co-stimulatory domains in CAR T cells.

### EXAMPLE 7

### Characterization of novel co-stimulatory domains in an inducible construct

### 1. Production of CAR T cells expressing a first generation CAR and an inducible construct comprising a novel co-stimulatory domain

Further constructs were prepared in order to evaluate the novel co-stimulatory domains encompassed by the invention as part of an inducible co-stimulatory construct, which is co-expressed with a first-generation anti-CD19 CAR comprising a CD3-ζ signaling domain.

In certain examples, a construct was prepared which comprised, from 5' to 3', an expression cassette for an inducible co-stimulatory construct, a T2A element, and a CAR expression cassette encoding a first-generation anti-CD19 CAR.

The first generation anti-CD19 CAR encoded by the CAR expression cassette included, from 5' to 3', the signal sequence (SEQ ID NO: 16), the FMC63-based CD19-specific scFv (SEQ ID NO: 17), and the CD8 hinge and transmembrane domains (SEQ ID NO: 18) described above, with an intracellular region comprising a CD3-ζ signaling domain (SEQ ID NO: 19). The SV40 bi-polyA signal sequence comprising SEQ ID NO: 34 and SEQ ID NO: 34 was positioned 3' downstream of the CAR sequence.

The inducible co-stimulatory construct included, from 5' to 3', either an N6 co-stimulatory domain alone (SEQ ID NO: 8), or a MyD88 domain (SEQ ID NO: 39) and an N6 domain (SEQ ID NO: 8) in tandem, followed by an Fv domain comprising two tandem ligand-binding FKBP12v36 domains (SEQ ID NO: 41; sequences obtained from WO 2015/123527), which bind the small molecule rimiducid to induce dimerization of the construct and activation of co-stimulatory signaling.

As described in previous examples, these constructs were cloned into the pDI plasmid, and expression of both the inducible co-stimulatory construct and the anti-CD19 CAR were controlled by the JeT promoter. Further, these constructs were flanked by TRAC homology arms to enable targeted gene insertion into the TRAC locus when delivered in conjunction with the TRC1-2x.87EE site-specific endonuclease. These constructs were further flanked by inverted terminal repeat sequences.

In some experiments, the donor template will be delivered as linearized DNA following linearization of the pDI plasmid. In other cases, the donor template will be packaged into AAV6 particles for viral delivery.

Cells expressing the anti-CD19 CAR in conjunction with an inducible co-stimulatory construct having only the N6 co-stimulatory domain are referred to as iN6 CAR T cells. Cells expressing the anti-CD19 CAR in conjunction with an inducible co-stimulatory construct having both the MyD88 and N6 co-stimulatory domains are referred to as iMyD88/N6 CAR T cells.

### 2. Evaluation of CAR T cells with inducible constructs for cell killing, proliferation, and cytokine secretion

In some experiments, iN6 CAR T cells or iMyD88/N6 CAR T cells are produced as described above in Example 4 by nucleofection of primed T cells with a linearized template plasmid, the TRC 1-2x.87EE meganuclease, and STING siRNA to reduce toxicity mediated by intracellular nucleic acid sensors. iN6 CAR T cells or iMyD88/N6 CAR T cells are further grown and expanded as described.

iN6 CAR T cells and iMyD88/N6 CAR T cells are characterized for cell killing proficiency, proliferation, and cytokine secretion in both the presence and absence of the small molecule rimiducid, which induces dimerization of the inducible construct and initiates co-stimulatory signaling in the cell. To evaluate cell killing proficiency and proliferation, CAR T cells are subjected to an antigen-induced stress test as described in Example 3 above, wherein the CAR T cells are co-cultured with K562 cells engineered to express CD19 ("K19" cells) at various effector:target ratios. Proliferation is also assessed as described in Example 4 by labeling cells with CellTrace Violet and co-culturing with antigen-bearing K19 cells at various effector:target ratios. Cytokine secretion (*e.g.*, human IL-2, TNFα, and IFNγ) is determined as described in Example 2 above following co-culture with K19 cells at various effector:target ratios.

Similar experiments examining cell killing, proliferation, and cytokine secretion are conducted using transduction of recombinant AAV particles for delivery of the donor template to primed T cells, which are further nucleofected with mRNA encoding the TRC 1-2x.87EE meganuclease.

### 3. Proliferation of iMyD88/N6 CAR T cells

To characterize the functionality of novel co-stimulatory domains in an inducible construct, primed T cells were nucleofected with linearized plasmid DNA expressing an iMyD88/N6 inducible co-stimulatory construct or, as a control, the N6 co-stimulatory domain expressed as part of the CAR. Cells were further nucleofected with the TRC1-2x.87EE site-specific endonuclease and STING siRNA as described above. As a negative control, a separate sample was nucleofected with the TRC1-2x.87EE site-specific endonuclease and STING siRNA only. The CAR donor template construct is illustrated in Figure 17. As described above, the construct encoding the N6 CAR is referred to as 7206 and is provided as SEQ ID NO: 38. The construct encoding the iMyD88/N6 co-stimulatory domain is referred to as 7235 and is provided as SEQ ID NO: 42.

Post-nucleofection, T cell samples were rested for 6 hours in X-Vivo (Lonza) media supplemented with 5% FBS and 30 ng/ml IL-2 (Gibco). Samples were then split in half into separate wells, with one well receiving rimiducid at a final concentration of 5 nanomolar and the other well left untreated. Cells were subsequently incubated for 5 days. On day 5, remaining CD3⁺ T cells were labeled using the human CD3 positive selection kit II (StemCell Technologies) and magnetically removed as per the manufacturer's recommendations. Remaining CD3-depleted fractions were re-suspended in X-Vivo media supplemented with 10 ng/ml IL-15 and 3 ng/ml IL-21 (Gibco). Samples that had received rimiducid on day 0 post-nucleofection were spiked with fresh rimiducid at a final concentration of 5 nanomolar, while untreated samples were resuspended in cytokine supplemented X-Vivo alone. Cells were then incubated for an additional 2 days.

To prepare samples for the assay, 2e⁶ T cells from the iMyD88/N6 and N6 conditions that had or had not received rimiducid, as well as TRC1-2x.87EE-only treated control T cells, were labeled *in vitro* with a 2 µM solution of cell trace violet (CTV) solution. Post-incubation, CTV labeling consistency and CAR T cell frequencies were assessed on the Becton-Dickinson LSR:Fortessa flow cytometer after staining with CD19-Biotin Fc (Acro Biosystems) and Streptavidin PE (BD). CAR T cell frequencies were normalized to 1% of the total T cell population added, with 2e⁵ total T cells (2e³ CAR T cells) added to two separate wells on a 96-well round bottom plate in X-Vivo media without cytokine supplementation. One well then received 4e³ targetK19 cells, while the other received 4e³ K562 cells as a control. Rimiducid was then added to samples that had received rimiducid on day 0 and day 5 post-nucleofection respectively. Cells were mixed and incubated for a total of 6 days.

On day 6 post-co-culture, samples were stained for flow cytometric analysis. To quantitate proliferation of individual T cell subsets, samples were stained with CD4 BV711 and CD8 BV785 antibodies (Biolegend), with the exclusion of dead cells during analysis occurring by addition of ghost dye BV510 (TONBO biosciences) to the staining cocktail. After staining, samples were run and data was collected on the Becton-Dickinson LSR:Fortessa flow cytometer.

### 4. Results of proliferation studies

The results of the proliferation assay comparing the novel inducible co-stimulatory construct iMyD88/N6 to the N6 CAR are shown in Figures 18-20. CD4⁺ and CD8⁺ T cells from non-CAR expressing TRC-only nucleofected cells showed similar levels of non-specific proliferation when co-cultured with either K19 (light shading) or K562 (dark shading) cells (Figure 18A and 18B).

By contrast, proliferation of CAR T cells expressing an N6 CAR was antigen-dependent, as dilution of CTV was greater when co-cultured with K19 (light shading) as opposed to K562 (dark shading) cells (Figure 19A and 19C). Further, CAR T cells expressing the non-inducible N6 CAR showed substantial proliferation of both T cell subsets in the presence (dark shading) or absence (light shading) of rimiducid and K19 cells (Figure 19B and 19D), showing that rimiducid does not have any function in the absence of a switch-dependent co-stimulatory domain.

For the inducible co-stimulatory construct, proliferation of both CD4⁺ and CD8⁺ T cells expressing iMyD88/N6 was greater when co-cultured with K19 (light shading) cells compared to K562 control cells (dark shading) (Figure 20A and 20C). Importantly, increased CTV dilution was dependent on rimiducid (dark shading) compared to non-rimiducid treated samples (light shading) when cultured with K19 cells for both T cell subsets analyzed, supporting the inducible function of the iMyD88/N6 switch (Figure 20B and 20D).

### 5. Conclusions

The expression of the novel inducible co-stimulatory construct iMyD88/N6 on CAR T cells resulted in the proliferation of both CD4⁺ and CD8⁺ T cells that was antigen-dependent. Strikingly, dilution of CTV on cultured T cells was greatest in the presence of rimiducid, showing the inducible nature of the co-stimulatory domain constructs when expressed in CAR T cells. As rimiducid had no effect on CAR T cell proliferation when the co-stimulatory domain is expressed as part of the CAR, these data support the functionality of the novel switch co-stimulatory domains on CAR T cell function.

## Claims

1. A nucleic acid molecule comprising a nucleotide sequence encoding a co-stimulatory domain having at least 90% sequence identity to the amino acid sequence of SEQ ID NO: 5.

2. The nucleic acid molecule of claim 1, wherein said nucleotide sequence encodes a chimeric antigen receptor (CAR) comprising said co-stimulatory domain.

3. The nucleic acid molecule of claim 2, wherein said CAR further comprises at least one intracellular signaling domain, preferably wherein said intracellular signaling domain is a CD3 ζ domain.

4. The nucleic acid molecule of any one of claims 1-3, wherein said nucleic acid molecule is an mRNA, a recombinant DNA construct, or a viral genome of a viral vector.

5. A recombinant DNA construct comprising said nucleic acid molecule of any one of claims 1-3.

6. The recombinant DNA construct of claim 5, wherein said recombinant DNA construct encodes a viral vector, wherein said viral vector comprises said nucleic acid molecule of any one of claims 1-3, preferably wherein said viral vector is a recombinant adeno-associated viral (AAV) vector.

7. A viral vector comprising said nucleic acid molecule of any one of claims 1-3, preferably wherein said viral vector is a recombinant AAV vector.

8. A genetically-modified T cell or natural killer (NK) cell comprising said nucleic acid molecule of any one of claims 1-3, preferably wherein said genetically-modified cell is a genetically-modified human T cell.

9. An *in vitro* method for producing a genetically-modified T cell or natural killer (NK) cell comprising at least one co-stimulatory domain of any one of claims 1-3, said method comprising introducing into a T cell or natural killer (NK) cell at least one nucleic acid molecule of any one of claims 1-3.

10. The method of claim 9, wherein said method further comprises introducing into said cell:
(i) a second nucleic acid molecule encoding an engineered nuclease, wherein said engineered nuclease is expressed in said cell, or;
(ii) an engineered nuclease protein;
wherein said engineered nuclease recognizes and cleaves a recognition sequence in the genome of said cell to produce a cleavage site, and wherein said nucleic acid molecule encoding said at least one co-stimulatory domain is inserted into the genome of said cell at said cleavage site.

11. The method of claim 10, wherein said engineered nuclease is an engineered meganuclease, a recombinant zinc-finger nuclease (ZFN), a recombinant transcription activator-like effector nuclease (TALEN), a CRISPR/Cas nuclease, or a megaTAL nuclease; preferably wherein said engineered nuclease is an engineered meganuclease.

12. The method of claim 10 or claim 11, wherein said nucleic acid molecule of any one of claims 1-3 further comprises sequences homologous to sequences flanking said cleavage site, such that said nucleic acid molecule is inserted into the genome of said cell at said cleavage site by homologous recombination.

13. A pharmaceutical composition comprising a pharmaceutically-acceptable carrier and said genetically-modified cell of claim 8.

14. A genetically modified cell of claim 8 for use in a method of immunotherapy for treating cancer in a subject in need thereof.

15. The genetically modified cell for the use of claim 14, wherein said cancer is a carcinoma, lymphoma, sarcoma, blastoma, or leukemia, preferably wherein said cancer is selected from the group consisting of a cancer of B-cell origin, such as B-lineage acute lymphoblastic leukemia, B-cell chronic lymphocytic leukemia, or B-cell non-Hodgkin's lymphoma; breast cancer; gastric cancer; neuroblastoma; osteosarcoma; lung cancer; melanoma; prostate cancer; colon cancer; renal cell carcinoma; ovarian cancer; rhabdomyo sarcoma; leukemia; and Hodgkin's lymphoma.

## Patentansprüche

1. Ein Nukleinsäuremolekül umfassend eine Nukleotidsequenz, die eine kostimulatorische Domäne mit mindestens 90% Sequenzidentität zu der Aminosäuresequenz von SEQ ID NO: 5 kodiert.

2. Das Nukleinsäuremolekül aus Anspruch 1, wobei jene Nukleotidsequenz einen chimären Antigenrezeptor (CAR) umfassend jene kostimulatorische Domäne kodiert.

3. Das Nukleinsäuremolekül aus Anspruch 2, wobei jener CAR weiter mindestens eine intrazelluläre Signalgebungsdomäne umfasst, vorzugsweise wobei jene intrazelluläre Signalgebungsdomäne eine CD3 ζ-Domäne ist.

4. Das Nukleinsäuremolekül aus irgendeinem der Ansprüche 1-3, wobei jenes Nukleinsäuremolekül eine mRNA, ein rekombinantes DNA-Konstrukt oder ein virales Genom eines viralen Vektors ist.

5. Ein rekombinantes DNA-Konstrukt umfassend jenes Nukleinsäuremolekül aus irgendeinem der Ansprüche 1-3.

6. Das rekombinante DNA-Konstrukt aus Anspruch 5, wobei jenes rekombinante DNA-Konstrukt einen viralen Vektor kodiert, wobei jener virale Vektor ein Nukleinsäuremolekül aus irgendeinem der Ansprüche 1-3 umfasst, vorzugsweise wobei jener virale Vektor ein rekombinanter Adeno-assoziierter viraler (AAV-) Vektor ist.

7. Ein viraler Vektor umfassend jenes Nukleinsäuremolekül aus irgendeinem der Ansprüche 1-3, vorzugsweise wobei jener virale Vektor ein rekombinanter AAV-Vektor ist.

8. Eine genetisch modifizierte T-Zelle oder natürliche Killer- (NK-) Zelle umfassend jenes Nukleinsäuremolekül aus irgendeinem der Ansprüche 1-3, vorzugsweise wobei jene genetisch modifizierte Zelle eine genetisch modifizierte humane T-Zelle ist.

9. Ein *in vitro*-Verfahren zur Herstellung einer genetisch modifizierten T-Zelle oder natürlichen Killer- (NK-) Zelle umfassend mindestens eine kostimulatorische Domäne aus irgendeinem der Ansprüche 1-3, jenes Verfahren umfassend Einführen in eine T-Zelle oder natürliche Killer- (NK-) Zelle mindestens eines Nukleinsäuremoleküls aus irgendeinem der Ansprüche 1-3.

10. Das Verfahren aus Anspruch 9, wobei jenes Verfahren weiter das Einführen von Folgendem in jene Zelle umfasst:
(i) eines zweiten Nukleinsäuremoleküls, das eine künstliche Nuklease kodiert, wobei jene künstliche Nuklease in jener Zelle exprimiert wird, oder;
(ii) eines künstlichen Nukleaseproteins;
wobei jene künstliche Nuklease eine Erkennungssequenz im Genom jener Zelle erkennt und spaltet, um eine Spaltstelle zu erzeugen, und wobei jenes Nukleinsäuremolekül, das jene mindestens eine kostimulatorische Domäne kodiert, an jener Spaltstelle ins Genom jener Zelle eingefügt wird.

11. Das Verfahren aus Anspruch 10, wobei jene künstliche Nuklease eine künstliche Meganuklease, eine rekombinante Zinkfinger-Nuklease (ZFN), eine rekombinante Transkriptionsfaktor-artige Effektor-Nuklease (TALEN), eine CRISPR/Cas-Nuklease oder eine megaTAL-Nuklease ist; vorzugsweise wobei jene künstliche Nuklease eine künstliche Meganuklease ist.

12. Das Verfahren aus Anspruch 10 oder Anspruch 11, wobei jenes Nukleinsäuremolekül aus irgendeinem der Ansprüche 1-3 weiter Sequenzen umfasst, die zu jene Spaltstelle flankierenden Sequenzen homolog sind, so dass jenes Nukleinsäuremolekül durch homologe Rekombination an jener Spaltstelle ins Genom jener Zelle eingefügt wird.

13. Eine pharmazeutische Zusammensetzung umfassend einen pharmazeutisch annehmbaren Träger und jene genetisch modifizierte Zelle aus Anspruch 8.

14. Eine genetisch modifizierte Zelle aus Anspruch 8 zur Verwendung in einem Verfahren der Immuntherapie zur Behandlung von Krebs in einem Subjekt, das dessen bedarf.

15. Die genetisch modifizierte Zelle für die Verwendung aus Anspruch 14, wobei jener Krebs ein Karzinom, Lymphom, Sarkom, Blastom oder Leukämie ist, vorzugsweise wobei jener Krebs ausgewählt ist aus der Gruppe bestehend aus einem Krebs mit B-Zell-Ursprung, wie etwa akuter lymphoblastischer Leukämie der B-Linie, chronischer lymphozytischer B-Zell-Leukämie oder B-Zell-Nicht-Hodgkin-Lymphom; Brustkrebs; Magenkrebs; Neuroblastom; Osteosarkom; Lungenkrebs; Melanom; Prostatakrebs; Kolonkrebs; Nierenzellkarzinom; Eierstockkrebs; Rhabdomyosarkom; Leukämie; und Hodgkin-Lymphom.

## Revendications

1. Molécule d'acide nucléique comprenant une séquence nucléotidique codant pour un domaine co-stimulateur présentant au moins 90 % d'identité de séquence avec la séquence d'acides aminés de SEQ ID NO : 5.

2. Molécule d'acide nucléique selon la revendication 1, dans laquelle ladite séquence nucléotidique code pour un récepteur antigénique chimérique (CAR) comprenant ledit domaine co-stimulateur.

3. Molécule d'acide nucléique selon la revendication 2, dans laquelle ledit CAR comprend en outre au moins un domaine de signalisation intracellulaire, de préférence dans laquelle ledit domaine de signalisation intracellulaire est un domaine CD3 ζ.

4. Molécule d'acide nucléique selon l'une quelconque des revendications 1 à 3, dans laquelle ladite molécule d'acide nucléique est un ARNm, une construction d'ADN recombinant ou un génome viral d'un vecteur viral.

5. Construction d'ADN recombinant comprenant ladite molécule d'acide nucléique selon l'une quelconque des revendications 1 à 3.

6. Construction d'ADN recombinant selon la revendication 5, dans laquelle ladite construction d'ADN recombinant code pour un vecteur viral, dans laquelle ledit vecteur viral comprend ladite molécule d'acide nucléique selon l'une quelconque des revendications 1 à 3, de préférence dans laquelle ledit vecteur viral est un vecteur viral adéno-associé (AAV) recombinant.

7. Vecteur viral comprenant ladite molécule d'acide nucléique selon l'une quelconque des revendications 1 à 3, de préférence dans lequel ledit vecteur viral est un vecteur AAV recombinant.

8. Cellule T ou cellule tueuse naturelle (NK) génétiquement modifiée comprenant ladite molécule d'acide nucléique selon l'une quelconque des revendications 1 à 3, de préférence dans laquelle ladite cellule génétiquement modifiée est une cellule T humaine génétiquement modifiée.

9. Procédé *in vitro* pour produire une cellule T ou une cellule tueuse naturelle (NK) génétiquement modifiée comprenant au moins un domaine co-stimulateur selon l'une quelconque des revendications 1 à 3, ledit procédé comprenant l'introduction dans une cellule T ou une cellule tueuse naturelle (NK) d'au moins une molécule d'acide nucléique selon l'une quelconque des revendications 1 à 3.

10. Procédé selon la revendication 9, dans lequel ledit procédé comprend en outre l'introduction dans ladite cellule :
(i) d'une seconde molécule d'acide nucléique codant pour une nucléase modifiée, dans lequel ladite nucléase modifiée est exprimée dans ladite cellule ; ou
(ii) d'une protéine de nucléase modifiée ; et
dans lequel ladite nucléase modifiée reconnaît et clive une séquence de reconnaissance dans le génome de ladite cellule pour produire un site de clivage, et dans lequel ladite molécule d'acide nucléique codant pour ledit au moins un domaine co-stimulateur est insérée dans le génome de ladite cellule au niveau dudit site de clivage.

11. Procédé selon la revendication 10, dans lequel ladite nucléase modifiée est une méganucléase modifiée, une nucléase à doigt de zinc (ZFN) recombinante, une nucléase effectrice de type activateur de transcription (TALEN) recombinante, une nucléase CRISPR/Cas, ou une nucléase mégaTAL ; de préférence, dans lequel ladite nucléase modifiée est une méganucléase modifiée.

12. Procédé selon la revendication 10 ou la revendication 11, dans lequel ladite molécule d'acide nucléique selon l'une quelconque des revendications 1 à 3 comprend en outre des séquences homologues à des séquences encadrant ledit site de clivage, de sorte que ladite molécule d'acide nucléique est insérée dans le génome de ladite cellule au niveau dudit site de clivage par recombinaison homologue.

13. Composition pharmaceutique comprenant un support pharmaceutiquement acceptable et ladite cellule génétiquement modifiée selon la revendication 8.

14. Cellule génétiquement modifiée selon la revendication 8 pour une utilisation dans un procédé d'immunothérapie pour le traitement d'un cancer chez un sujet en ayant besoin.

15. Cellule génétiquement modifiée pour l'utilisation de la revendication 14, dans laquelle ledit cancer est un carcinome, un lymphome, un sarcome, un blastome ou une leucémie, de préférence dans laquelle ledit cancer est sélectionné dans le groupe consistant en un cancer d'origine lymphocytaire B, tel qu'une leucémie lymphoblastique aiguë à cellules B, une leucémie lymphoïde chronique à cellules B, ou un lymphome non hodgkinien à cellules B ; un cancer du sein ; un cancer gastrique ; un neuroblastome ; un ostéosarcome ; un cancer du poumon ; un mélanome ; un cancer de la prostate ; un cancer du côlon ; un carcinome à cellules rénales ; un cancer de l'ovaire ; un rhabdomyosarcome ; une leucémie ; et un lymphome de Hodgkin.
